# EUROPEAN PATENT SPECIFICATION

(11) **EP 4 011 884 B1**
(45) Date of publication and mention of the grant of the patent: **25.12.2024**
(21) Application number: 21757420.1
(22) Date of filing: 19.02.2021
(51) Int. Cl.: C07D 487/22, C09K 11/06, H10K 85/60, H10K 50/11

(54) **COMPOUNDS AND ORGANIC ELECTROLUMINESCENT ELEMENT**
VERBINDUNGEN UND ORGANISCHES ELEKTROLUMINESZENTES ELEMENT
COMPOSÉS ET ÉLÉMENT ÉLECTROLUMINESCENT ORGANIQUE

(30) Priority: 21.02.2020 JP 2020028708
(43) Date of publication of application: 15.06.2022
(73) Proprietor: Idemitsu Kosan Co.,Ltd., Tokyo 100-8321 (JP)
(72) Inventor: TAKAHASHI, Ryota, Sodegaura-shi, Chiba 299-0293 (JP); KUSHIDA, Tomokatsu, Sodegaura-shi, Chiba 299-0293 (JP); KUDO, Yu, Sodegaura-shi, Chiba 299-0293 (JP); NAKANO, Yuki, Sodegaura-shi, Chiba 299-0293 (JP)
(74) Representative: Hoffmann Eitle
(86) International application number: PCT/JP2021/006260
(87) International publication number: WO 2021/167045

(56) References cited:
- WO-A1-2019/111971
- WO-A1-2019/111971
- WO-A1-2019/240251
- WO-A1-2019/240251
- WO-A1-2020/250961
- US-A1- 2019 221 747

## Description

### Technical Field

The invention relates to a novel compound and an organic electroluminescence device as set forth in the claims.

### Background Art

When voltage is applied to an organic electroluminescence device (hereinafter, referred to as an organic EL device in several cases), holes and electrons are injected into an emitting layer from an anode and a cathode, respectively. Then, thus injected holes and electrons are recombined in the emitting layer, and excitons are formed therein.

Conventional organic EL devices have not yet sufficient device performance. Although materials for an organic EL device are being improved gradually to increase the performances of the organic EL device, high performances are further offered. In particular, improvement in lifetime of an organic EL device is an important task relating to a lifetime of commercial products provided with the organic EL device, and thus a material enabling to realize a long-lifetime organic EL device is required.

Patent Documents 1 and 2 disclose using a compound having a specific structure in an emitting layer of an organic EL device.

### Related Art Documents

### Patent Documents

[Patent Document 1] WO 2019/111971
[Patent Document 2] WO 2019/240251

### Summary of the Invention

It is an object of the invention to provide a compound capable of fabricating an organic EL device having a long lifetime.

As a result of extensive studies to achieve the above object, the inventors have found that an organic EL device having excellent lifetime can be obtained when a compound having a specific structure is used as set forth in the claims, and have completed the invention.

According to the invention, a compound capable of fabricating an organic EL device having a long lifetime can be provided.

### Brief Description of the Drawings

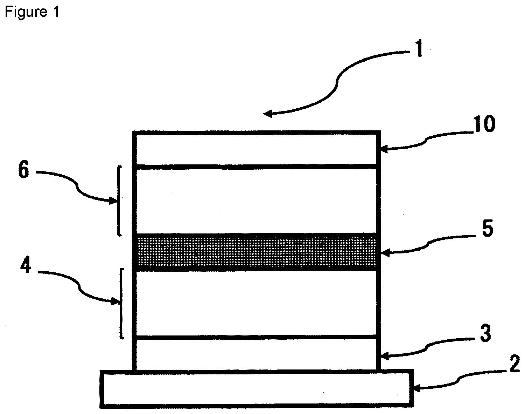

Figure 1 is a diagram showing a schematic configuration of an organic EL device according to an aspect of the invention.

### Mode for Carrying out the Invention

### [Definition]

In this specification, a hydrogen atom includes its isotopes different in the number of neutrons, namely, a protium, a deuterium and a tritium.

In this specification, at a bondable position in a chemical formula where a symbol such as "R", or "D" representing a deuterium atom is not indicated, a hydrogen atom, that is, a protium atom, a deuterium atom or a tritium atom is bonded.

In this specification, the number of ring carbon atoms represents the number of carbon atoms forming a subject ring itself among the carbon atoms of a compound having a structure in which atoms are bonded in a ring form (for example, a monocyclic compound, a fused ring compound, a cross-linked compound, a carbocyclic compound, or a heterocyclic compound). When the subject ring is substituted by a substituent, the carbon contained in the substituent is not included in the number of ring carbon atoms. The same shall apply to "the number of ring carbon atoms" described below, unless otherwise specified. For example, a benzene ring has 6 ring carbon atoms, a naphthalene ring includes 10 ring carbon atoms, a pyridine ring includes 5 ring carbon atoms, and a furan ring includes 4 ring carbon atoms. Further, for example, a 9,9-diphenylfluorenyl group includes 13 ring carbon atoms, and a 9,9'-spirobifluorenyl group includes 25 ring carbon atoms.

When a benzene ring is substituted by, for example, an alkyl group as a substituent, the number of carbon atoms of the alkyl group is not included in the number of ring carbon atoms of the benzene ring. Therefore, the number of ring carbon atoms of the benzene ring substituted by the alkyl group is 6. When a naphthalene ring is substituted by, for example, an alkyl group as a substituent, the number of carbon atoms of the alkyl group is not included in the number of ring carbon atoms of the naphthalene ring. Therefore, the number of ring carbon atoms of the naphthalene ring substituted by the alkyl group is 10.

In this specification, the number of ring atoms represents the number of atoms forming a subject ring itself among the atoms of a compound having a structure in which atoms are bonded in a ring form (for example, the structure includes a monocyclic ring, a fused ring and a ring assembly) (for example, a monocyclic compound, a fused ring compound, a cross-linked compound, a carbocyclic compound and a heterocyclic compound). The number of ring atoms does not include atoms which do not form the ring (for example, a hydrogen atom which terminates a bond of the atoms forming the ring), or atoms contained in a substituent when the ring is substituted by the substituent. The same shall apply to "the number of ring atoms" described below, unless otherwise specified. For example, the number of atoms of a pyridine ring is 6, the number of atoms of a quinazoline ring is 10, and the number of a furan ring is 5. For example, hydrogen atoms bonded to a pyridine ring and atoms constituting a substituent substituted on the pyridine ring are not included in the number of ring atoms of the pyridine ring. Therefore, the number of ring atoms of a pyridine ring with which a hydrogen atom or a substituent is bonded is 6. For example, hydrogen atoms and atoms constituting a substituent which are bonded with a quinazoline ring is not included in the number of ring atoms of the quinazoline ring. Therefore, the number of ring atoms of a quinazoline ring with which a hydrogen atom or a substituent is bonded is 10.

In this specification, "XX to YY carbon atoms" in the expression "a substituted or unsubstituted ZZ group including XX to YY carbon atoms" represents the number of carbon atoms in the case where the ZZ group is unsubstituted by a substituent, and does not include the number of carbon atoms of a substituent in the case where the ZZ group is substituted by the substituent. Here, "YY" is larger than "XX", and "XX" means an integer of 1 or more and "YY" means an integer of 2 or more.

In this specification, "XX to YY atoms" in the expression "a substituted or unsubstituted ZZ group including XX to YY atoms" represents the number of atoms in the case where the ZZ group is unsubstituted by a substituent, and does not include the number of atoms of a substituent in the case where the ZZ group is substituted by the substituent. Here, "YY" is larger than "XX", and "XX" means an integer of 1 or more and "YY" means an integer of 2 or more.

In this specification, the unsubstituted ZZ group represents the case where the "substituted or unsubstituted ZZ group" is a "ZZ group unsubstituted by a substituent", and the substituted ZZ group represents the case where the "substituted or unsubstituted ZZ group" is a " ZZ group substituted by a substituent" .

In this specification, a term "unsubstituted" in the case of "a substituted or unsubstituted ZZ group" means that hydrogen atoms in the ZZ group are not substituted by a substituent. Hydrogen atoms in a term "unsubstituted ZZ group" are a protium atom, a deuterium atom, or a tritium atom.

In this specification, a term "substituted" in the case of "a substituted or unsubstituted ZZ group" means that one or more hydrogen atoms in the ZZ group are substituted by a substituent. Similarly, a term "substituted" in the case of "a BB group substituted by an AA group" means that one or more hydrogen atoms in the BB group are substituted by the AA group.

### "Substituent as described in this specification"

Hereinafter, the substituent described in this specification will be explained.

The number of ring carbon atoms of the "unsubstituted aryl group" described in this specification is 6 to 50, preferably 6 to 30, and more preferably 6 to 18, unless otherwise specified.

The number of ring atoms of the "unsubstituted heterocyclic group" described in this specification is 5 to 50, preferably 5 to 30, and more preferably 5 to 18, unless otherwise specified.

The number of carbon atoms of the "unsubstituted alkyl group" described in this specification is 1 to 50, preferably 1 to 20, and more preferably 1 to 6, unless otherwise specified.

The number of carbon atoms of the "unsubstituted alkenyl group" described in this specification is 2 to 50, preferably 2 to 20, and more preferably 2 to 6, unless otherwise specified.

The number of carbon atoms of the "unsubstituted alkynyl group" described in this specification is 2 to 50, preferably 2 to 20, and more preferably 2 to 6, unless otherwise specified.

The number of ring carbon atoms of the "unsubstituted cycloalkyl group" described in this specification is 3 to 50, preferably 3 to 20, and more preferably 3 to 6, unless otherwise specified.

The number of ring carbon atoms of the "unsubstituted arylene group" described in this specification is 6 to 50, preferably 6 to 30, and more preferably 6 to 18, unless otherwise specified.

The number of ring atoms of the "unsubstituted divalent heterocyclic group" described in this specification is 5 to 50, preferably 5 to 30, and more preferably 5 to 18, unless otherwise specified.

The number of carbon atoms of the "unsubstituted alkylene group" described in this specification is 1 to 50, preferably 1 to 20, and more preferably 1 to 6, unless otherwise specified.

### · "Substituted or unsubstituted aryl group"

Specific examples of the "substituted or unsubstituted aryl group" described in this specification (specific example group G1) include the following unsubstituted aryl groups (specific example group G1A), substituted aryl groups (specific example group G1B), and the like. (Here, the unsubstituted aryl group refers to the case where the "substituted or unsubstituted aryl group" is an " aryl group unsubstituted by a substituent" , and the substituted aryl group refers to the case where the "substituted or unsubstituted aryl group" is an " aryl group substituted by a substituent".). In this specification, in the case where simply referred as an "aryl group", it includes both a "unsubstituted aryl group" and a "substituted aryl group."

The "substituted aryl group" means a group in which one or more hydrogen atoms of the "unsubstituted aryl group" are substituted by a substituent. Specific examples of the "substituted aryl group" include, for example, groups in which one or more hydrogen atoms of the "unsubstituted aryl group" of the following specific example group G1A are substituted by a substituent, the substituted aryl groups of the following specific example group G1B, and the like. It should be noted that the examples of the "unsubstituted aryl group" and the examples of the "substituted aryl group" enumerated in this specification are mere examples, and the "substituted aryl group" described in this specification also includes a group in which a hydrogen atom bonded with a carbon atom of the aryl group itself in the "substituted aryl group" of the following specific group G1B is further substituted by a substituent, and a group in which a hydrogen atom of a substituent in the "substituted aryl group" of the following specific group G1B is further substituted by a substituent.

· Unsubstituted aryl group (specific example group G1A):
   a phenyl group,
   a p-biphenyl group,
   a m-biphenyl group,
   an o-biphenyl group,
   a p-terphenyl-4-yl group,
   a p-terphenyl-3-yl group,
   a p-terphenyl-2-yl group,
   a m-terphenyl-4-yl group,
   a m-terphenyl-3-yl group,
   a m-terphenyl-2-yl group,
   an o-terphenyl-4-yl group,
   an o-terphenyl-3-yl group,
   an o-terphenyl-2-yl group,
   a 1-naphthyl group,
   a 2-naphthyl group,
   an anthryl group,
   a benzanthryl group,
   a phenanthryl group,
   a benzophenanthryl group,
   a phenalenyl group,
   a pyrenyl group,
   a chrysenyl group,
   a benzochrysenyl group,
   a triphenylenyl group,
   a benzotriphenylenyl group,
   a tetracenyl group,
   a pentacenyl group,
   a fluorenyl group,
   a 9,9'-spirobifluorenyl group,
   a benzofluorenyl group,
   a dibenzofluorenyl group,
   a fluoranthenyl group,
   a benzofluoranthenyl group,
   a perylenyl group, and
   a monovalent aryl group derived by removing one hydrogen atom from the ring structures represented by any of the following general formulas (TEMP-1) to (TEMP-15). 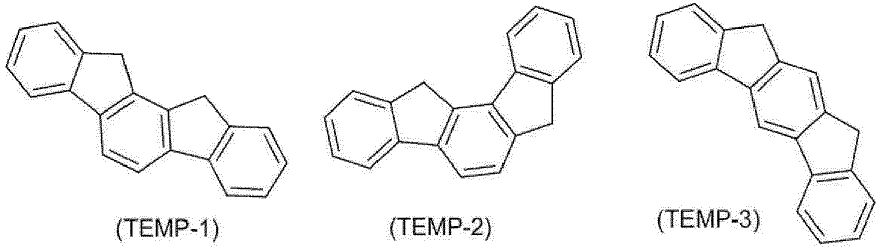 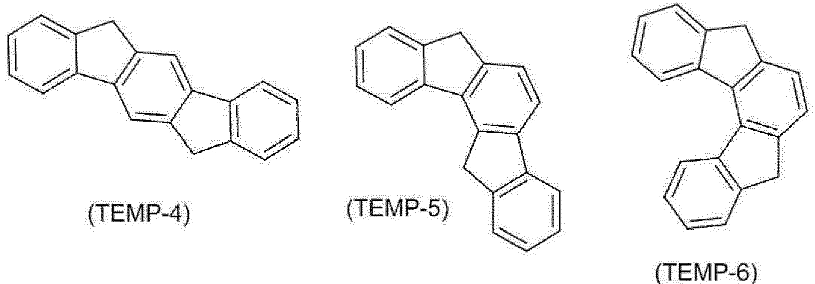 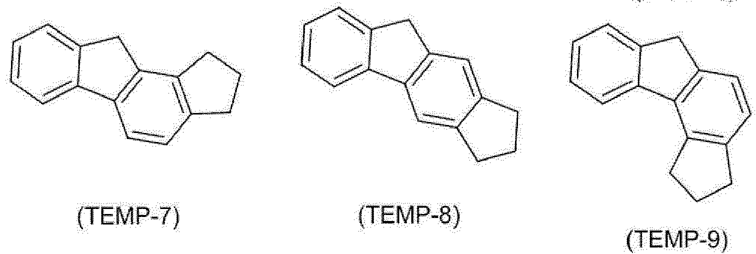 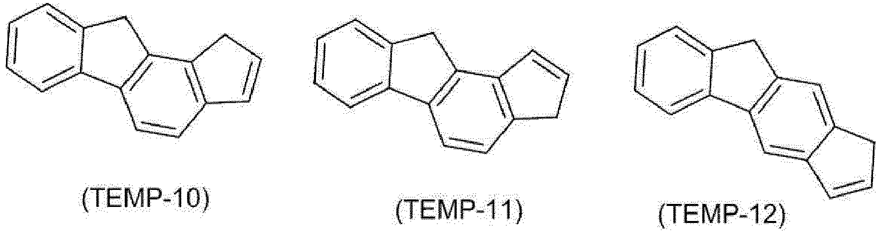 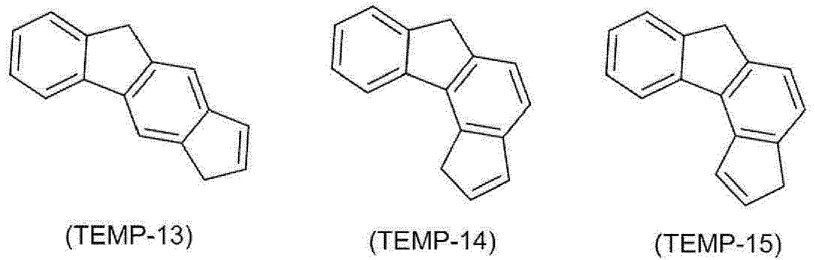
· Substituted aryl group (specific example group G1B):
   an o-tolyl group,
   a m-tolyl group,
   a p-tolyl group,
   a p-xylyl group,
   a m-xylyl group,
   an o-xylyl group,
   a p-isopropylphenyl group,
   a m-isopropylphenyl group,
   an o-isopropylphenyl group,
   a p-t-butylphenyl group,
   a m-t-butylphenyl group,
   an o-t-butylphenyl group,
   a 3,4,5-trimethylphenyl group,
   a 9,9-dimethylfluorenyl group,
   a 9,9-diphenylfluorenyl group,
   a 9,9-bis(4-methylphenyl)fluorenyl group,
   a 9,9-bis(4-isopropylphenyl)fluorenyl group,
   a 9,9-bis(4-t-butylphenyl)fluorenyl group,
   a cyanophenyl group,
   a triphenylsilylphenyl group,
   a trimethylsilylphenyl group,
   a phenylnaphthyl group,
   a naphthylphenyl group, and
   a group in which one or more hydrogen atoms of a monovalent group derived from the ring structures represented by any of the general formulas (TEMP-1) to (TEMP-15) are substituted by a substituent.

### · "Substituted or unsubstituted heterocyclic group"

The "heterocyclic group" described in this specification is a ring group having at least one hetero atom in the ring atom. Specific examples of the hetero atom include a nitrogen atom, an oxygen atom, a sulfur atom, a silicon atom, a phosphorus atom, and a boron atom.

The "heterocyclic group" in this specification is a monocyclic group or a fused ring group.

The "heterocyclic group" in this specification is an aromatic heterocyclic group or a non-aromatic heterocyclic group.

Specific examples of the "substituted or unsubstituted heterocyclic group" (specific example group G2) described in this specification include the following unsubstituted heterocyclic group (specific example group G2A), the following substituted heterocyclic group (specific example group G2B), and the like. (Here, the unsubstituted heterocyclic group refers to the case where the "substituted or unsubstituted heterocyclic group" is a " heterocyclic group unsubstituted by a substituent", and the substituted heterocyclic group refers to the case where the "substituted or unsubstituted heterocyclic group" is a " heterocyclic group substituted by a substituent".). In this specification, in the case where simply referred as a "heterocyclic group", it includes both the "unsubstituted heterocyclic group" and the "substituted heterocyclic group."

The "substituted heterocyclic group" means a group in which one or more hydrogen atom of the "unsubstituted heterocyclic group" are substituted by a substituent. Specific examples of the "substituted heterocyclic group" include a group in which a hydrogen atom of "unsubstituted heterocyclic group" of the following specific example group G2A is substituted by a substituent, the substituted heterocyclic groups of the following specific example group G2B, and the like. It should be noted that the examples of the "unsubstituted heterocyclic group" and the examples of the "substituted heterocyclic group" enumerated in this specification are mere examples, and the "substituted heterocyclic group" described in this specification includes groups in which hydrogen atom bonded with a ring atom of the heterocyclic group itself in the "substituted heterocyclic group" of the specific example group G2B is further substituted by a substituent, and a group in which hydrogen atom of a substituent in the "substituted heterocyclic group" of the specific example group G2B is further substituted by a substituent.

Specific example group G2A includes, for example, the following unsubstituted heterocyclic group containing a nitrogen atom (specific example group G2A1), the following unsubstituted heterocyclic group containing an oxygen atom (specific example group G2A2), the following unsubstituted heterocyclic group containing a sulfur atom (specific example group G2A3), and the monovalent heterocyclic group derived by removing one hydrogen atom from the ring structures represented by any of the following general formulas (TEMP-16) to (TEMP-33) (specific example group G2A4).

Specific example group G2B includes, for example, the following substituted heterocyclic group containing a nitrogen atom (specific example group G2B1), the following substituted heterocyclic group containing an oxygen atom (specific example group G2B2), the following substituted heterocyclic group containing a sulfur atom (specific example group G2B3), and the following group in which one or more hydrogen atoms of the monovalent heterocyclic group derived from the ring structures represented by any of the following general formulas (TEMP-16) to (TEMP-33) are substituted by a substituent (specific example group G2B4).

· Unsubstituted heterocyclic group containing a nitrogen atom (specific example group G2A1):
   a pyrrolyl group,
   an imidazolyl group,
   a pyrazolyl group,
   a triazolyl group,
   a tetrazolyl group,
   an oxazolyl group,
   an isoxazolyl group,
   an oxadiazolyl group,
   a thiazolyl group,
   an isothiazolyl group,
   a thiadiazolyl group,
   a pyridyl group,
   a pyridazinyl group,
   a pyrimidinyl group,
   a pyrazinyl group,
   a triazinyl group,
   an indolyl group,
   an isoindolyl group,
   an indolizinyl group,
   a quinolizinyl group,
   a quinolyl group,
   an isoquinolyl group,
   a cinnolyl group,
   a phthalazinyl group,
   a quinazolinyl group,
   a quinoxalinyl group,
   a benzimidazolyl group,
   an indazolyl group,
   a phenanthrolinyl group,
   a phenanthridinyl group,
   an acridinyl group,
   a phenazinyl group,
   a carbazolyl group,
   a benzocarbazolyl group,
   a morpholino group,
   a phenoxazinyl group,
   a phenothiazinyl group,
   an azacarbazolyl group, and
   a diazacarbazolyl group.
· Unsubstituted heterocyclic group containing an oxygen atom (specific example group G2A2):
   a furyl group,
   an oxazolyl group,
   an isoxazolyl group,
   an oxadiazolyl group,
   a xanthenyl group,
   a benzofuranyl group,
   an isobenzofuranyl group,
   a dibenzofuranyl group,
   a naphthobenzofuranyl group,
   a benzoxazolyl group,
   a benzisoxazolyl group,
   a phenoxazinyl group,
   a morpholino group,
   a dinaphthofuranyl group,
   an azadibenzofuranyl group,
   a diazadibenzofuranyl group,
   an azanaphthobenzofuranyl group, and
   a diazanaphthobenzofuranyl group.
· Unsubstituted heterocyclic group containing a sulfur atom (specific example group G2A3):
   a thienyl group,
   a thiazolyl group,
   an isothiazolyl group,
   a thiadiazolyl group,
   a benzothiophenyl group (benzothienyl group),
   an isobenzothiophenyl group (isobenzothienyl group),
   a dibenzothiophenyl group (dibenzothienyl group),
   a naphthobenzothiophenyl group (naphthobenzothienyl group),
   a benzothiazolyl group,
   a benzisothiazolyl group,
   a phenothiazinyl group,
   a dinaphthothiophenyl group (dinaphthothienyl group),
   an azadibenzothiophenyl group (azadibenzothienyl group),
   a diazadibenzothiophenyl group (diazadibenzothienyl group),
   an azanaphthobenzothiophenyl group (azanaphthobenzothienyl group), and
   a diazanaphthobenzothiophenyl group (diazanaphthobenzothienyl group).
· Monovalent heterocyclic group derived by removing one hydrogen atom from the ring structures represented by any of the following general formulas (TEMP-16) to (TEMP-33) (specific example group G2A4):

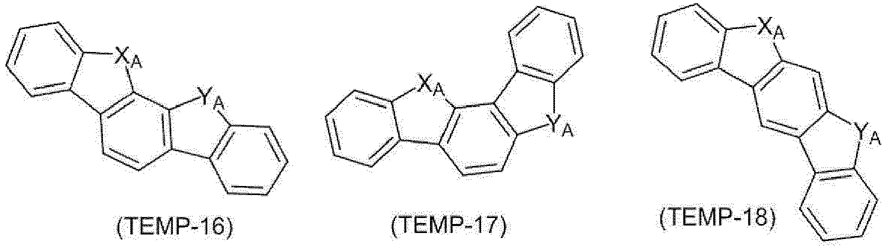

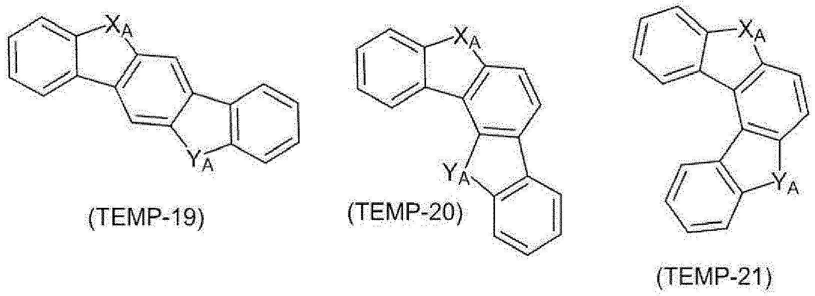

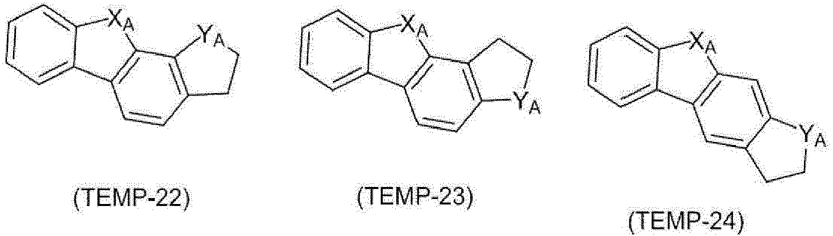

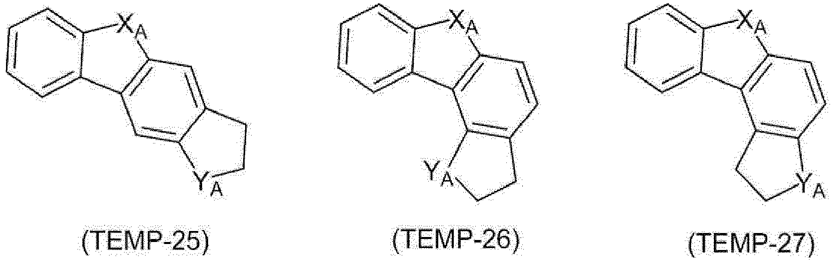

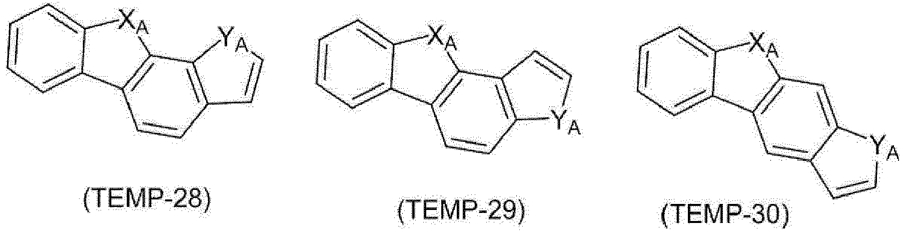

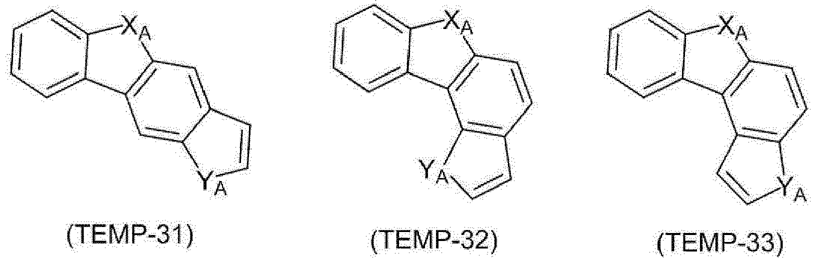

In the general formulas (TEMP-16) to (TEMP-33), X_{A} and Y_{A} are independently an oxygen atom, a sulfur atom, NH, or CH₂. Provided that at least one of X_{A} and Y_{A} is an oxygen atom, a sulfur atom, or NH.

In the general formulas (TEMP-16) to (TEMP-33), when at least one of X_{A} and Y_{A} is NH or CH₂, the monovalent heterocyclic group derived from the ring structures represented by any of the general formulas (TEMP-16) to (TEMP-33) includes a monovalent group derived by removing one hydrogen atom from these NH or CH₂.

· Substituted heterocyclic group containing a nitrogen atom (specific example group G2B1):
   a (9-phenyl)carbazolyl group,
   a (9-biphenylyl)carbazolyl group,
   a (9-phenyl)phenylcarbazolyl group,
   a (9-naphthyl)carbazolyl group,
   a diphenylcarbazol-9-yl group,
   a phenylcarbazol-9-yl group,
   a methylbenzimidazolyl group,
   an ethylbenzimidazolyl group,
   a phenyltriazinyl group,
   a biphenylyltriazinyl group,
   a diphenyltriazinyl group,
   a phenylquinazolinyl group, and
   a biphenylylquinazolinyl group.
· Substituted heterocyclic group containing an oxygen atom (specific example group G2B2):
   a phenyldibenzofuranyl group,
   a methyldibenzofuranyl group,
   a t-butyldibenzofuranyl group, and
   a monovalent residue of spiro[9H-xanthene-9,9'-[9H]fluorene].
· Substituted heterocyclic group containing a sulfur atom (specific example group G2B3):
   a phenyldibenzothiophenyl group,
   a methyldibenzothiophenyl group,
   a t-butyldibenzothiophenyl group, and
   a monovalent residue of spiro[9H-thioxanthene-9,9'-[9H]fluorene].
· Group in which one or more hydrogen atoms of the monovalent heterocyclic group derived from the ring structures represented by any of the following general formulas (TEMP-16) to (TEMP-33) are substituted by a substituent (specific example group G2B4):
   The "one or more hydrogen atoms of the monovalent heterocyclic group" means one or more hydrogen atoms selected from hydrogen atoms bonded with ring carbon atoms of the monovalent heterocyclic group, a hydrogen atom bonded with a nitrogen atom when at least one of X_{A} and Y_{A} is NH, and hydrogen atoms of a methylene group when one of X_{A} and Y_{A} is CH₂.

### · "Substituted or unsubstituted alkyl group"

Specific examples of the "substituted or unsubstituted alkyl group" (specific example group G3) described in this specification include the following unsubstituted alkyl groups (specific example group G3A) and the following substituted alkyl groups (specific example group G3B). (Here, the unsubstituted alkyl group refers to the case where the "substituted or unsubstituted alkyl group" is an " alkyl group unsubstituted by a substituent", and the substituted alkyl group refers to the case where the "substituted or unsubstituted alkyl group" is an " alkyl group substituted by a substituent".). In this specification, in the case where simply referred as an "alkyl group" includes both the "unsubstituted alkyl group" and the "substituted alkyl group."

The "substituted alkyl group" means a group in which one or more hydrogen atoms in the "unsubstituted alkyl group" are substituted by a substituent. Specific examples of the "substituted alkyl group" include groups in which one or more hydrogen atoms in the following "unsubstituted alkyl group" (specific example group G3A) are substituted by a substituent, the following substituted alkyl group (specific example group G3B), and the like. In this specification, the alkyl group in the "unsubstituted alkyl group" means a linear alkyl group. Thus, the "unsubstituted alkyl group" includes a straight-chain "unsubstituted alkyl group" and a branched-chain "unsubstituted alkyl group". It should be noted that the examples of the "unsubstituted alkyl group" and the examples of the "substituted alkyl group" enumerated in this specification are mere examples, and the "substituted alkyl group" described in this specification includes a group in which hydrogen atom of the alkyl group itself in the "substituted alkyl group" of the specific example group G3B is further substituted by a substituent, and a group in which hydrogen atom of a substituent in the "substituted alkyl group" of the specific example group G3B is further substituted by a substituent.

· Unsubstituted alkyl group (specific example group G3A):
   a methyl group,
   an ethyl group,
   a n-propyl group,
   an isopropyl group,
   a n-butyl group,
   an isobutyl group,
   a s-butyl group, and
   a t-butyl group.
· Substituted alkyl group (specific example group G3B):
   a heptafluoropropyl group (including isomers),
   a pentafluoroethyl group,
   a 2,2,2-trifluoroethyl group, and
   a trifluoromethyl group.

### · "Substituted or unsubstituted alkenyl group"

Specific examples of the "substituted or unsubstituted alkenyl group" described in this specification (specific example group G4) include the following unsubstituted alkenyl group (specific example group G4A), the following substituted alkenyl group (specific example group G4B), and the like. (Here, the unsubstituted alkenyl group refers to the case where the "substituted or unsubstituted alkenyl group" is a " alkenyl group unsubstituted by a substituent", and the "substituted alkenyl group" refers to the case where the "substituted or unsubstituted alkenyl group" is a " alkenyl group substituted by a substituent."). In this specification, in the case where simply referred as an "alkenyl group" includes both the "unsubstituted alkenyl group" and the "substituted alkenyl group."

The "substituted alkenyl group" means a group in which one or more hydrogen atoms in the "unsubstituted alkenyl group" are substituted by a substituent. Specific examples of the "substituted alkenyl group" include a group in which the following "unsubstituted alkenyl group" (specific example group G4A) has a substituent, the following substituted alkenyl group (specific example group G4B), and the like. It should be noted that the examples of the "unsubstituted alkenyl group" and the examples of the "substituted alkenyl group" enumerated in this specification are mere examples, and the "substituted alkenyl group" described in this specification includes a group in which a hydrogen atom of the alkenyl group itself in the "substituted alkenyl group" of the specific example group G4B is further substituted by a substituent, and a group in which a hydrogen atom of a substituent in the "substituted alkenyl group" of the specific example group G4B is further substituted by a substituent.

· Unsubstituted alkenyl group (specific example group G4A):
   a vinyl group,
   an allyl group,
   a 1-butenyl group,
   a 2-butenyl group, and
   a 3-butenyl group.
· Substituted alkenyl group (specific example group G4B):
   a 1,3-butanedienyl group,
   a 1-methylvinyl group,
   a 1-methylallyl group,
   a 1,1-dimethylallyl group,
   a 2-methylally group, and
   a 1,2-dimethylallyl group.
· "Substituted or unsubstituted alkynyl group"

Specific examples of the "substituted or unsubstituted alkynyl group" described in this specification (specific example group G5) include the following unsubstituted alkynyl group (specific example group G5A) and the like. (Here, the unsubstituted alkynyl group refers to the case where the "substituted or unsubstituted alkynyl group" is an " alkynyl group unsubstituted by a substituent".). In this specification, in the case where simply referred as an "alkynyl group" includes both the "unsubstituted alkynyl group" and the "substituted alkynyl group."

The "substituted alkynyl group" means a group in which one or more hydrogen atoms in the "unsubstituted alkynyl group" are substituted by a substituent. Specific examples of the "substituted alkynyl group" include a group in which one or more hydrogen atoms in the following "unsubstituted alkynyl group" (specific example group G5A) are substituted by a substituent, and the like.

· Unsubstituted alkynyl group (specific example group G5A):
   an ethynyl group.
· "Substituted or unsubstituted cycloalkyl group"

Specific examples of the "substituted or unsubstituted cycloalkyl group" described in this specification (specific example group G6) include the following unsubstituted cycloalkyl group (specific example group G6A), the following substituted cycloalkyl group (specific example group G6B), and the like. (Here, the unsubstituted cycloalkyl group refers to the case where the "substituted or unsubstituted cycloalkyl group" is a " cycloalkyl group unsubstituted by a substituent", and the substituted cycloalkyl group refers to the case where the "substituted or unsubstituted cycloalkyl group" is a " cycloalkyl group substituted by a substituent".). In this specification, in the case where simply referred as a "cycloalkyl group" includes both the "unsubstituted cycloalkyl group" and the "substituted cycloalkyl group."

The "substituted cycloalkyl group" means a group in which one or more hydrogen atoms in the "unsubstituted cycloalkyl group" are substituted by a substituent. Specific examples of the "substituted cycloalkyl group" include a group in which one or more hydrogen atoms in the following "unsubstituted cycloalkyl group" (specific example group G6A) are substituted by a substituent, and examples of the following substituted cycloalkyl group (specific example group G6B), and the like. It should be noted that the examples of the "unsubstituted cycloalkyl group" and the examples of the "substituted cycloalkyl group" enumerated in this specification are mere examples, and the "substituted cycloalkyl group" in this specification includes a group in which one or more hydrogen atoms bonded with the carbon atom of the cycloalkyl group itself in the "substituted cycloalkyl group" of the specific example group G6B are substituted by a substituent, and a group in which a hydrogen atom of a substituent in the "substituted cycloalkyl group" of specific example group G6B is further substituted by a substituent.

· Unsubstituted cycloalkyl group (specific example group G6A):
   a cyclopropyl group,
   a cyclobutyl group,
   a cyclopentyl group,
   a cyclohexyl group,
   a 1-adamantyl group,
   a 2-adamantyl group,
   a 1-norbornyl group, and
   a 2-norbornyl group.
· Substituted cycloalkyl group (specific example group G6B):
   a 4-methylcyclohexyl group.
· "Group represented by -Si (R₉₀₁)(R₉₀₂)(R₉₀₃)"

Specific examples of the group represented by -Si(R₉₀₁)(R₉₀₂)(R₉₀₃) described in this specification (specific example group G7) include:

-Si(G1)(G1)(G1),

-Si(G1)(G2)(G2),

-Si(G1)(G1)(G2),

-Si(G2)(G2)(G2),

-Si(G3)(G3)(G3),

and

-Si(G6)(G6)(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

Plural G1's in -Si(G1)(G1)(G1) are the same or different.

Plural G2's in -Si(G1)(G2)(G2) are the same or different.

Plural G1's in -Si(G1)(G1)(G2) are the same or different.

Plural G2's in -Si(G2)(G2)(G2) are be the same or different.

Plural G3's in -Si(G3)(G3)(G3) are the same or different.

Plural G6's in -Si(G6)(G6)(G6) are be the same or different.

### · "Group represented by -O-(R₉₀₄)"

Specific examples of the group represented by -O-(R₉₀₄) in this specification (specific example group G8) include:

-O(G1),

-O(G2),

-O(G3), and

-O(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

### · "Group represented by -S-(R₉₀₅)"

Specific examples of the group represented by -S-(R₉₀₅) in this specification (specific example group G9) include:

-S(G1),

-S(G2),

-S(G3),

and

-S(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

### · "Group represented by -N(R₉₀₆)(R₉₀₇)"

Specific examples of the group represented by -N(R₉₀₆)(R₉₀₇) in this specification (specific example group G10) include:

-N(G1)(G1),

-N(G2)(G2),

-N(G1)(G2),

-N(G3)(G3),

and

-N(G6)(G6).

G1 is the "substituted or unsubstituted aryl group" described in the specific example group G1.

G2 is the "substituted or unsubstituted heterocyclic group" described in the specific example group G2.

G3 is the "substituted or unsubstituted alkyl group" described in the specific example group G3.

G6 is the "substituted or unsubstituted cycloalkyl group" described in the specific example group G6.

Plural G1's in -N(G1)(G1) are the same or different.

Plural G2's in -N(G2)(G2) are the same or different.

Plural G3's in -N(G3)(G3) are the same or different.

Plural G6's in -N(G6)(G6) are the same or different.

### · "Halogen atom"

Specific examples of the "halogen atom" described in this specification (specific example group G11) include a fluorine atom, a chlorine atom, a bromine atom, an iodine atom, and the like.

### · "Substituted or unsubstituted fluoroalkyl group"

The "substituted or unsubstituted fluoroalkyl group" described in this specification is a group in which at least one hydrogen atom bonded with a carbon atom constituting the alkyl group in the "substituted or unsubstituted alkyl group" is substituted by a fluorine atom, and includes a group in which all hydrogen atoms bonded with a carbon atom constituting the alkyl group in the "substituted or unsubstituted alkyl group" are substituted by a fluorine atom (a perfluoro group). The number of carbon atoms of the "unsubstituted fluoroalkyl group" is 1 to 50, preferably 1 to 30, more preferably 1 to 18, unless otherwise specified in this specification. The "substituted fluoroalkyl group" means a group in which one or more hydrogen atoms of the "fluoroalkyl group" are substituted by a substituent. The "substituted fluoroalkyl group" described in this specification also includes a group in which one or more hydrogen atoms bonded with a carbon atom of the alkyl chains in the "substituted fluoroalkyl group" are further substituted by a substituent, and a group in which one or more hydrogen atom of a substituent in the "substituted fluoroalkyl group" are further substituted by a substituent. Specific examples of the "unsubstituted fluoroalkyl group" include a group in which one or more hydrogen atoms in the "alkyl group" (specific group G3) are substituted by a fluorine atom, and the like.

### · "Substituted or unsubstituted haloalkyl group"

The "substituted or unsubstituted haloalkyl group" described in this specification is a group in which at least one hydrogen atom bonded with a carbon atom constituting the alkyl group in the "substituted or unsubstituted alkyl group" is substituted by a halogen atom, and also includes a group in which all hydrogen atoms bonded with a carbon atom constituting the alkyl group in the "substituted or unsubstituted alkyl group" are substituted by a halogen atom. The number of carbon atoms of the "unsubstituted haloalkyl group" is 1 to 50, preferably 1 to 30, more preferably 1 to 18, unless otherwise specified in this specification. The "substituted haloalkyl group" means a group in which one or more hydrogen atoms of the "haloalkyl group" are substituted by a substituent. The "substituted haloalkyl group" described in this specification also includes a group in which one or more hydrogen atoms bonded with a carbon atom of the alkyl chain in the "substituted haloalkyl group" are further substituted by a substituent, and a group in which one or more hydrogen atoms of a substituent in the "substituted haloalkyl group" are further substituted by a substituent. Specific examples of the "unsubstituted haloalkyl group" include a group in which one or more hydrogen atoms in the "alkyl group" (specific example group G3) are substituted by a halogen atom, and the like. A haloalkyl group is sometimes referred to as an alkyl halide group.

### · "Substituted or unsubstituted alkoxy group"

Specific examples of the "substituted or unsubstituted alkoxy group" described in this specification include a group represented by -O(G3), wherein G3 is the "substituted or unsubstituted alkyl group" described in the specific example group G3. The number of carbon atoms of the "unsubstituted alkoxy group" is 1 to 50, preferably 1 to 30, more preferably 1 to 18, unless otherwise specified in this specification.

### · "Substituted or unsubstituted alkylthio group"

Specific examples of the "substituted or unsubstituted alkylthio group" described in this specification include a group represented by -S(G3), wherein G3 is the "substituted or unsubstituted alkyl group" described in the specific example group G3. The number of carbon atoms of the "unsubstituted alkylthio group" is 1 to 50, preferably 1 to 30, more preferably 1 to 18, unless otherwise specified in this specification.

### · "Substituted or unsubstituted aryloxy group"

Specific examples of the "substituted or unsubstituted aryloxy group" described in this specification include a group represented by -O(G1), wherein G1 is the "substituted or unsubstituted aryl group" described in the specific example group G1. The number of ring carbon atoms of the "unsubstituted aryloxy group" is 6 to 50, preferably 6 to 30, more preferably 6 to 18, unless otherwise specified in this specification.

### · "Substituted or unsubstituted arylthio group"

Specific examples of the "substituted or unsubstituted arylthio group" described in this specification include a group represented by -S(G1), wherein G1 is a "substituted or unsubstituted aryl group" described in the specific example group G1. The number of ring carbon atoms of the "unsubstituted arylthio group" is 6 to 50, preferably 6 to 30, more preferably 6 to 18, unless otherwise specified in this specification.

### · "Substituted or unsubstituted trialkylsilyl group"

Specific examples of the "trialkylsilyl group" described in this specification include a group represented by -Si(G3)(G3)(G3), where G3 is the "substituted or unsubstituted alkyl group" described in the specific example group G3. Plural G3's in -Si(G3)(G3)(G3) are the same or different. The number of carbon atoms in each alkyl group of the "trialkylsilyl group" is 1 to 50, preferably 1 to 20, more preferably 1 to 6, unless otherwise specified in this specification.

### · "Substituted or unsubstituted aralkyl group"

Specific examples of the "substituted or unsubstituted aralkyl group" described in this specification is a group represented by -(G3)-(G1), wherein G3 is the "substituted or unsubstituted alkyl group" described in the specific example group G3, and G1 is the "substituted or unsubstituted aryl group" described in the specific example group G1. Therefore, the "aralkyl group" is a group in which a hydrogen atom of the "alkyl group" is substituted by an "aryl group" as a substituent, and is one form of the "substituted alkyl group." The "unsubstituted aralkyl group" is the "unsubstituted alkyl group" substituted by the "unsubstituted aryl group", and the number of carbon atoms of the "unsubstituted aralkyl group" is 7 to 50, preferably 7 to 30, more preferably 7 to 18, unless otherwise specified in this specification.

Specific examples of the "substituted or unsubstituted aralkyl group" include a benzyl group, a 1-phenylethyl group, a 2-phenylethyl group, a 1-phenylisopropyl group, a 2-phenylisopropyl group, a phenyl-t-butyl group, an α-naphthylmethyl group, a 1-α-naphthylethyl group, a 2-α-naphthylethyl group, a 1-α-naphthylisopropyl group, a 2-α-naphthylisopropyl group, a β-naphthylmethyl group, a 1-β-naphthylethyl group, a 2-β-naphthylethyl group, a 1-β-naphthylisopropyl group, a 2-β-naphthylisopropyl group, and the like.

Unless otherwise specified in this specification, examples of the substituted or unsubstituted aryl group described in this specification preferably include a phenyl group, a p-biphenyl group, a m-biphenyl group, an o-biphenyl group, a p-terphenyl-4-yl group, a p-terphenyl-3-yl group, a p-terphenyl-2-yl group, a m-terphenyl-4-yl group, a m-terphenyl-3-yl group, a m-terphenyl-2-yl group, an o-terphenyl-4-yl group, an o-terphenyl-3-yl group, an o-terphenyl-2-yl group, a 1-naphthyl group, a 2-naphthyl group, an anthryl group, a phenanthryl group, a pyrenyl group, a chrysenyl group, a triphenylenyl group, a fluorenyl group, a 9,9'-spirobifluorenyl group, 9,9-dimethylfluorenyl group, 9,9-diphenylfluorenyl group, and the like.

Unless otherwise specified in this specification, examples of the substituted or unsubstituted heterocyclic groups described in this specification preferably include a pyridyl group, a pyrimidinyl group, a triazinyl group, a quinolyl group, an isoquinolyl group, a quinazolinyl group, a benzimidazolyl group, a phenanthrolinyl group, a carbazolyl group (a 1-carbazolyl group, a 2-carbazolyl group, a 3-carbazolyl group, a 4-carbazolyl group, or a 9-carbazolyl group), a benzocarbazolyl group, an azacarbazolyl group, a diazacarbazolyl group, a dibenzofuranyl group, a naphthobenzofuranyl group, an azadibenzofuranyl group, a diazadibenzofuranyl group, a dibenzothiophenyl group, a naphthobenzothiophenyl group, an azadibenzothiophenyl group, a diazadibenzothiophenyl group, a (9-phenyl)carbazolyl group (a (9-phenyl)carbazol-1-yl group, a (9-phenyl)carbazol-2-yl group, a (9-phenyl)carbazol-3-yl group, or a (9-phenyl)carbazol-4-yl group), a (9-biphenylyl)carbazolyl group, a (9-phenyl)phenylcarbazolyl group, a diphenylcarbazol-9-yl group, a phenylcarbazol-9-yl group, a phenyltriazinyl group, a biphenylyltriazinyl group, a diphenyltriazinyl group, a phenyldibenzofuranyl group, a phenyldibenzothiophenyl group, and the like.

In this specification, the carbazolyl group is specifically any of the following groups, unless otherwise specified in this specification.

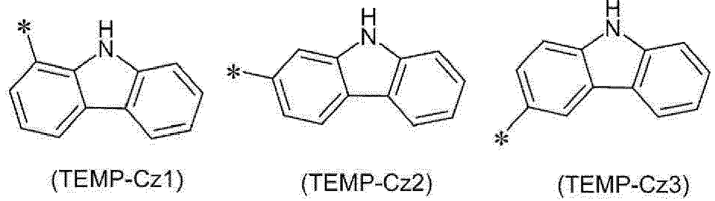

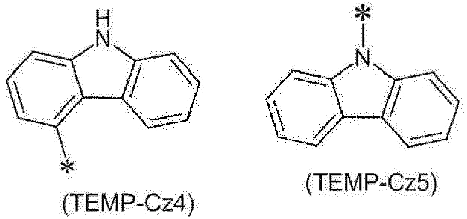

In this specification, the (9-phenyl)carbazolyl group is specifically any of the following groups, unless otherwise specified in this specification.

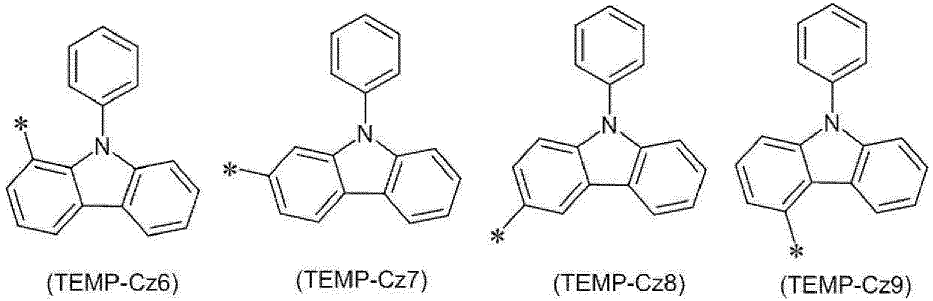

In the general formulas (TEMP-Cz1) to (TEMP-Cz9), * represents a bonding position.

In this specification, the dibenzofuranyl group and the dibenzothiophenyl group are specifically any of the following groups, unless otherwise specified in this specification.

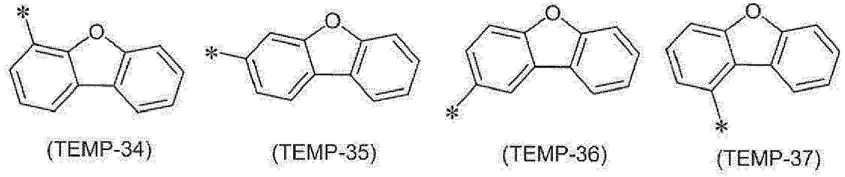

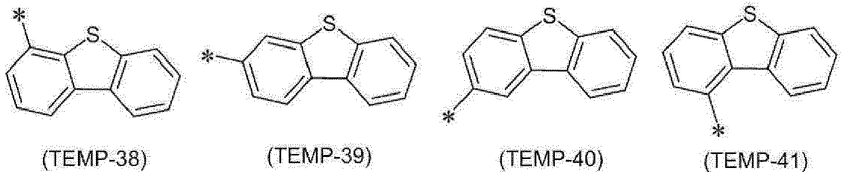

In the general formulas (TEMP-34) to (TEMP-41), * represents a bonding position.

The substituted or unsubstituted alkyl group described in this specification is preferably a methyl group, an ethyl group, a propyl group, an isopropyl group, a n-butyl group, an isobutyl group, a t-butyl group, or the like, unless otherwise specified in this specification.

### · "Substituted or unsubstituted arylene group"

The "substituted or unsubstituted arylene group" described in this specification is a divalent group derived by removing one hydrogen atom on the aryl ring of the "substituted or unsubstituted aryl group", unless otherwise specified. Specific examples of the "substituted or unsubstituted arylene group" (specific example group G12) include a divalent group derived by removing one hydrogen atom on the aryl ring of the "substituted or unsubstituted aryl group" described in the specific example group G1, and the like.

### · "Substituted or unsubstituted divalent heterocyclic group"

The "substituted or unsubstituted divalent heterocyclic group" described in this specification is a divalent group derived by removing one hydrogen atom on the heterocyclic ring of the "substituted or unsubstituted heterocyclic group", unless otherwise specified. Specific examples of the "substituted or unsubstituted divalent heterocyclic group" (specific example group G13) include a divalent group derived by removing one hydrogen atom on the heterocyclic ring of the "substituted or unsubstituted heterocyclic group" described in the specific example group G2, and the like.

### · "Substituted or unsubstituted alkylene group"

The "substituted or unsubstituted alkylene group" described in this specification is a divalent group derived by removing one hydrogen atom on the alkyl chain of the "substituted or unsubstituted alkyl group", unless otherwise specified. Specific examples of the "substituted or unsubstituted alkylene group" (specific example group G14) include a divalent group derived by removing one hydrogen atom on the alkyl chain of the "substituted or unsubstituted alkyl group" described in the specific example group G3, and the like.

The substituted or unsubstituted arylene group described in this specification is preferably any group of the following general formulas (TEMP-42) to (TEMP-68), unless otherwise specified in this specification.

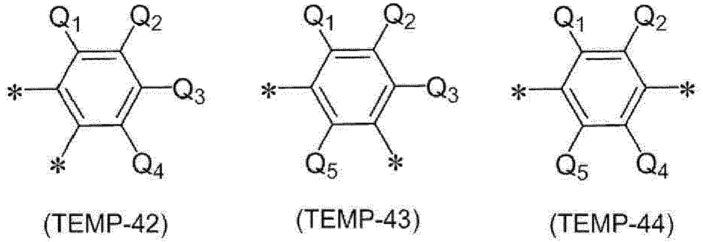

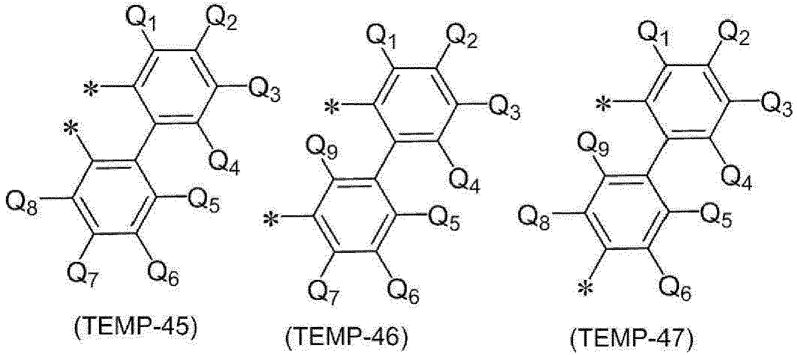

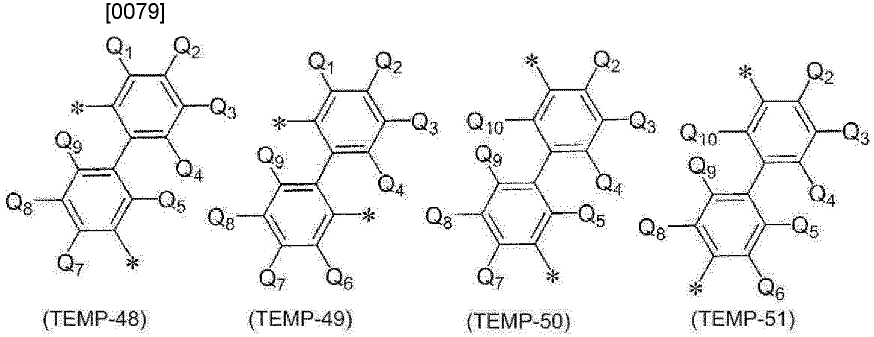

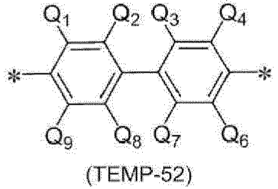

In the general formulas (TEMP-42) to (TEMP-52), Q₁ to Q₁₀ are independently a hydrogen atom or a substituent.

In the general formulas (TEMP-42) to (TEMP-52), * represents a bonding position.

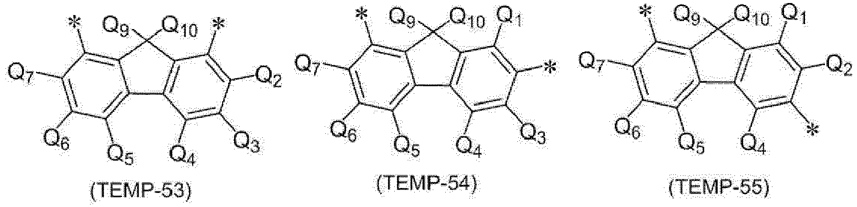

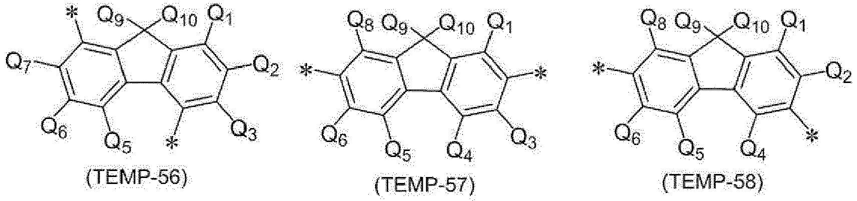

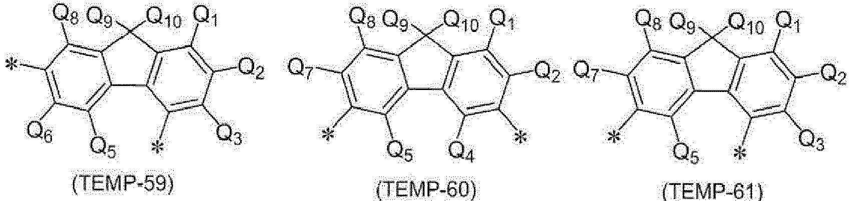

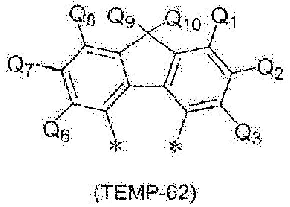

In the general formulas (TEMP-53) to (TEMP-62), Q₁ to Q₁₀ are independently a hydrogen atom or a substituent.

Q₉ and Q₁₀ may be bonded with each other via a single bond to form a ring.

In the general formulas (TEMP-53) to (TEMP-62), * represents a bonding position.

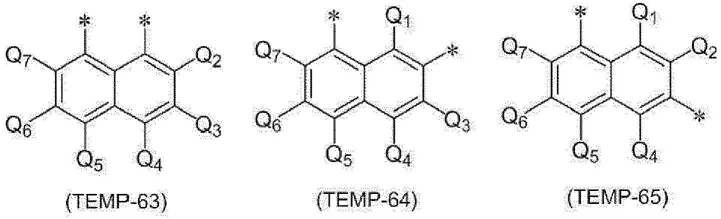

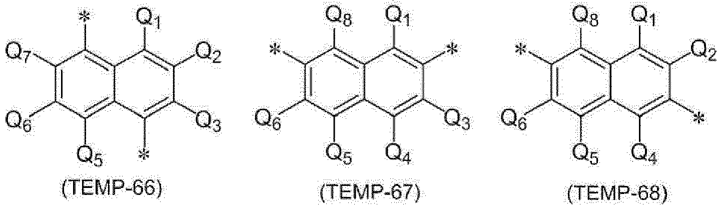

In the general formulas (TEMP-63) to (TEMP-68), Q₁ to Q₈ are independently a hydrogen atom or a substituent.

In the general formulas (TEMP-63) to (TEMP-68), * represents a bonding position.

The substituted or unsubstituted divalent heterocyclic group described in this specification is preferably any group of the following general formulas (TEMP-69) to (TEMP-102), unless otherwise specified in this specification.

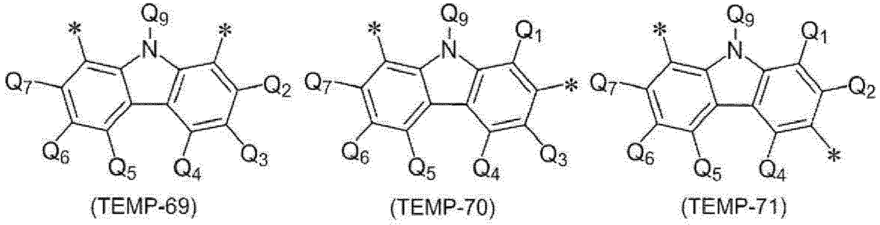

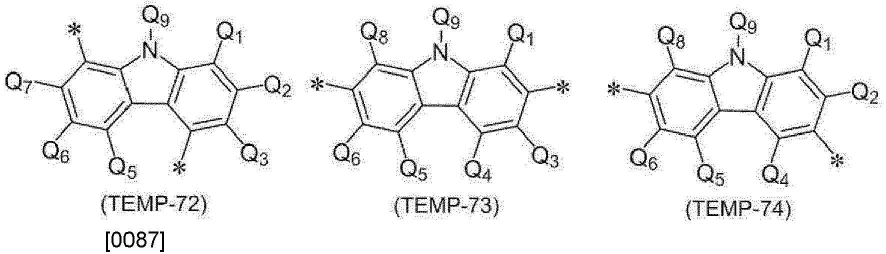

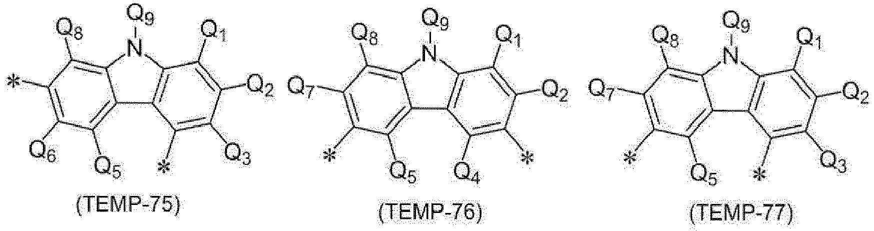

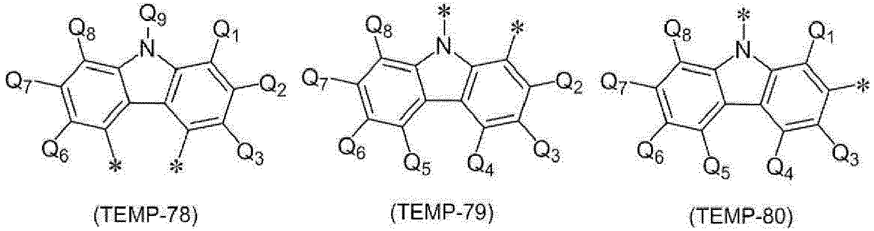

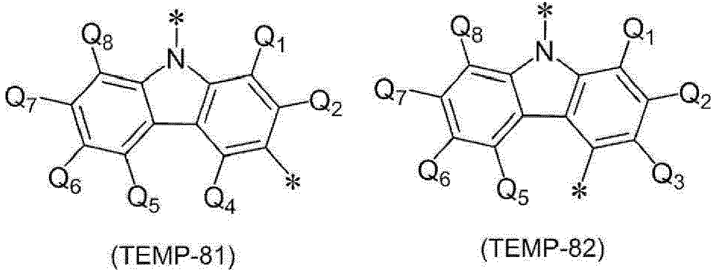

In the general formulas (TEMP-69) to (TEMP-82), Q₁ to Q₉ are independently a hydrogen atom or a substituent.

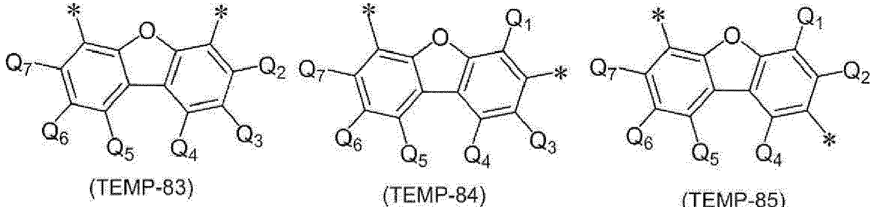

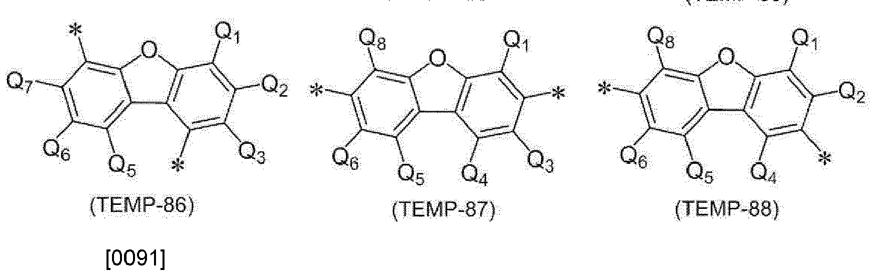

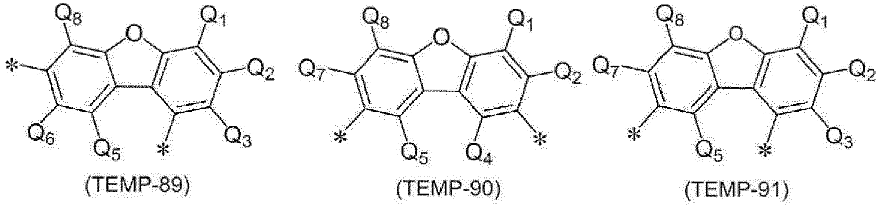

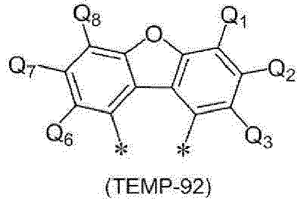

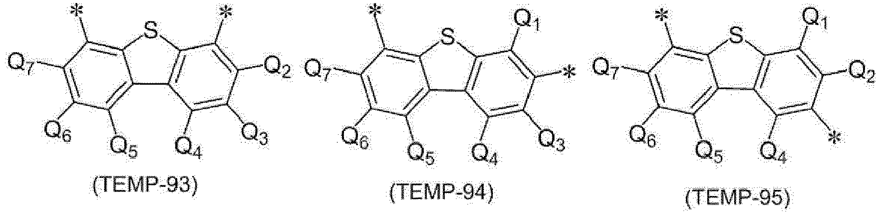

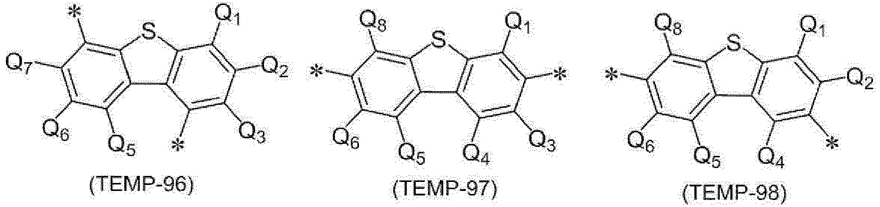

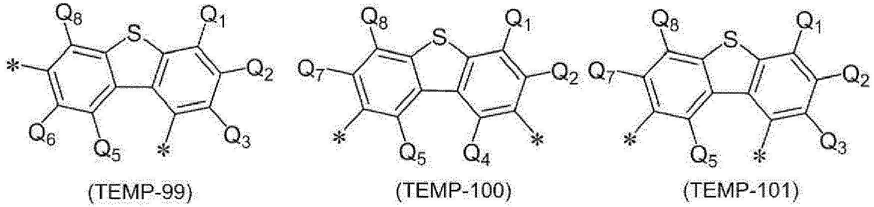

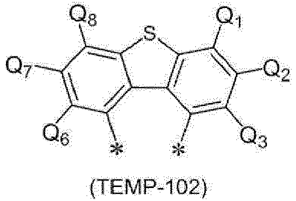

In the general formulas (TEMP-83) to (TEMP-102), Q₁ to Q₈ are independently a hydrogen atom or a substituent.

The above is the explanation of the "Substituent described in this specification."

### · "The case where bonded with each other to form a ring"

In this specification, the case where "one or more sets of adjacent two or more form a substituted or unsubstituted monocycle by bonding with each other, form a substituted or unsubstituted fused ring by bonding with each other, or do not bond with each other" means the case where "one or more sets of adjacent two or more form a substituted or unsubstituted monocycle by bonding with each other"; the case where "one or more sets of adjacent two or more form a substituted or unsubstituted fused ring by bonding with each other"; and the case where "one or more sets of adjacent two or more do not bond with each other."

The case where "one or more sets of adjacent two or more form a substituted or unsubstituted monocycle by bonding with each other" and the case where "one or more sets of adjacent two or more form a substituted or unsubstituted fused ring by bonding with each other" in this specification (these cases may be collectively referred to as "the case where forming a ring by bonding with each other") will be described below. The case of an anthracene compound represented by the following general formula (TEMP-103) in which the mother skeleton is an anthracene ring will be described as an example.

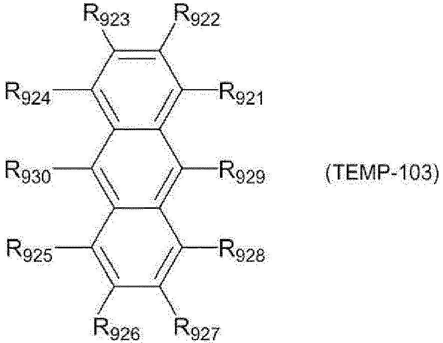

For example, in the case where "one or more sets of adjacent two or more among R₉₂₁ to R₉₃₀ form a ring by bonding with each other", the one set of adjacent two includes a pair of R₉₂₁ and R₉₂₂, a pair of R₉₂₂ and R₉₂₃, a pair of R₉₂₃ and R₉₂₄, a pair of R₉₂₄ and R₉₃₀, a pair of R₉₃₀ and R₉₂₅, a pair of R₉₂₅ and R₉₂₆, a pair of R₉₂₆ and R₉₂₇, a pair of R₉₂₇ and R₉₂₈, a pair of R₉₂₈ and R₉₂₉, and a pair of R₉₂₉ and R₉₂₁.

The "one or more sets" means that two or more sets of the adjacent two or more sets may form a ring at the same time. For example, R₉₂₁ and R₉₂₂ form a ring Q_{A} by bonding with each other, and at the same, time R₉₂₅ and R₉₂₆ form a ring Q_{B} by bonding with each other, the anthracene compound represented by the general formula (TEMP-103) is represented by the following general formula (TEMP-104).

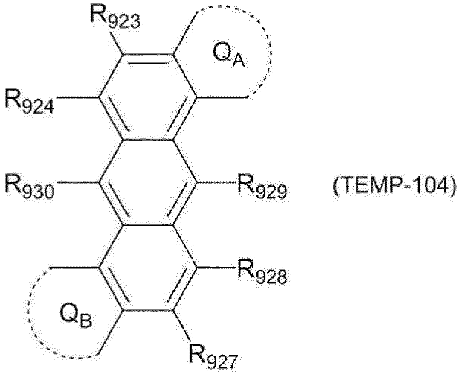

The case where the "set of adjacent two or more" form a ring includes not only the case where the set (pair) of adjacent "two" is bonded with as in the above-mentioned examples, but also the case where the set of adjacent "three or more" are bonded with each other. For example, it means the case where R₉₂₁ and R₉₂₂ form a ring Q_{A} by bonding with each other, and R₉₂₂ and R₉₂₃ form a ring Qc by bonding with each other, and adjacent three (R₉₂₁, R₉₂₂ and R₉₂₃) form rings by bonding with each other and together fused to the anthracene mother skeleton. In this case, the anthracene compound represented by the general formula (TEMP-103) is represented by the following general formula (TEMP-105). In the following general formula (TEMP-105), the ring Q_{A} and the ring Qc share R₉₂₂.

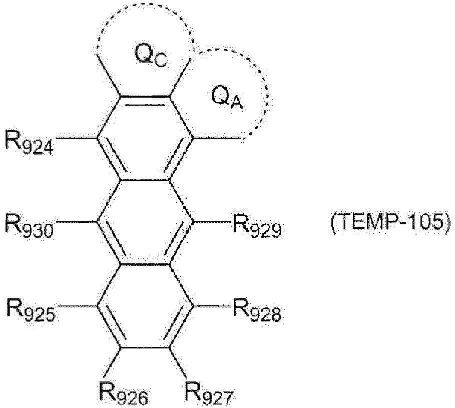

The "monocycle" or "fused ring" formed may be a saturated ring or an unsaturated ring, as a structure of the formed ring alone. Even when the "one pair of adjacent two" forms a "monocycle" or a "fused ring" , the "monocycle" or the "fused ring" may form a saturated ring or an unsaturated ring. For example, the ring Q_{A} and the ring Q_{B} formed in the general formula (TEMP-104) are independently a "monocycle" or a "fused ring." The ring Q_{A} and the ring Qc formed in the general formula (TEMP-105) are "fused ring." The ring Q_{A} and ring Qc of the general formula (TEMP-105) are fused ring by fusing the ring Q_{A} and the ring Qc together. When the ring Q_{A} of the general formula (TMEP-104) is a benzene ring, the ring Q_{A} is a monocycle. When the ring Q_{A} of the general formula (TMEP-104) is a naphthalene ring, the ring Q_{A} is a fused ring.

The "unsaturated ring" means an aromatic hydrocarbon ring or an aromatic heterocyclic ring. The "saturated ring" means an aliphatic hydrocarbon ring, or a non-aromatic heterocyclic ring.

Specific examples of the aromatic hydrocarbon ring include a structure in which the group listed as a specific example in the specific example group G1 is terminated by a hydrogen atom.

Specific examples of the aromatic heterocyclic ring include a structure in which the aromatic heterocyclic group listed as a specific example in the example group G2 is terminated by a hydrogen atom.

Specific examples of the aliphatic hydrocarbon ring include a structure in which the group listed as a specific example in the specific example group G6 is terminated by a hydrogen atom.

The term "to form a ring" means forming a ring only with plural atoms of the mother skeleton, or with plural atoms of the mother skeleton and one or more arbitrary elements in addition. For example, the ring Q_{A} shown in the general formula (TEMP-104), which is formed by bonding R₉₂₁ and R₉₂₂ with each other, is a ring formed from the carbon atom of the anthracene skeleton with which R₉₂₁ is bonded, the carbon atom of the anthracene skeleton with which R₉₂₂ is bonded, and one or more arbitrary elements. For example, in the case where the ring Q_{A} is formed with R₉₂₁ and R₉₂₂, when a monocyclic unsaturated ring is formed with the carbon atom of the anthracene skeleton with which R₉₂₁ is bonded, the carbon atom of the anthracene skeleton with which R₉₂₂ is bonded, and four carbon atoms, the ring formed with R₉₂₁ and R₉₂₂ is a benzene ring.

Here, the "arbitrary element" is preferably at least one element selected from the group consisting of a carbon element, a nitrogen element, an oxygen element, and a sulfur element, unless otherwise specified in this specification. In the arbitrary element (for example, a carbon element or a nitrogen element), a bond which does not form a ring may be terminated with a hydrogen atom or the like, or may be substituted with "arbitrary substituent" described below. When an arbitrary element other than a carbon element is contained, the ring formed is a heterocyclic ring.

The number of "one or more arbitrary element(s)" constituting a monocycle or a fused ring is preferably 2 or more and 15 or less, more preferably 3 or more and 12 or less, and still more preferably 3 or more and 5 or less, unless otherwise specified in this specification.

The "monocycle" is preferable among the "monocycle" and the "fused ring", unless otherwise specified in this specification.

The "unsaturated ring" is preferable among the "saturated ring" and the "unsaturated ring", unless otherwise specified in this specification.

Unless otherwise specified in this specification, the "monocycle " is preferably a benzene ring.

Unless otherwise specified in this specification, the "unsaturated ring" is preferably a benzene ring.

Unless otherwise specified in this specification, when "one or more sets of adjacent two or more" are "bonded with each other to form a substituted or unsubstituted monocycle" or "bonded with each other to form a substituted or unsubstituted fused ring", this specification, one or more sets of adjacent two or more are preferably bonded with each other to form a substituted or unsubstituted "unsaturated ring" from plural atoms of the mother skeleton and one or more and 15 or less elements which is at least one kind selected from a carbon elements, a nitrogen element, an oxygen element, and a sulfur element.

The substituent in the case where the above-mentioned "monocycle" or "fused ring" has a substituent is, for example, an "arbitrary substituent" described below. Specific examples of the substituent which the above-mentioned "monocycle" or "fused ring" has include the substituent described above in the "Substituent described in this specification" section.

The substituent in the case where the above-mentioned "saturated ring" or "unsaturated ring" has a substituent is, for example, an "arbitrary substituent" described below. Specific examples of the substituent which the above-mentioned "monocycle" or "fused ring" has include the substituent described above in the "Substituent described in this specification" section.

The foregoing describes the case where "one or more sets of adjacent two or more form a substituted or unsubstituted monocycle by bonding with each other" and the case where "one or more sets of adjacent two or more form a substituted or unsubstituted fused ring by bonding with each other" (the case where "forming a ring by bonding with each other").

### · Substituent in the case of "substituted or unsubstituted"

In one embodiment in this specification, the substituent (in this specification, sometimes referred to as an "arbitrary substituent") in the case of "substituted or unsubstituted" is, for example, a group selected from the group consisting of:
an unsubstituted alkyl group including 1 to 50 carbon atoms,
an unsubstituted alkenyl group including 2 to 50 carbon atoms,
an unsubstituted alkynyl group including 2 to 50 carbon atoms,
an unsubstituted cycloalkyl group including 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group,
an unsubstituted aryl group including 6 to 50 ring carbon atoms, and
an unsubstituted heterocyclic group including 5 to 50 ring atoms,
wherein, R₉₀₁ to R₉₀₇ are independently
a hydrogen atom,
a substituted or unsubstituted alkyl group including 1 to 50 carbon atoms,
a substituted or unsubstituted cycloalkyl group including 3 to 50 ring carbon atoms,
a substituted or unsubstituted aryl group including 6 to 50 ring carbon atoms, or
a substituted or unsubstituted heterocyclic group including 5 to 50 ring atoms.

When two or more R₉₀₁'s are present, the two or more R₉₀₁'s may be the same or different.

When two or more R₉₀₂'s are present, the two or more R₉₀₂'s may be the same or different.

When two or more R₉₀₃'s are present, the two or more R₉₀₃'s may be the same or different.

When two or more R₉₀₄'s are present, the two or more R₉₀₄'s may be the same or different.

When two or more R₉₀₅'s are present, the two or more R₉₀₅'s may be the same or different.

When two or more R₉₀₆'s are present, the two or more R₉₀₆'s may be the same or different.

When two or more R₉₀₇'s are present, the two or more R₉₀₇'s may be the same or different.

In one embodiment, the substituent in the case of "substituted or unsubstituted" is a group selected from the group consisting of:
an alkyl group including 1 to 50 carbon atoms,
an aryl group including 6 to 50 ring carbon atoms, and
a heterocyclic group including 5 to 50 ring atoms.

In one embodiment, the substituent in the case of "substituted or unsubstituted" is a group selected from the group consisting of:
an alkyl group including 1 to 18 carbon atoms,
an aryl group including 6 to 18 ring carbon atoms, and
a heterocyclic group including 5 to 18 ring atoms.

Specific examples of each of the arbitrary substituents include specific examples of substituent described in the section "Substituent described in this specification" above.

Unless otherwise specified in this specification, adjacent arbitrary substituents may form a "saturated ring" or an "unsaturated ring", preferably form a substituted or unsubstituted saturated 5-membered ring, a substituted or unsubstituted saturated 6-membered ring, a substituted or unsubstituted unsaturated 5-membered ring, or a substituted or unsubstituted unsaturated 6-membered ring, more preferably form a benzene ring.

Unless otherwise specified in this specification, the arbitrary substituent may further have a substituent. The substituent which the arbitrary substituent further has is the same as that of the above-mentioned arbitrary substituent.

In this specification, the numerical range represented by "AA to BB" means the range including the numerical value AA described on the front side of "AA to BB" as the lower limit and the numerical value BB described on the rear side of "AA to BB" as the upper limit.

### [Novel Compound]

A compound according to an aspect of the invention is a compound represented by the formulas (9-2) and (9-3) as disclosed below.

When the compound according to an aspect of the invention as set forth below and in the claims is used, an organic EL device having a long lifetime can be fabricated.

The expression "one or more sets of adjacent two or more of R₁ to R₁₆ may form a substituted or unsubstituted, saturated or unsaturated ring" will be described.

Examples of the "one or more sets of adjacent two or more of R₁ to R₁₆ " include, for example, a combination of R₁ and R₂; R₂ and R₃; R₃ and R₄; R₅ and R₆; R₆ and R₇; R₁, R₂, and R₃; and the like.

The substituent in "substituted" of "substituted or unsubstituted" for the saturated or unsaturated ring is the same as the substituent in the case of "substituted or unsubstituted" described later.

The term "saturated or unsaturated ring" refers to, for example, in the case when R₁ and R₂ form a ring, a ring formed by a carbon atom with which R₁ is bonded, a carbon atom with which R₂ is bonded, and one or more arbitrary atoms. Specifically, in the case when R₁ and R₂ form a ring, the ring formed by R₁ and R₂ is a benzene ring when a carbon atom with which R₁ is bonded, a carbon atom with which R₂ is bonded, and four carbon atoms form an unsaturated ring.

The "arbitrary atom" is preferably a C atom, an N atom, an O atom, or an S atom. In the arbitrary atom (e.g., in the case of a C atom or an N atom), a bonding hand which does not form a ring may be terminated with a hydrogen atom or the like.

The "one or more arbitrary atoms" is preferably 2 or more and 15 or less, more preferably 3 or more and 12 or less, and further preferably 3 or more and 5 or less arbitrary atoms.

Hereafter, the phrase "one or more sets of adjacent two or more of X to Y may form a substituted or unsubstituted, saturated or unsaturated ring" is equivalent to replacing X with R₁ and Y with R₁₆.

In one embodiment, the invention relates to a compound represented by the following formula (9-2): 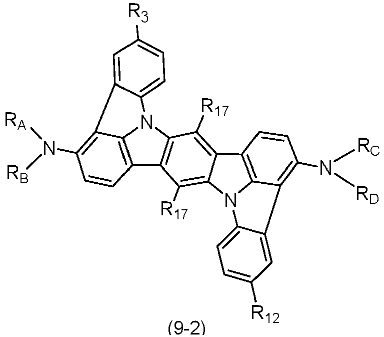 wherein in the formula (9-2),
R3 and R12 are independently a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), - C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms;
R17 is a hydrogen atom, a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R31)(R32)(R33), -C(=O)R34, -COOR35, -N(R36)(R37), a halogen atom, a cyano group, a nitro group, a substituted or unsubstituted aryl group including 6 to 50 ring carbon atoms, or a substituted or unsubstituted monovalent heterocyclic group including 5 to 50 ring atoms; two R₁₇s may be the same as or different from each other; and
R_{A}, R_{B}, R_{C} and R_{D} are independently a substituted or unsubstituted alkyl group including 1 to 50 carbon atoms, a substituted or unsubstituted aryl group including 6 to 50 ring carbon atoms, or a substituted or unsubstituted monovalent heterocyclic group including 5 to 50 ring atoms.

In one embodiment, the invention relates to a compound represented by following formula (9-3): 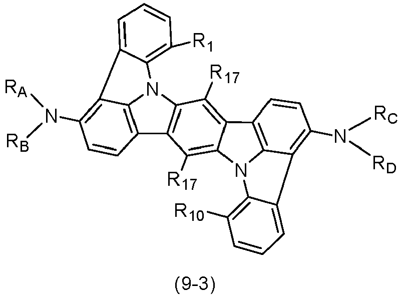 wherein in the formula (9-3),
R1 and R10 are independently a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), - C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms;
R17 is a hydrogen atom, a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R31)(R32)(R33), -C(=O)R34, -COOR35, -N(R36)(R37), a halogen atom, a cyano group, a nitro group, a substituted or unsubstituted aryl group including 6 to 50 ring carbon atoms, or a substituted or unsubstituted monovalent heterocyclic group including 5 to 50 ring atoms;
two R₁₇s may be the same as or different from each other; and
R_{A}, R_{B}, R_{C} and R_{D} are independently a substituted or unsubstituted alkyl group including 1 to 50 carbon atoms, a substituted or unsubstituted aryl group including 6 to 50 ring carbon atoms, or a substituted or unsubstituted monovalent heterocyclic group including 5 to 50 ring atoms

Usually, in the compounds represented by the formulas (9-2), and (9-3), R_{A} and R_{B}, and R_{C} and R_{D} do not form a ring by bonding with R₁ to R₁₇ if present.

In one embodiment, R_{A}, R_{B}, R_{C}, and R_{D} are independently a substituted or unsubstituted aryl group including 6 to 50 ring carbon atoms or a substituted or unsubstituted monovalent heterocyclic group including 5 to 50 ring atoms.

In one embodiment, in the case when R_{A}, R_{B}, R_{C}, and R_{D} are independently a substituted or unsubstituted aryl group including 6 to 50 ring carbon atoms or a substituted or unsubstituted monovalent heterocyclic group including 5 to 50 ring atoms, all or a part of the hydrogen atoms contained in R_{A}, R_{B}, R_{C}, and R_{D} are deuterium atoms.

In one embodiment, R_{A}, R_{B}, R_{C}, and R_{D} are independently a substituted or unsubstituted phenyl group. All or a part of the hydrogen atoms contained in the phenyl group may be deuterium atoms.

In one embodiment, examples of the substituent of the aryl group including 6 to 50 ring carbon atoms (for example, a phenyl group) or the monovalent heterocyclic group including 5 to 50 ring atoms include an alkyl group including 1 to 5 carbon atoms, an aryl group including 6 to 12 carbon atoms (e.g., a phenyl group or a naphthyl group), and both of an alkyl group including 1 to 5 carbon atoms and an aryl group including 6 to 12 carbon atoms may be substituted for a phenyl group, for example. In addition, all or a part of the hydrogen atoms contained in the aryl group including 6 to 12 carbon atoms may be deuterium atoms.

In one embodiment, R_{A}, R_{B}, R_{C}, and R_{D} are independently a phenyl group substituted with an alkyl group including 1 to 5 carbon atoms, a phenyl group substituted with an aryl group including 6 to 12 carbon atoms, a phenyl group substituted with an alkyl group including 1 to 5 carbon atoms and an aryl group including 6 to 12 carbon atoms, or an unsubstituted phenyl group.

In one embodiment, examples of the combination of R_{A} and R_{B} (R_{A}, R_{B}) include, for example, (an unsubstituted phenyl group, an unsubstituted phenyl group), (an unsubstituted phenyl group, a phenyl group substituted with an alkyl group including 1 to 5 carbon atoms), (a phenyl group substituted with an alkyl group including 1 to 5 carbon atoms, a phenyl group substituted with an alkyl group including 1 to 5 carbon atoms), (an unsubstituted phenyl group, a phenyl group substituted with an aryl group including 6 to 12 carbon atoms), (a phenyl group substituted with an alkyl group including 1 to 5 carbon atoms, a phenyl group substituted with an aryl group including 6 to 12 carbon atoms), (a phenyl group substituted with an aryl group including 6 to 12 carbon atoms, a phenyl group substituted with an aryl group including 6 to 12 carbon atoms), (an unsubstituted phenyl group, a phenyl group substituted with an alkyl group including 1 to 5 carbon atoms and an aryl group including 6 to 12 carbon atoms, or an unsubstituted phenyl group), and the like.

Examples of the combination of Rc and R_{D} (Rc, R_{D}) include the same combinations as the combination of R_{A} and R_{B} (R_{A}, R_{B}).

In one embodiment, R₁₇s are independently a hydrogen atom.

The substituent in the case of "substituted or unsubstituted" in the compound represented by the formulas (9-2) and (9-3) is selected from the group consisting of an alkyl group including 1 to 50 carbon atoms, a haloalkyl group including 1 to 50 carbon atoms, an alkenyl group including 2 to 50 carbon atoms, an alkynyl group including 2 to 50 carbon atoms, a cycloalkyl group including 3 to 50 ring carbon atoms, an alkoxy group including 1 to 50 carbon atoms, an alkylthio group including 1 to 50 carbon atoms, an aryloxy group including 6 to 50 ring carbon atoms, and an arylthio group including 6 to 50 ring carbon atoms, an aralkyl group including 7 to 50 carbon atoms, - Si(R₄₁)(R₄₂)(R₄₃), -C(=O)R₄₄, -COOR₄₅, -S(=O)₂R₄₆, -P(=O)(R₄₇)(R₄₈), -Ge(R₄₉)(R₅₀)(R₅₁), - N(R₅₂)(R₅₃), a hydroxy group, a halogen atom, a cyano group, a nitro group, an aryl group including 6 to 50 ring carbon atoms, and monovalent heterocyclic group including 5 to 50 ring atoms; and R₄₁ to R₅₃ are independently a hydrogen atom, an alkyl group including 1 to 50 carbon atoms, an aryl group including 6 to 50 ring carbon atoms, or a monovalent heterocyclic group including 5 to 50 ring atoms; when two or more of each of R₄₁ to R₅₃ are present, the two or more of each of R₄₁ to R₅₃ may be the same as or different from each other.

In one embodiment, the substituent in the case of "substituted or unsubstituted" in the compound represented by the formulas (9-2) and (9-3) is an alkyl group including 1 to 50 carbon atoms, an aryl group including 6 to 50 ring carbon atoms, and a monovalent heterocyclic group including 5 to 50 ring atoms.

In one embodiment, the substituent in the case of "substituted or unsubstituted" in the compound represented by the formulas (9-2) and (9-3)is selected from the group consisting of an alkyl group including 1 to 30 carbon atoms, an aryl group including 6 to 30 ring carbon atoms, and a monovalent heterocyclic group including 5 to 30 ring atoms.

In one embodiment, the substituent in the case of "substituted or unsubstituted" in the compound represented by the formulas (9-2) and (9-3) is selected from the group consisting of an alkyl group including 1 to 18 carbon atoms, an aryl group including 6 to 18 ring carbon atoms, and a monovalent heterocyclic group including 5 to 18 ring atoms.

Specific examples of each substituent of the compound represented by the formulas(9-2) and (9-3), the substituent in the case of "substituted or unsubstituted," and the halogen atom are the same as those described above, respectively.

The compound represented by the formulas (9-2) and (9-3)can be synthesized in accordance with the synthetic methods described in Examples by using known alternative reactions or raw materials tailored to the target compound.

Specific examples of the compound represented by the formulas (9-2) and (9-3) will be described below, but these are merely illustrative, and the compound represented by the formulas (9-2) and (9-3)are not limited to the following specific examples. In the following specific examples, Me represents a methyl group, and Ph represents a phenyl group.

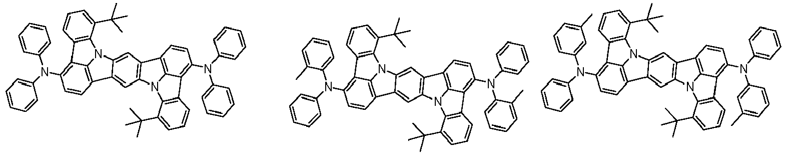

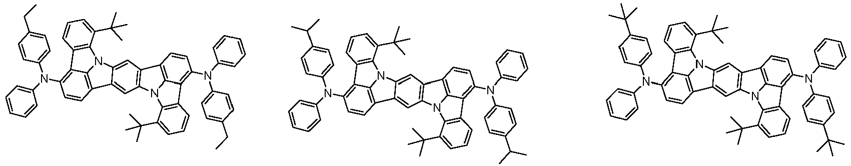

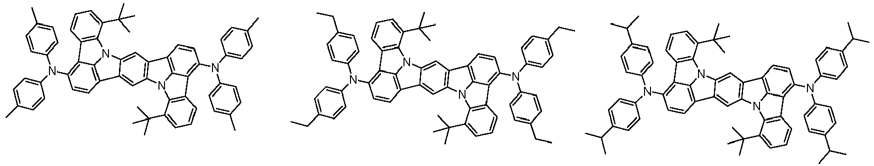

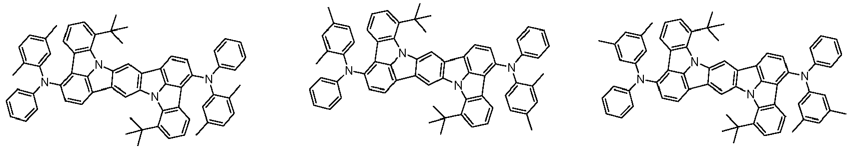

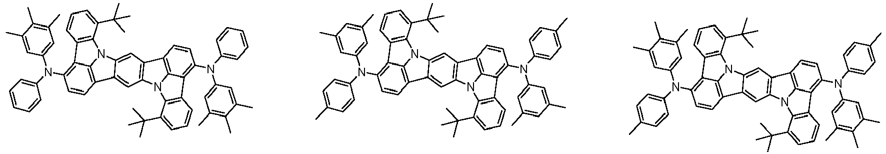

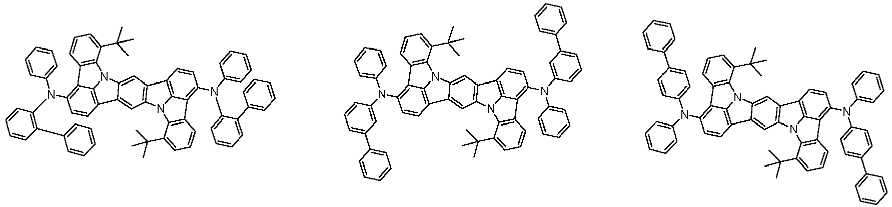

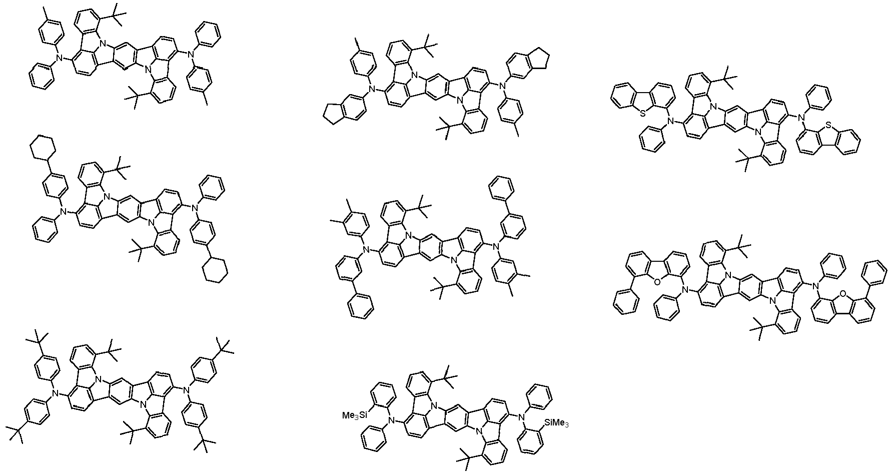

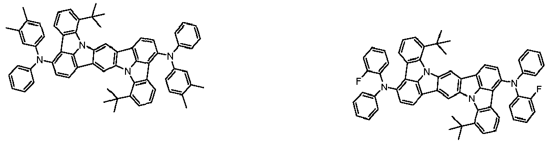

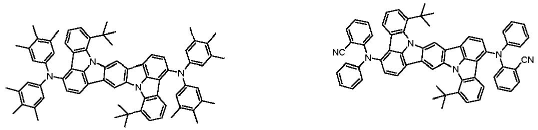

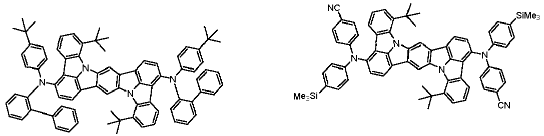

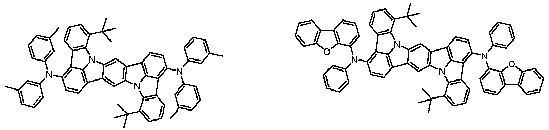

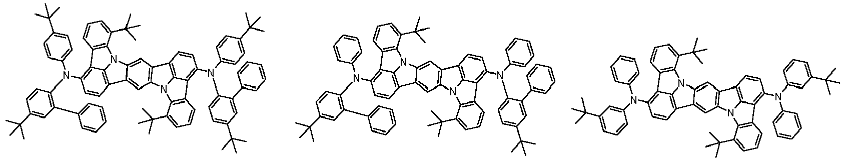

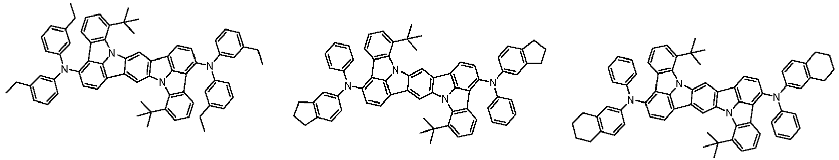

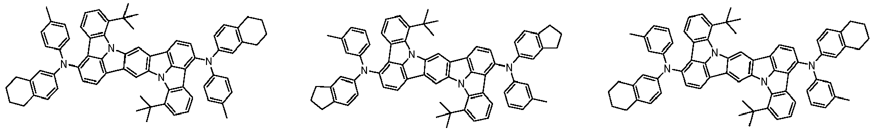

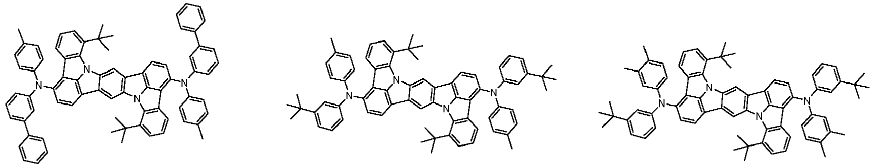

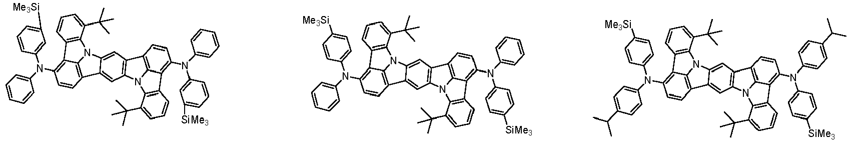

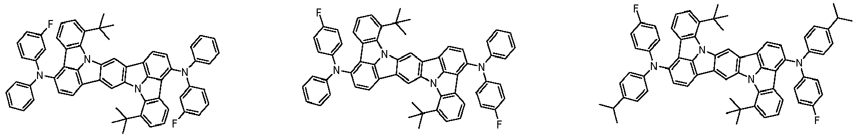

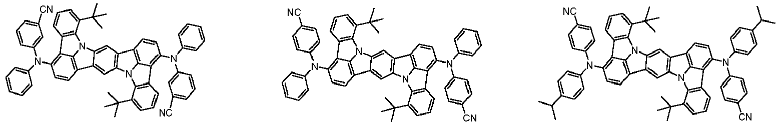

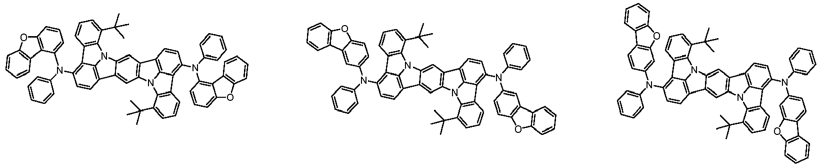

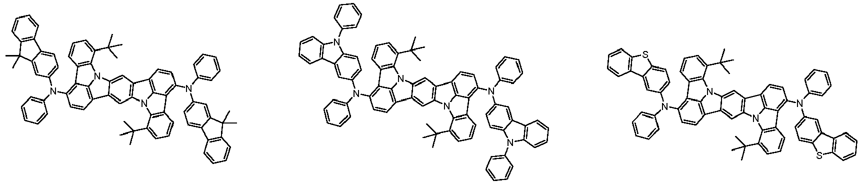

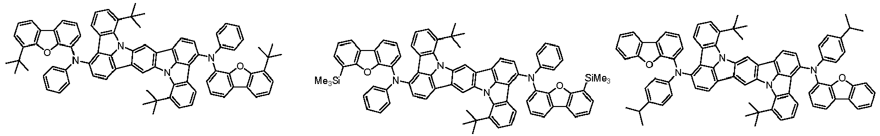

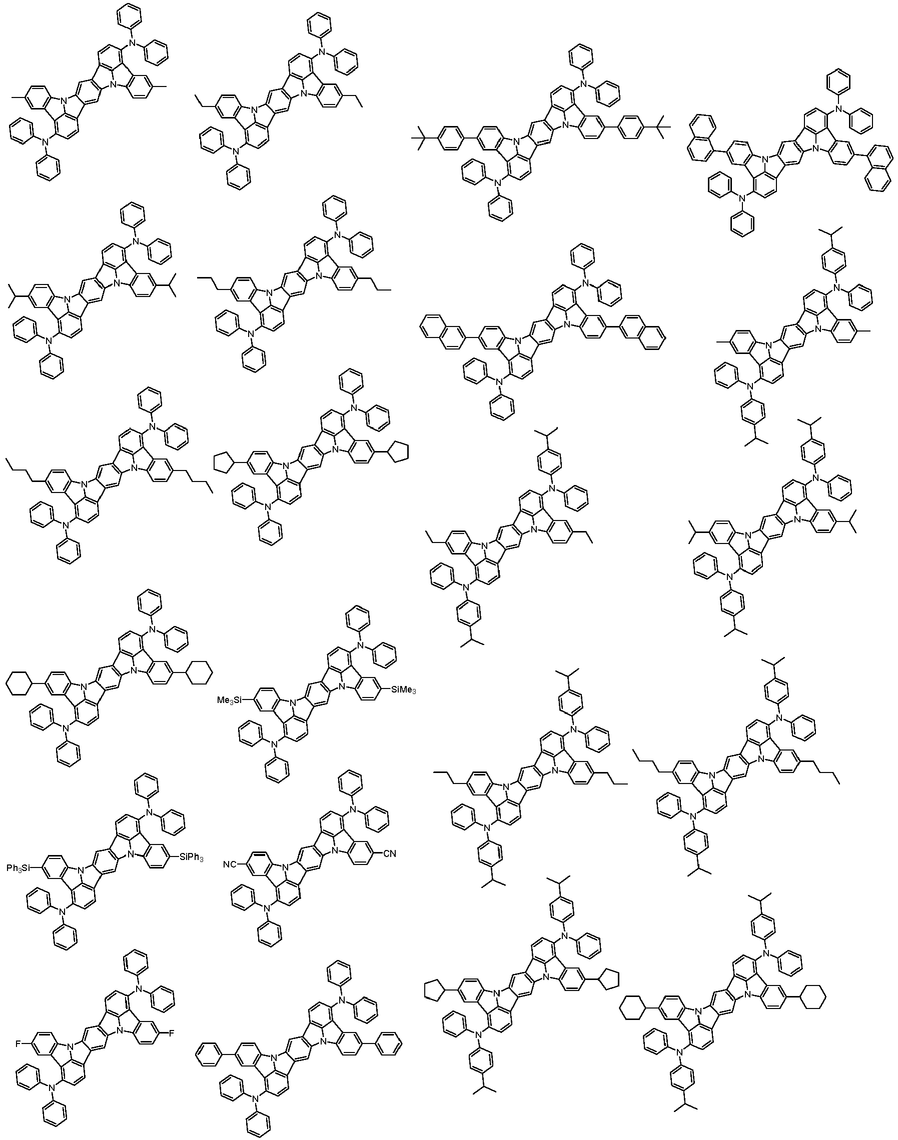

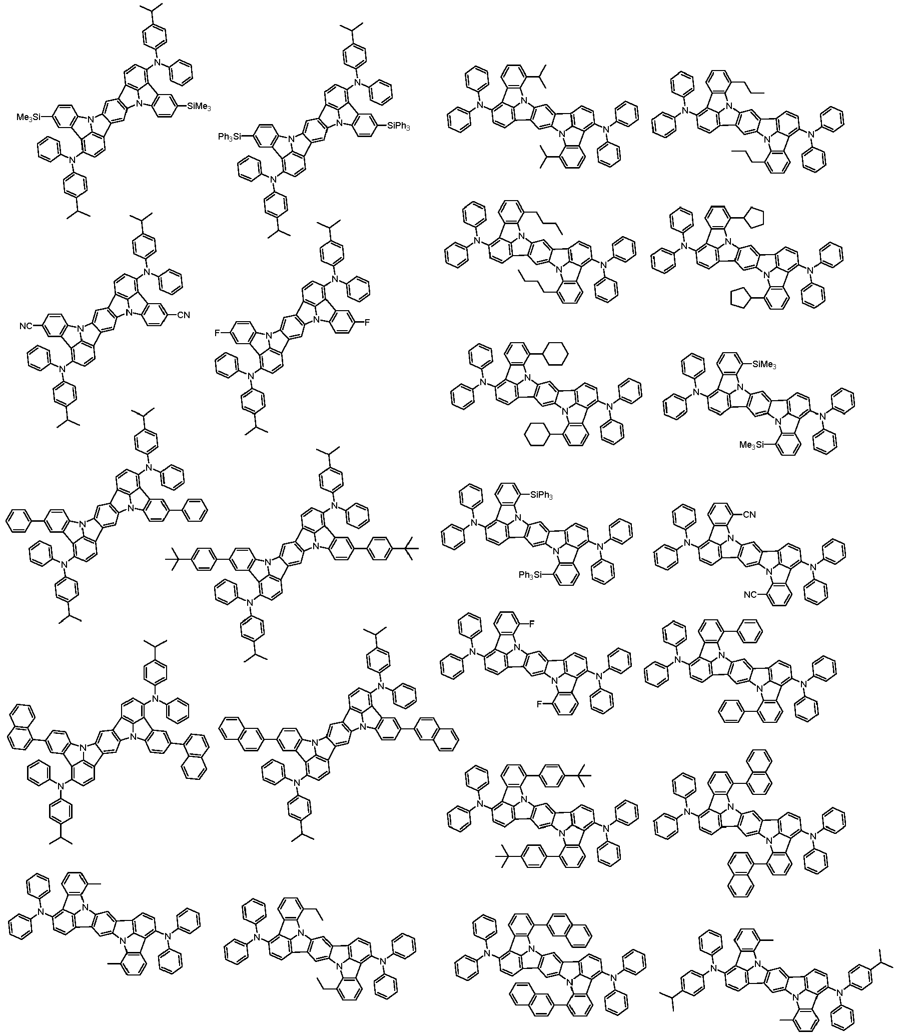

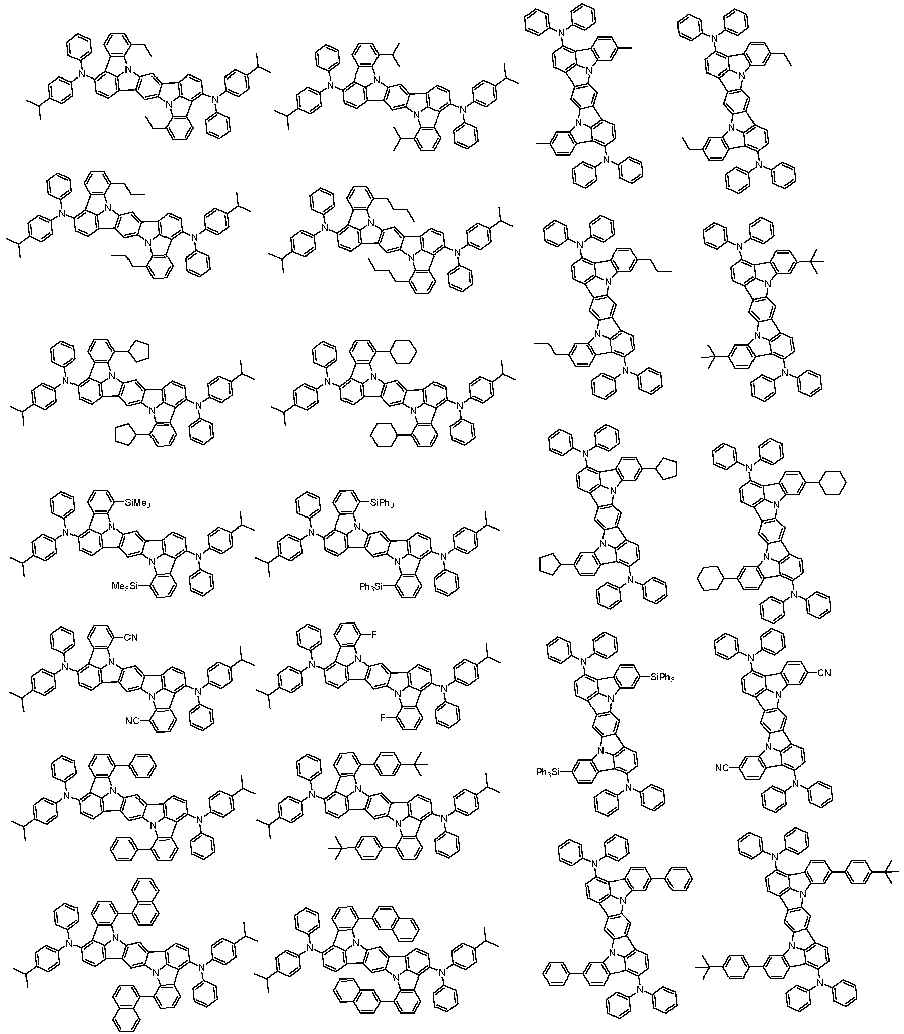

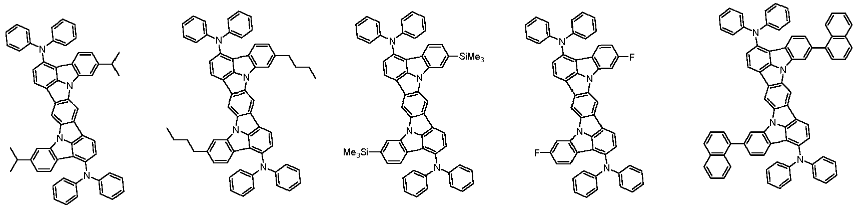

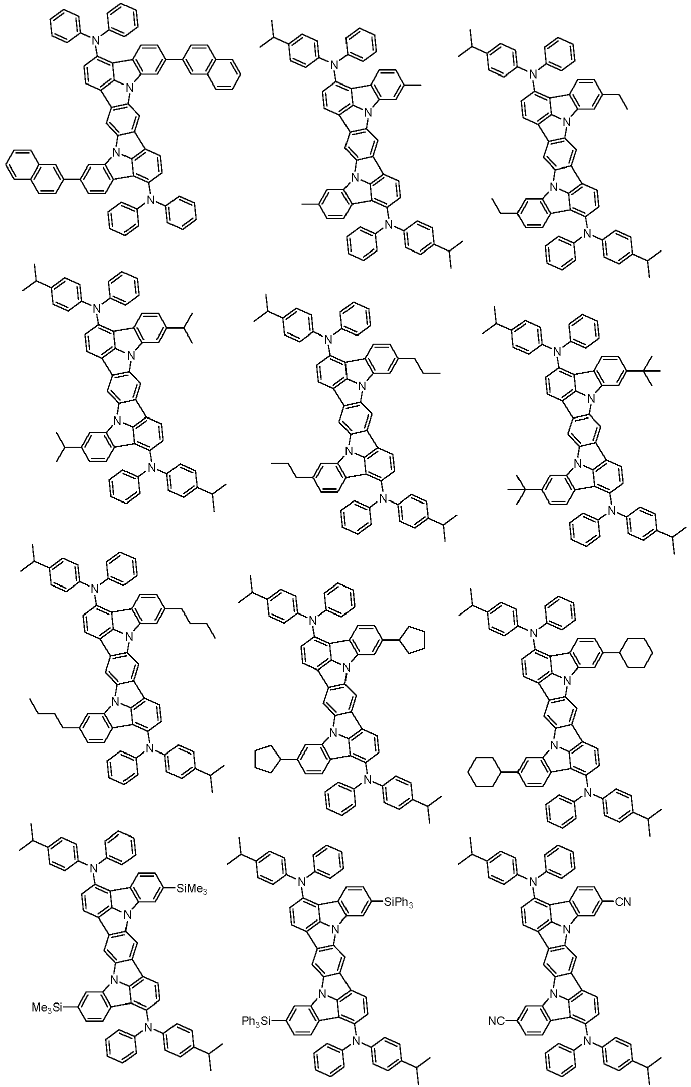

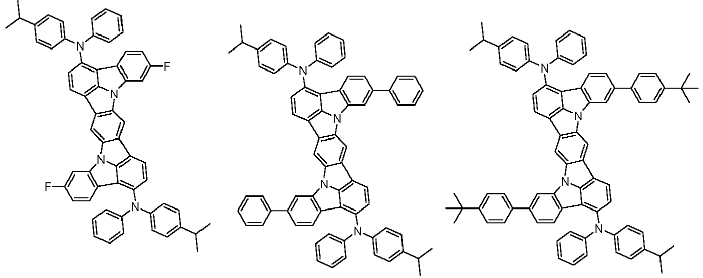

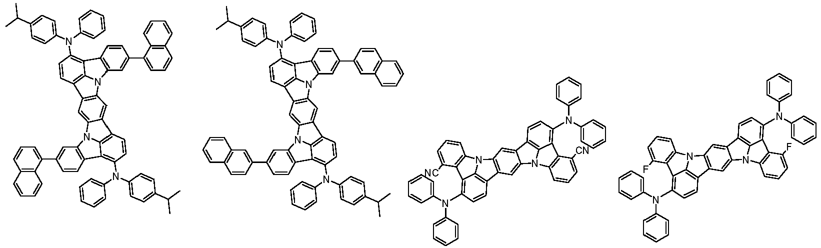

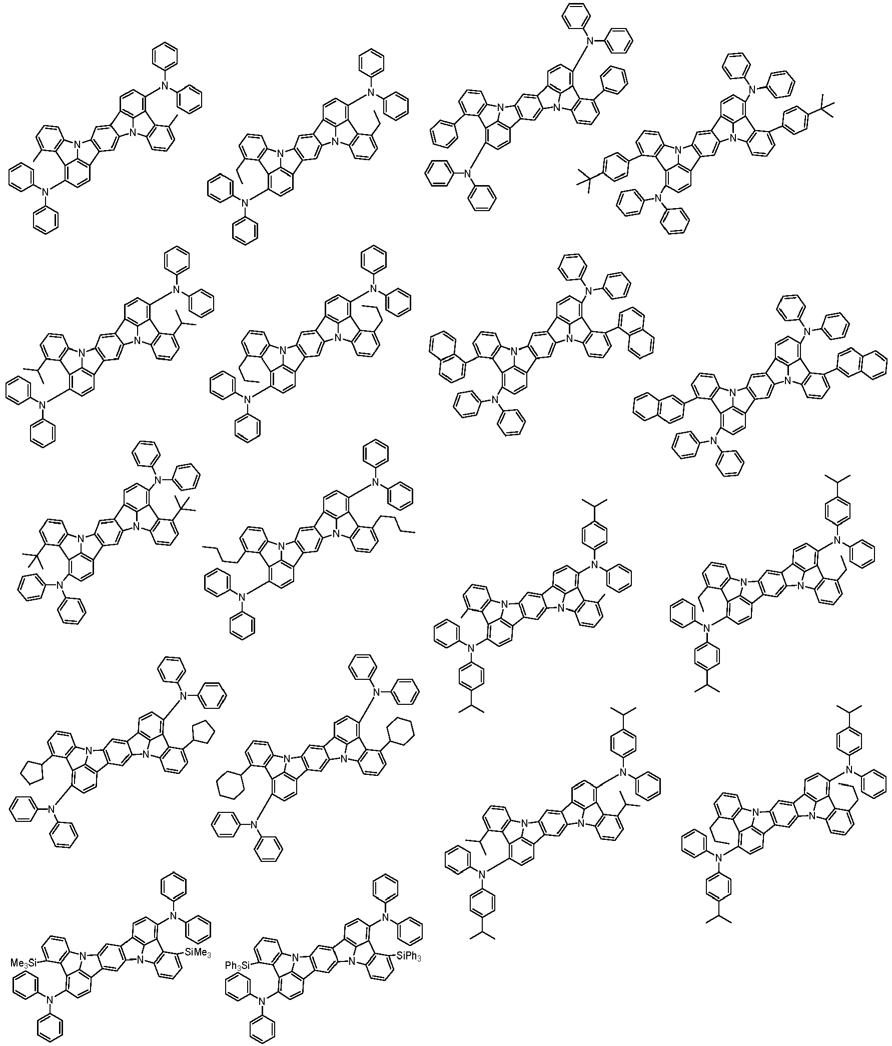

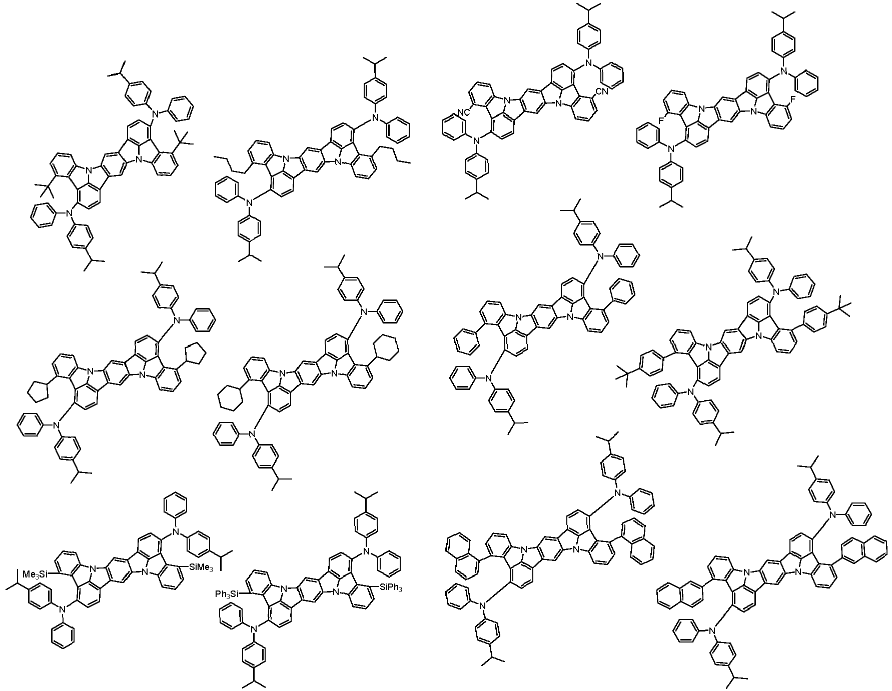

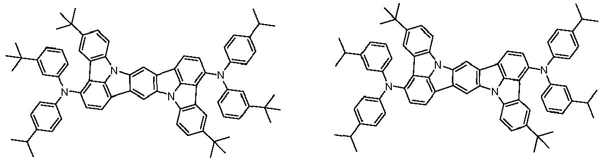

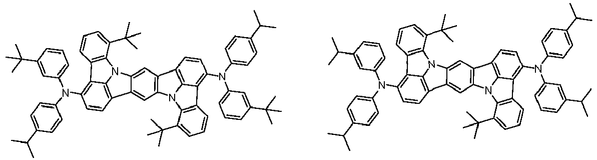

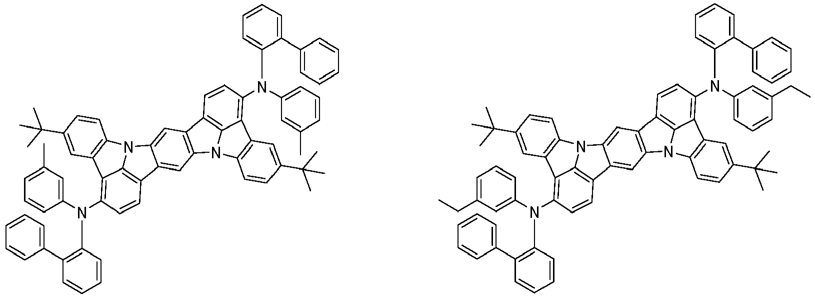

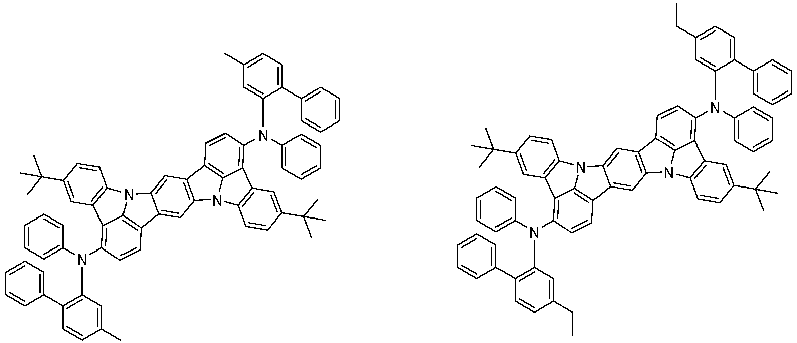

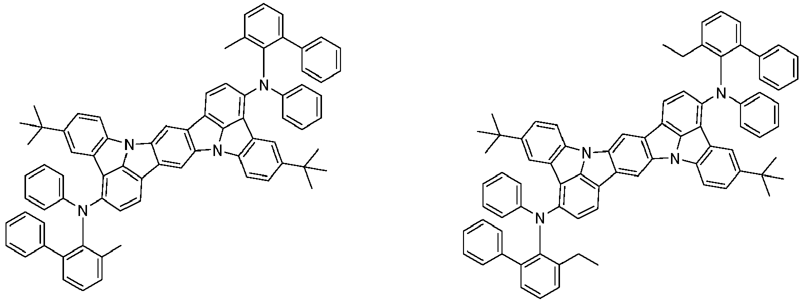

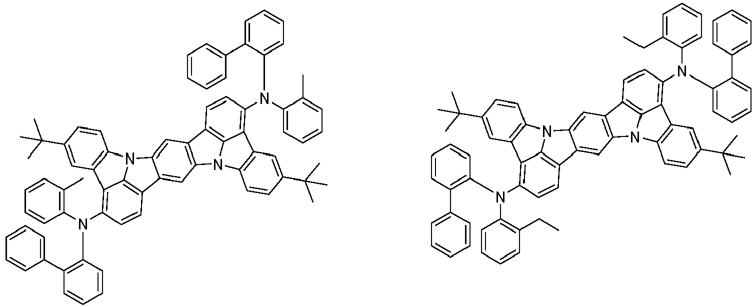

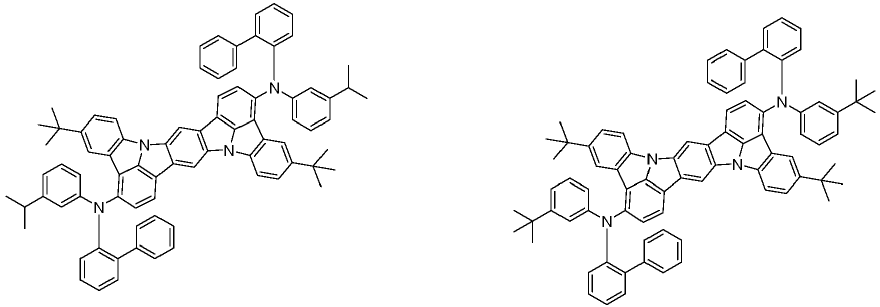

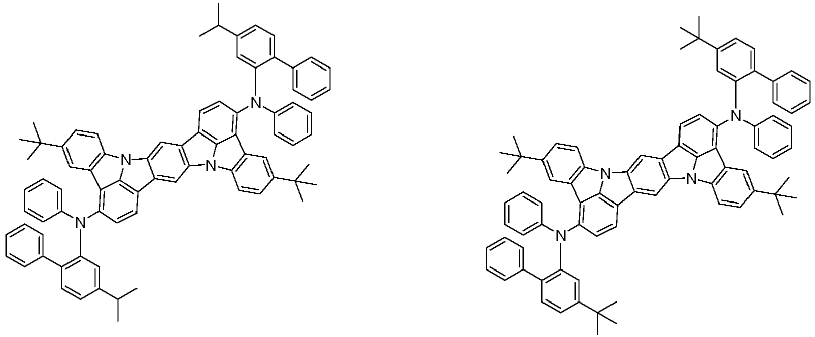

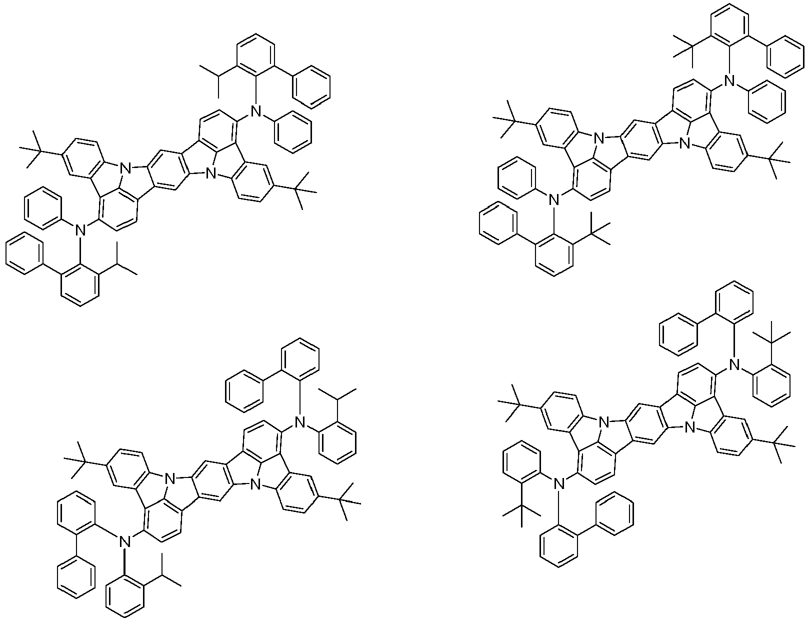

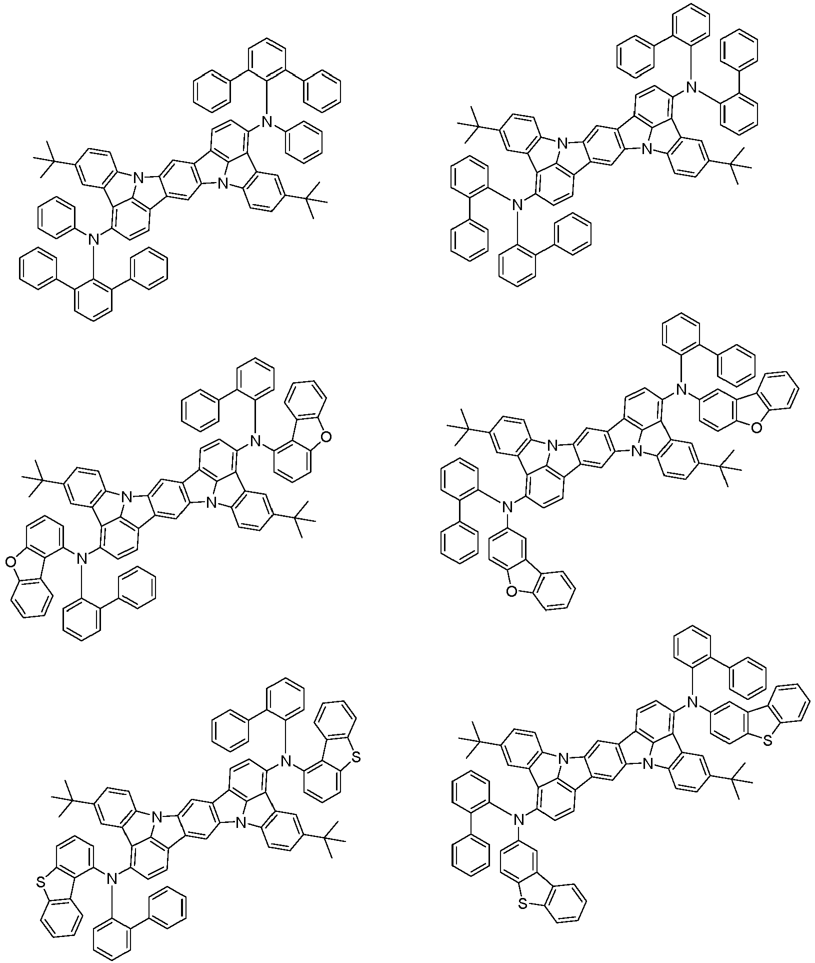

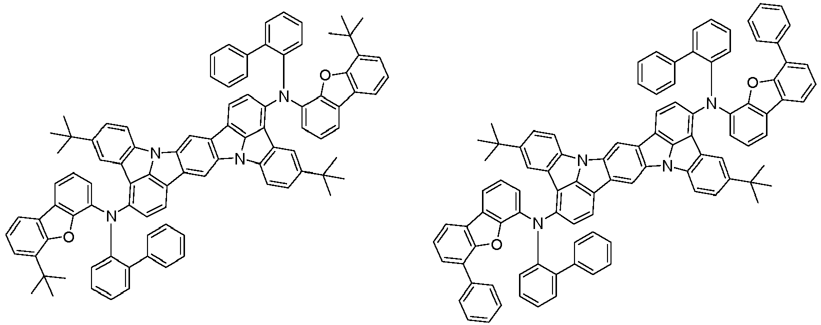

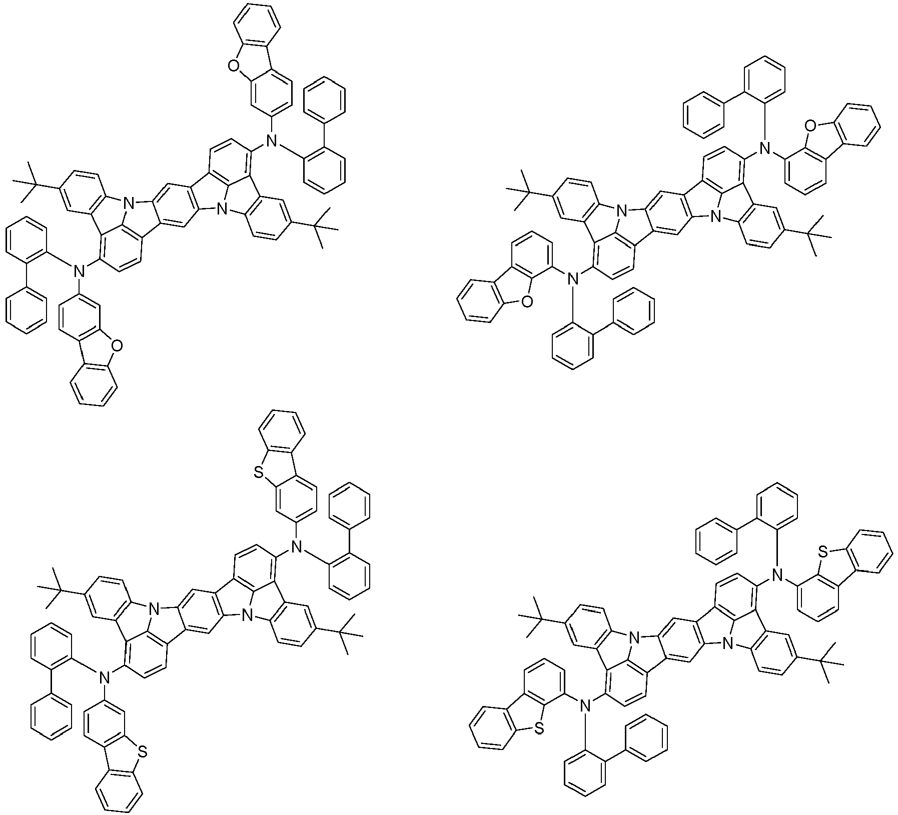

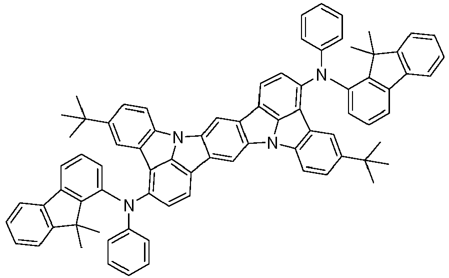

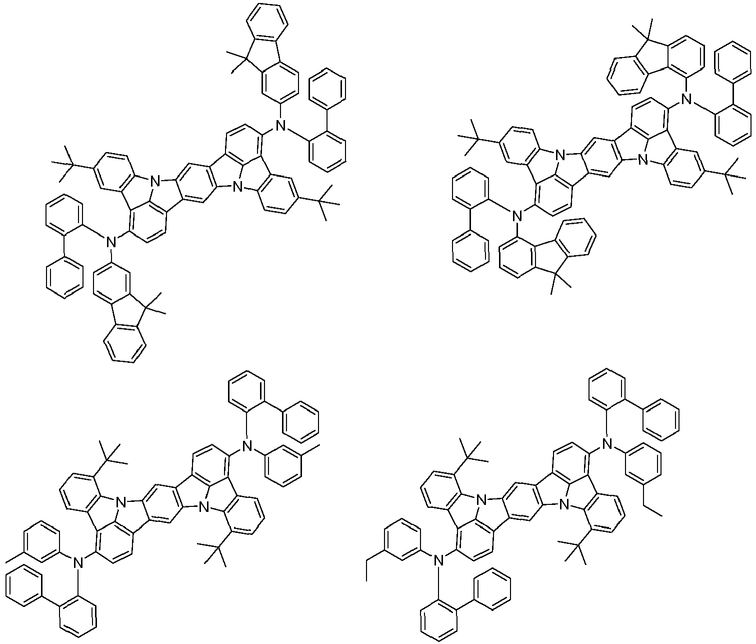

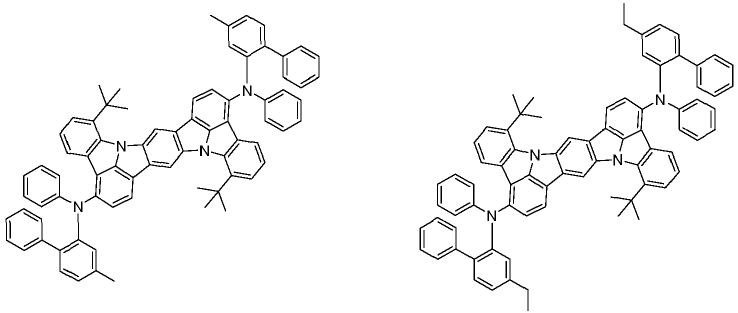

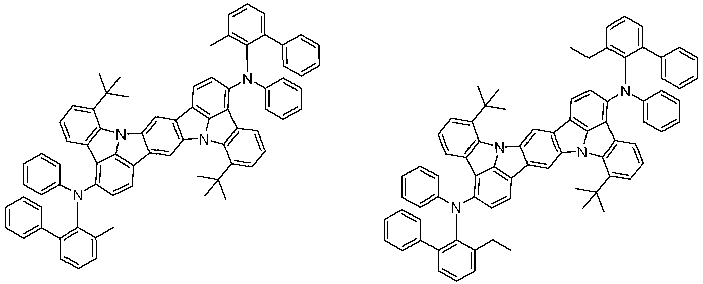

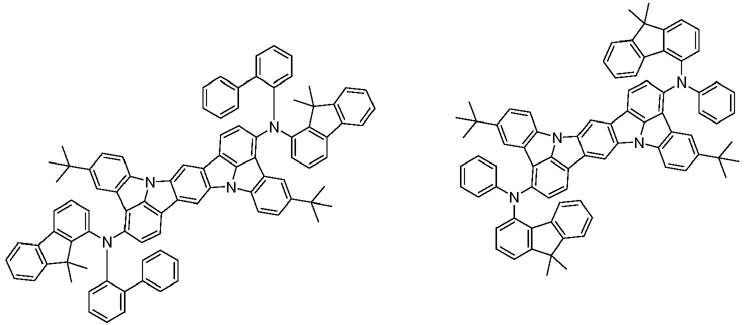

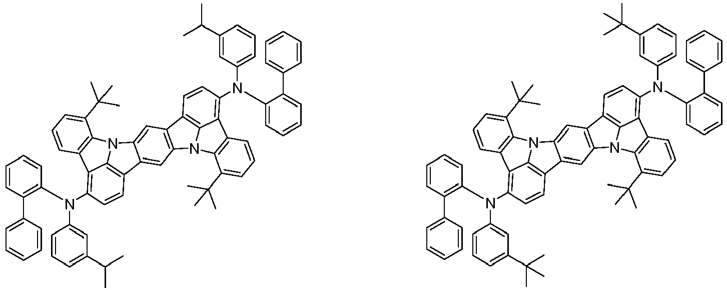

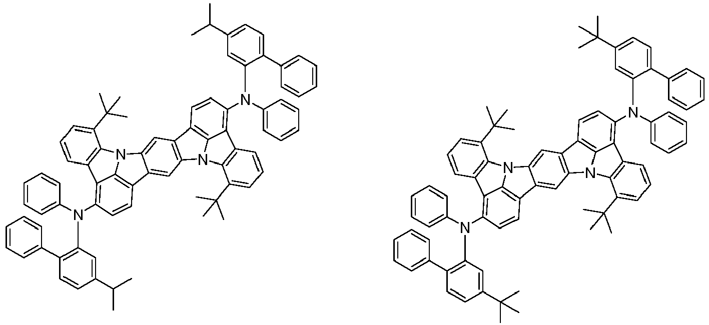

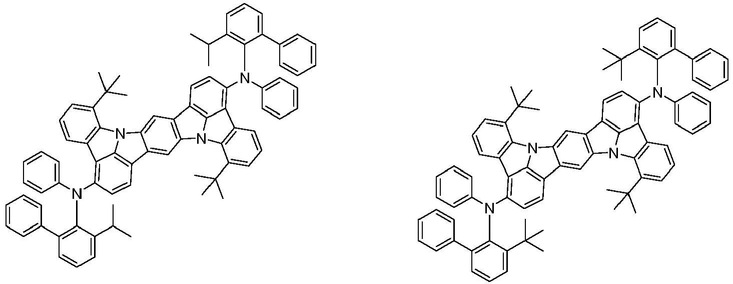

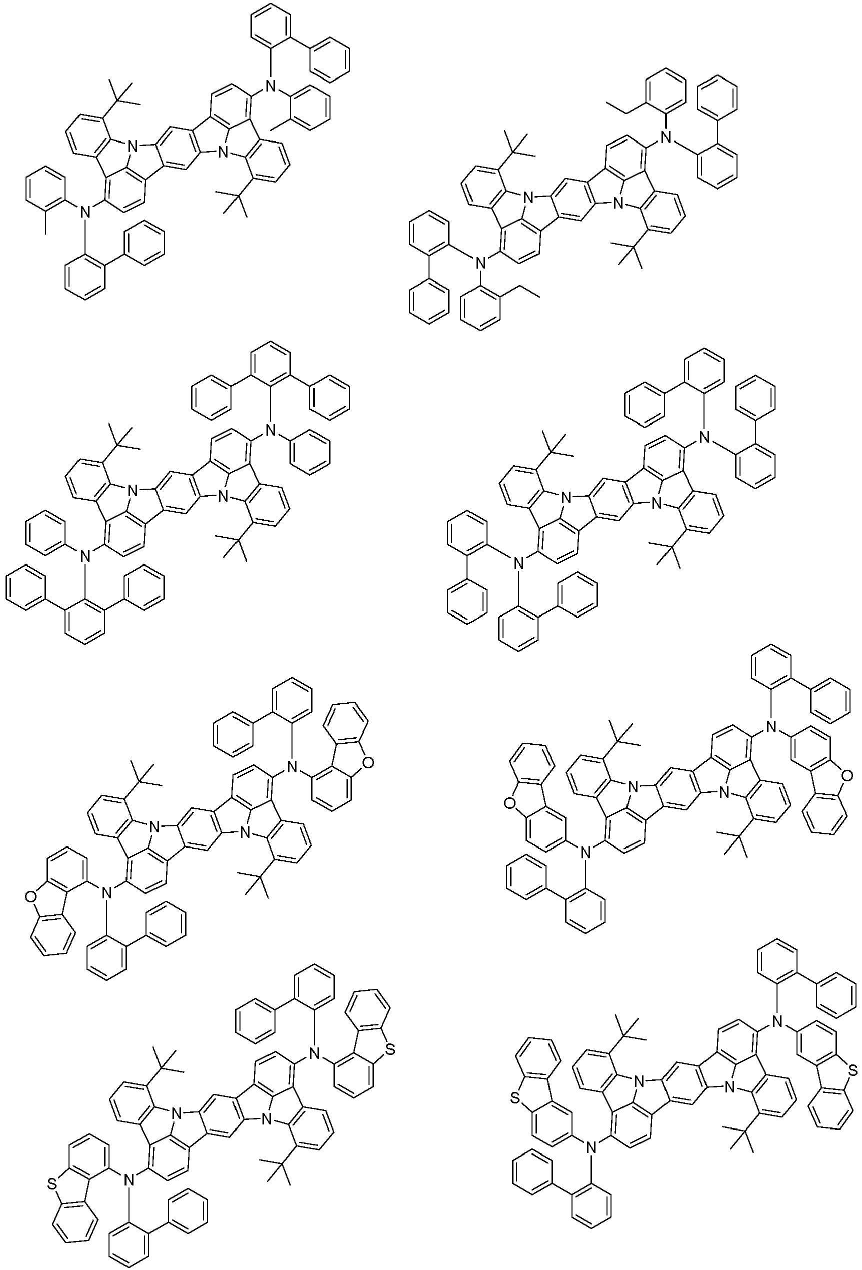

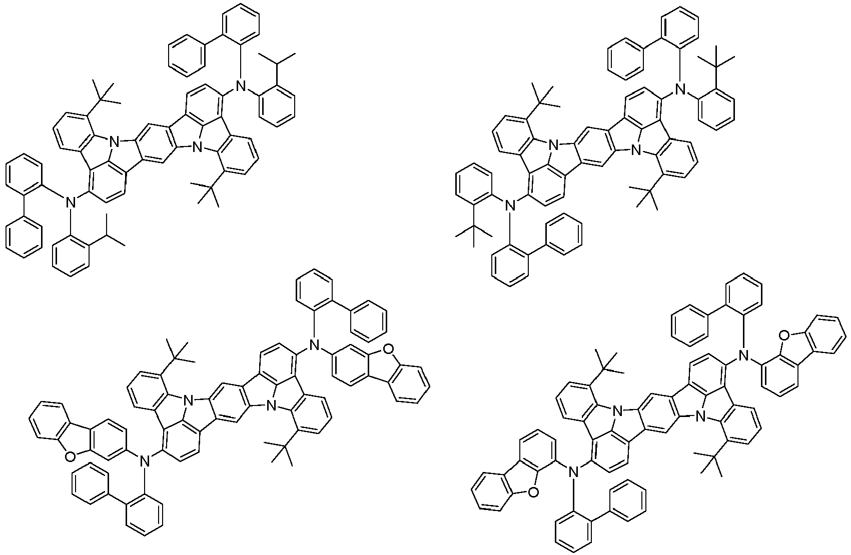

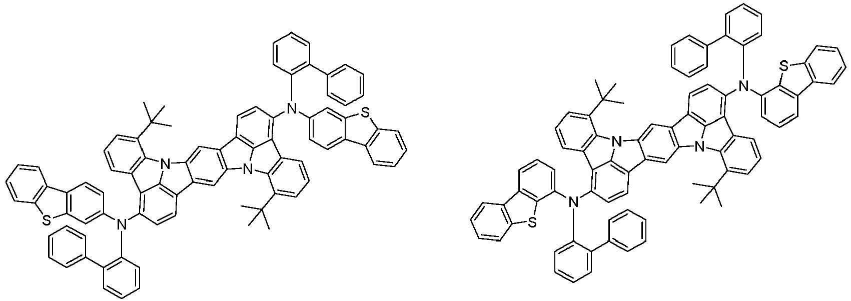

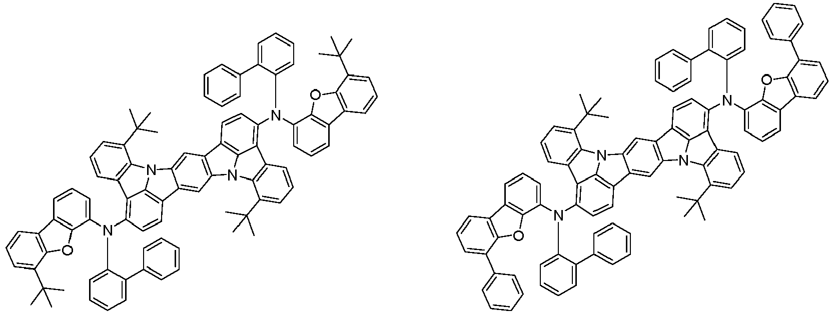

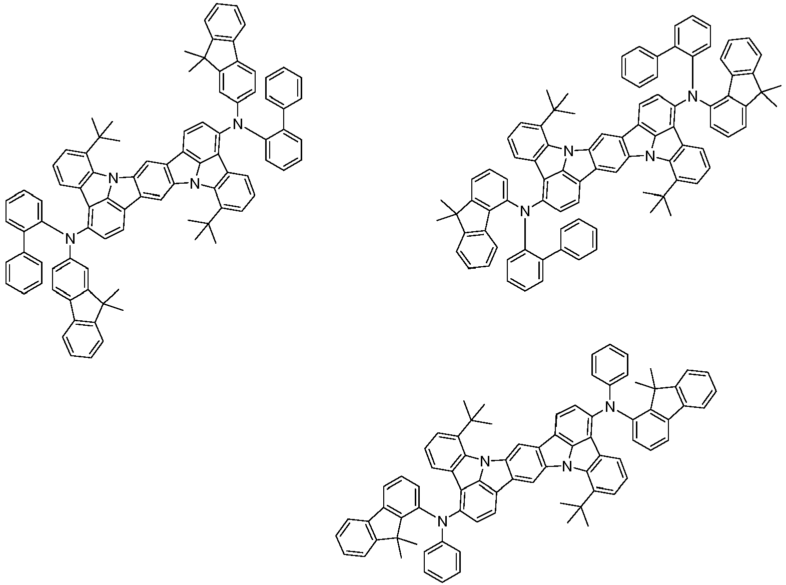

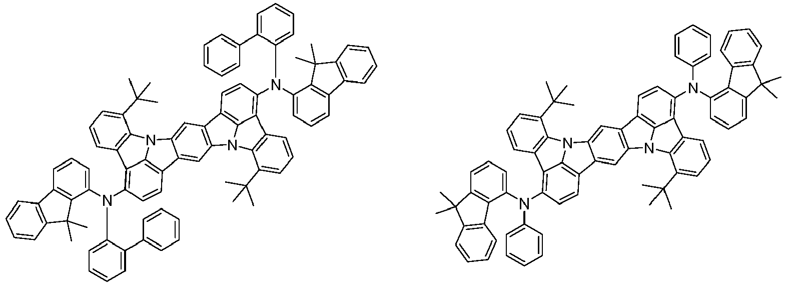

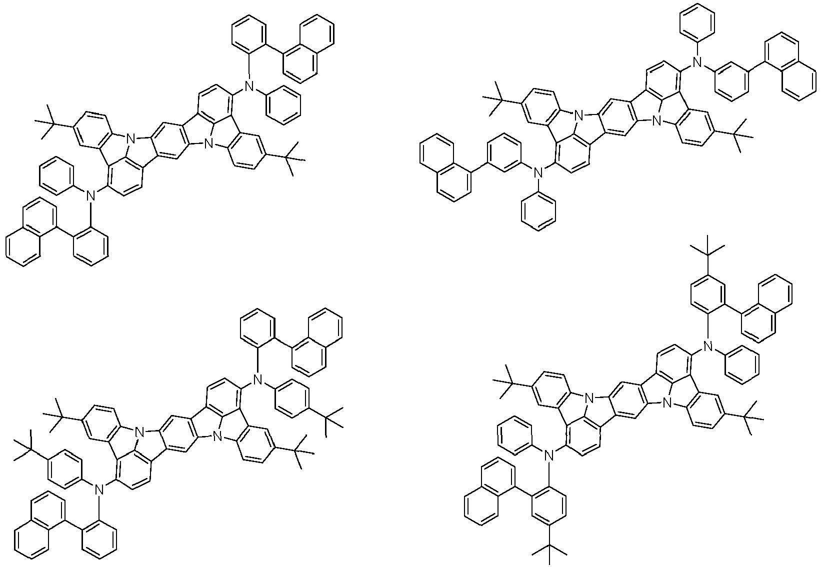

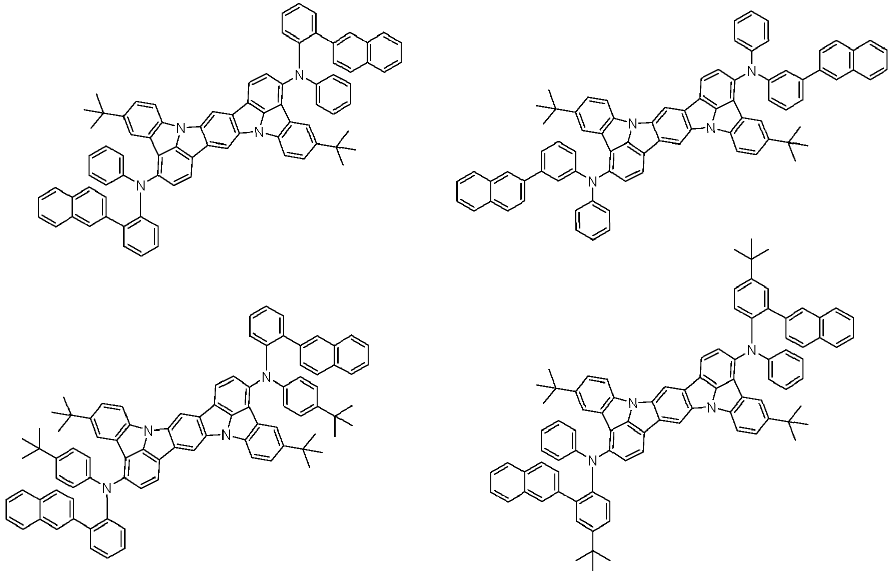

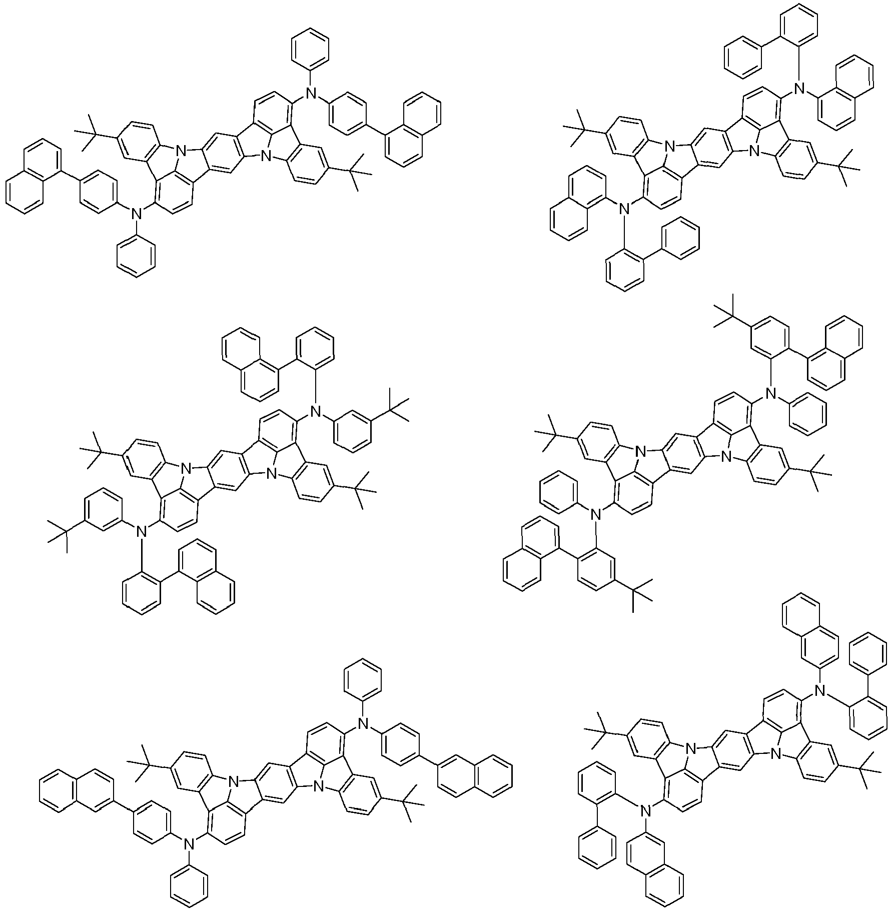

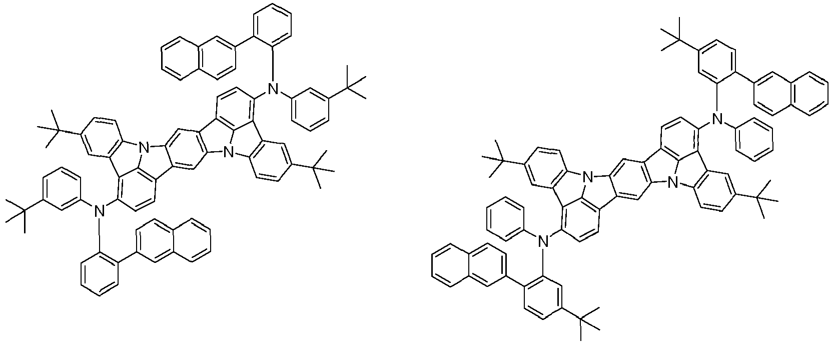

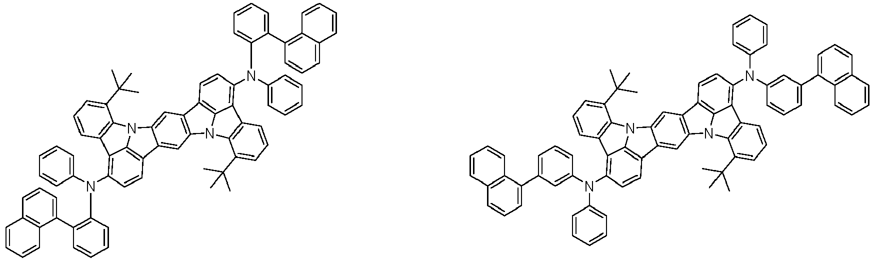

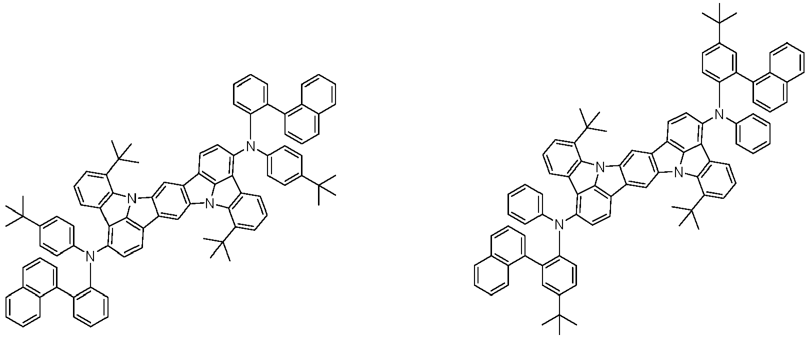

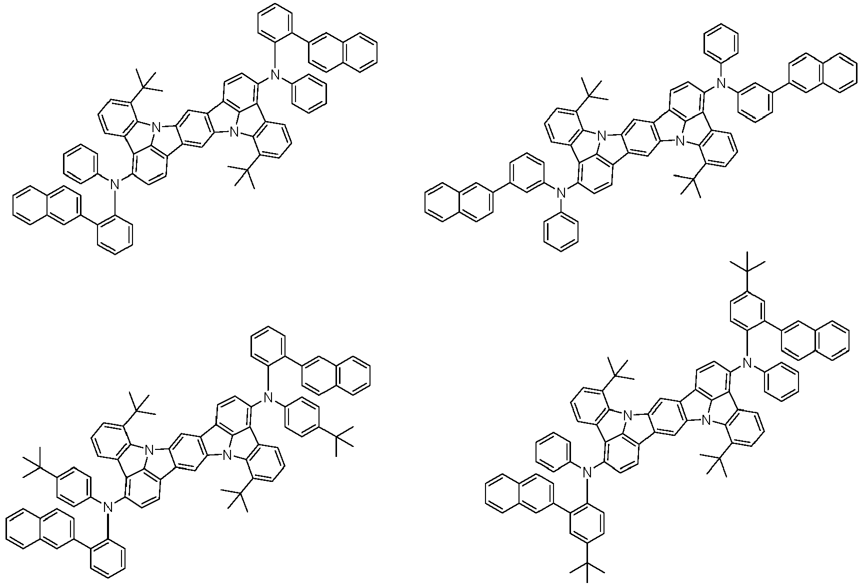

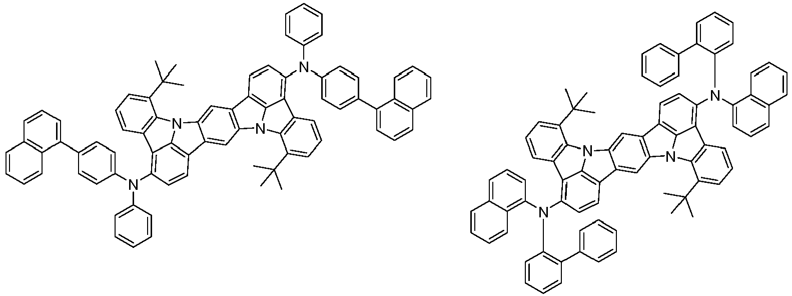

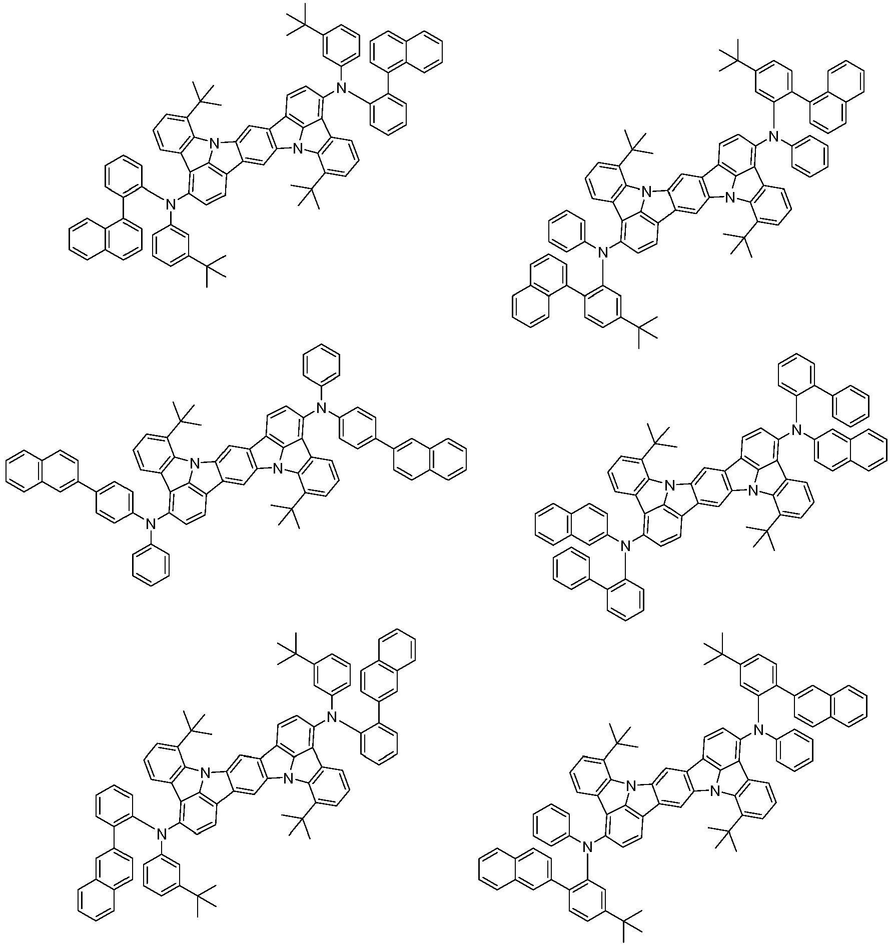

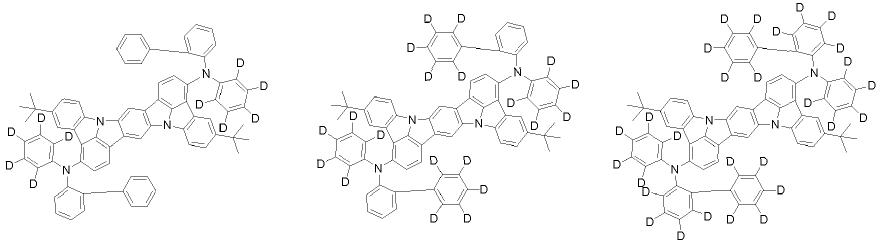

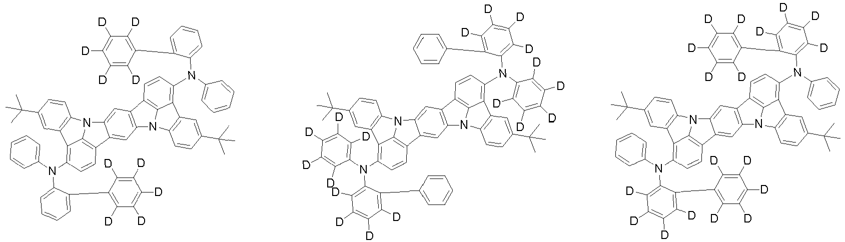

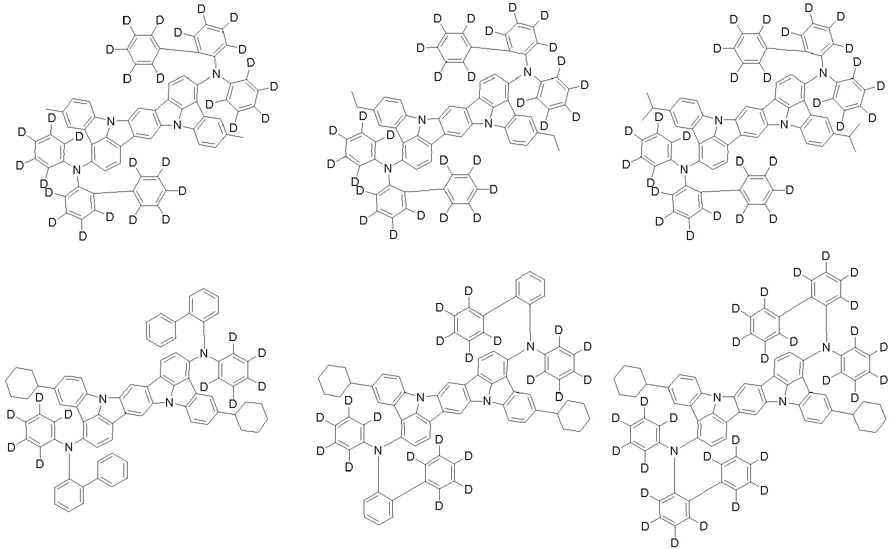

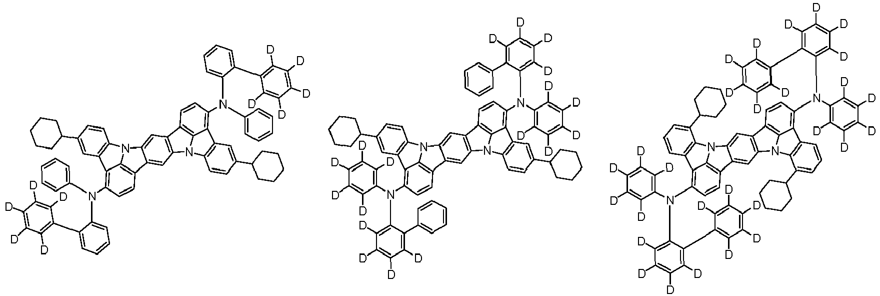

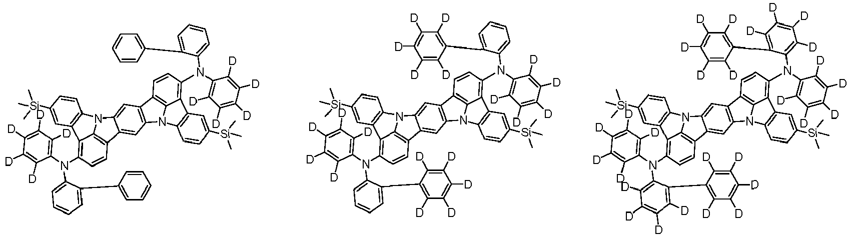

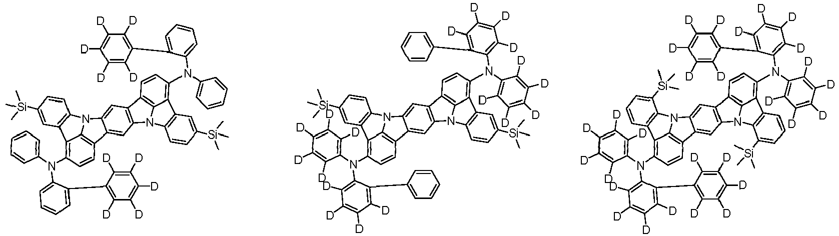

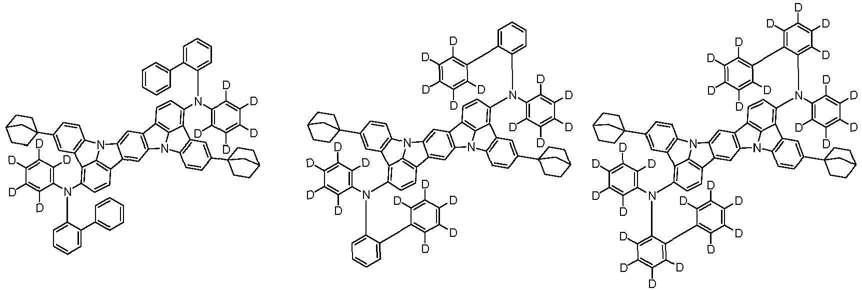

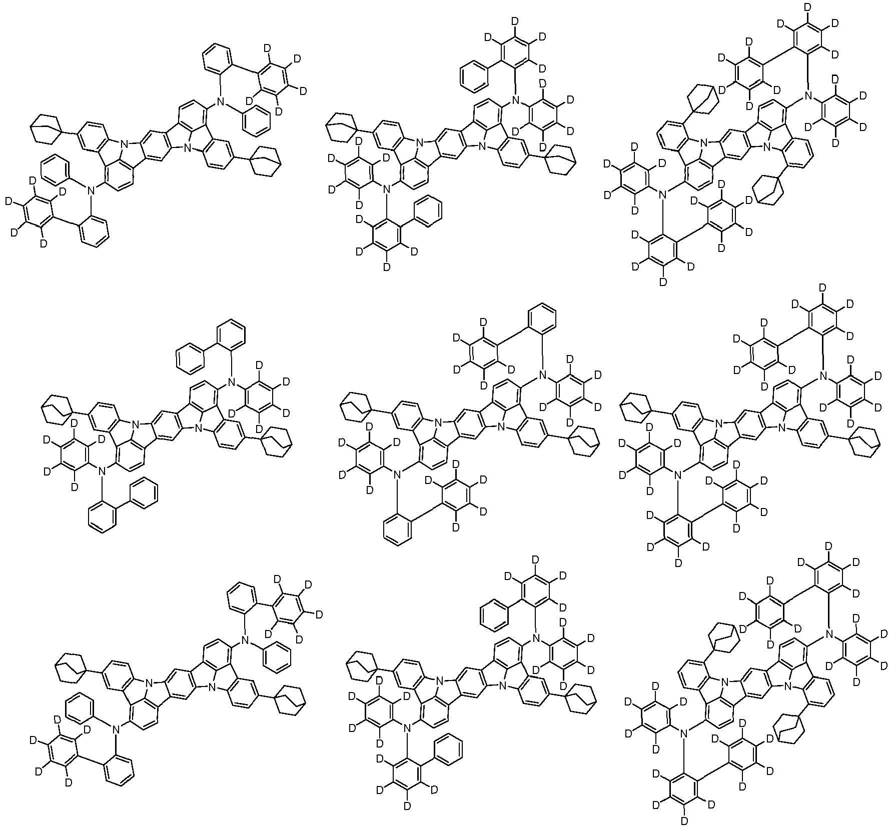

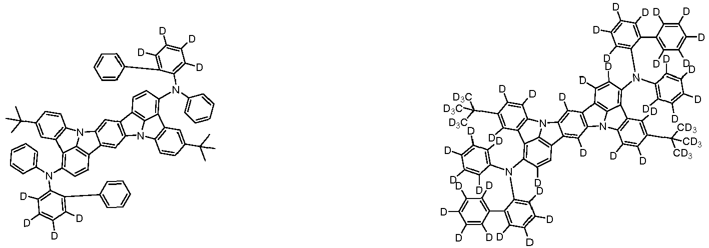

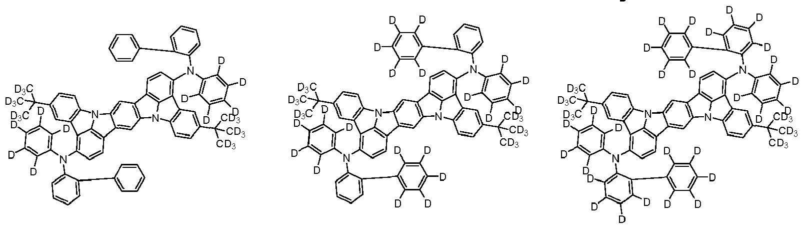

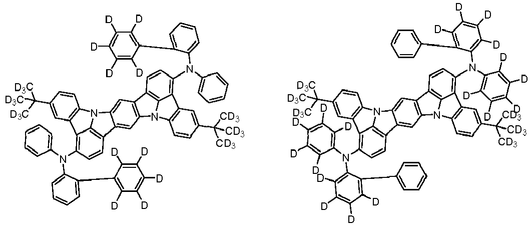

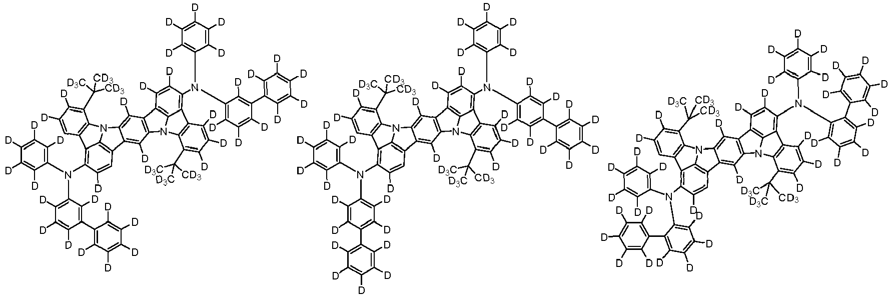

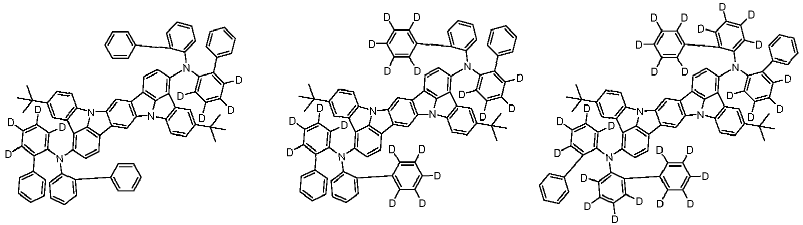

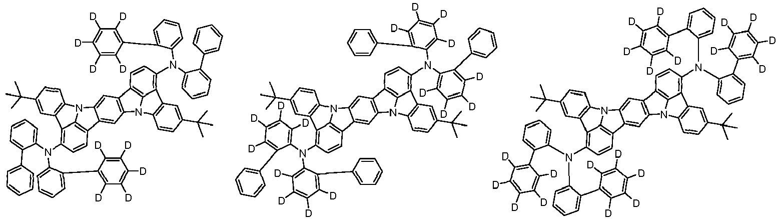

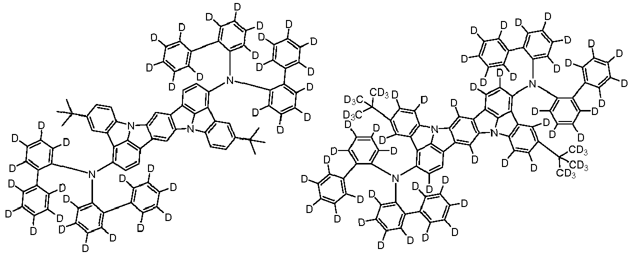

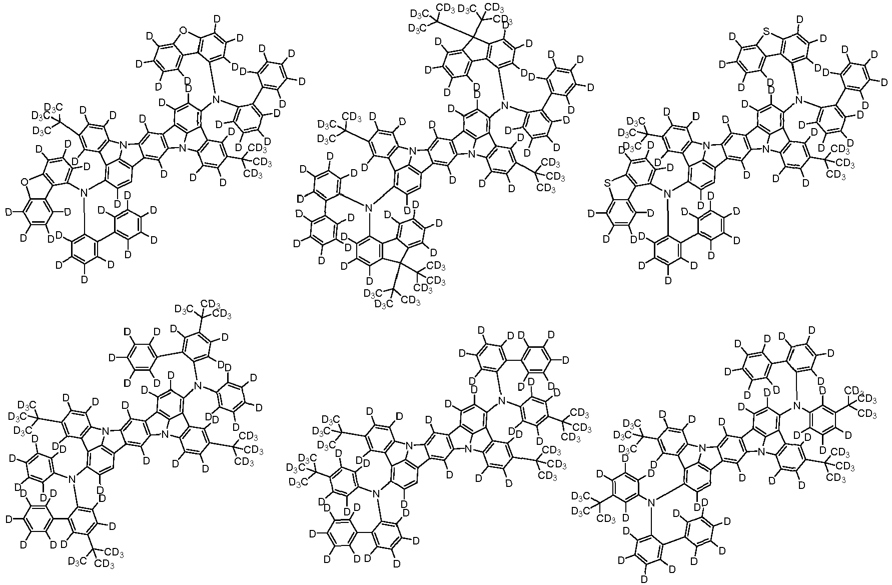

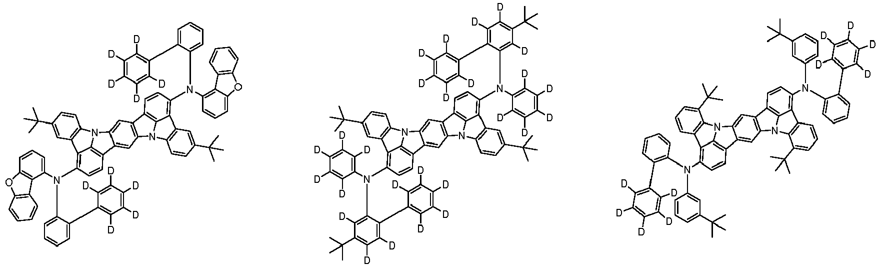

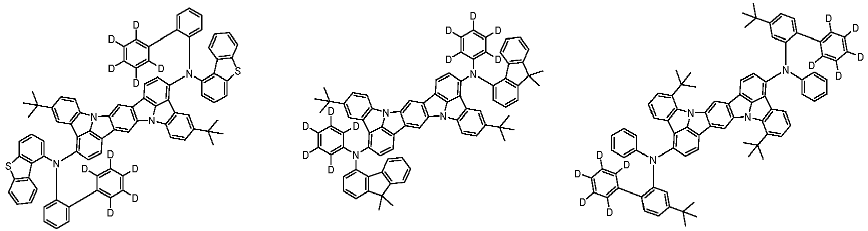

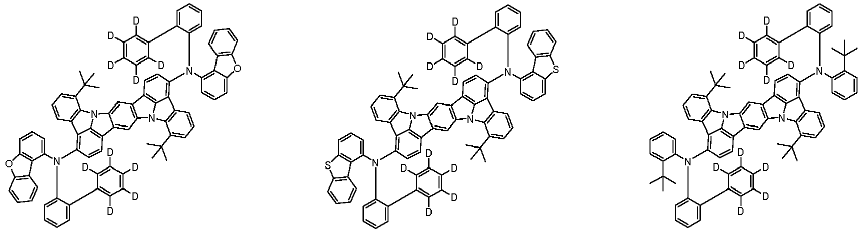

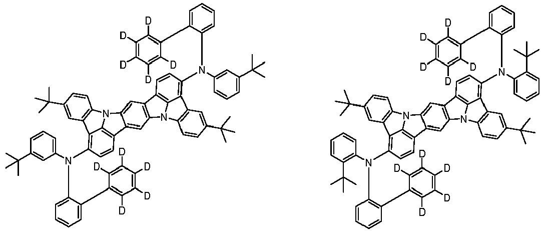

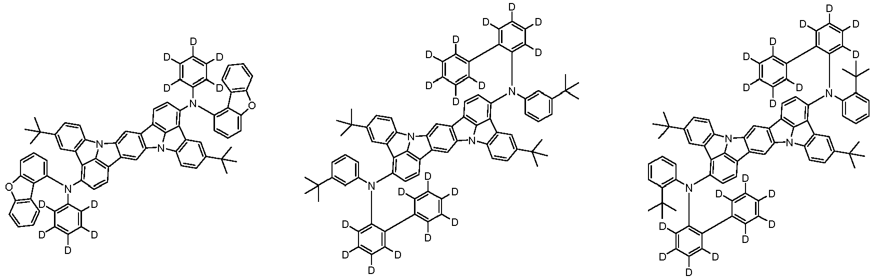

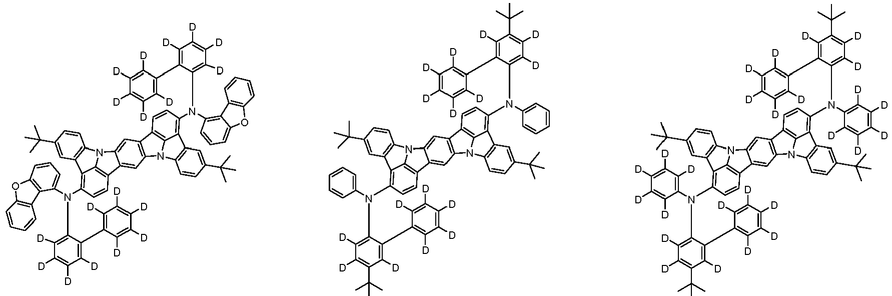

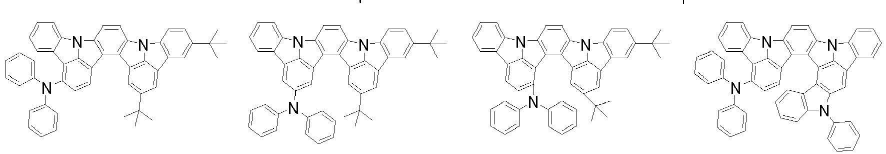

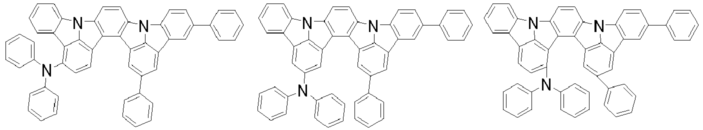

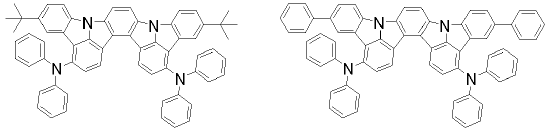

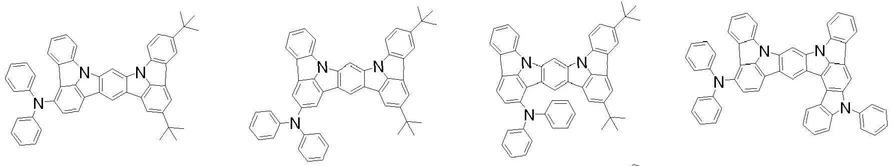

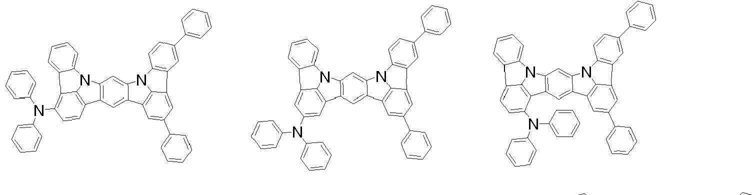

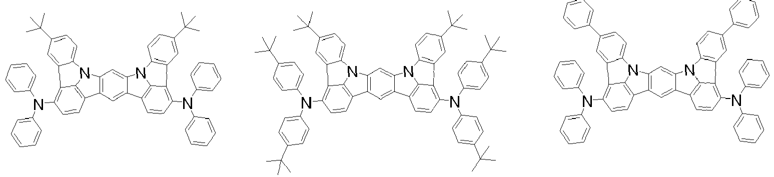

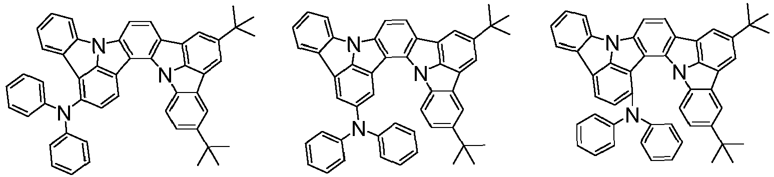

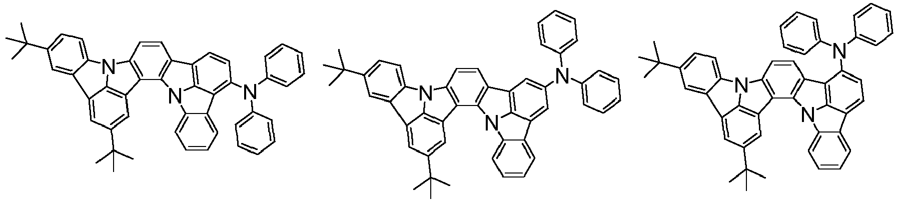

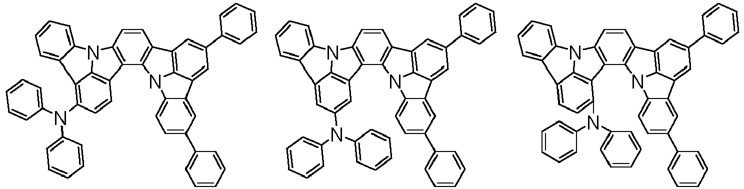

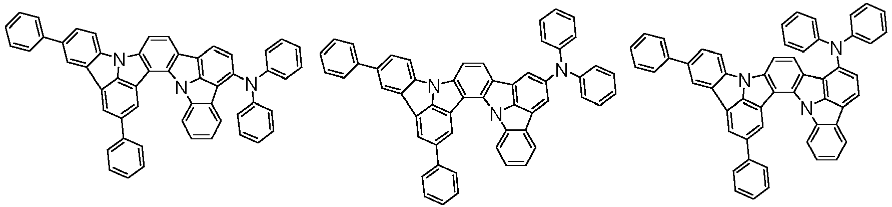

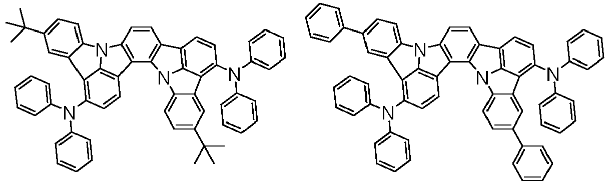

### [Material for an organic electroluminescence device]

The compound according to an aspect of the invention is useful as a material for an organic EL device, and is useful as a material for an emitting layer of an organic EL device, and is particularly useful as a dopant material for an emitting layer.

By using the compound according to an aspect of the invention for an emitting layer of an organic EL device, an organic EL device having a long lifetime can be obtained.

### [Organic EL device]

An organic EL device according to an aspect of the invention contain a cathode, an anode, and at least one organic layer disposed between the cathode and the anode, and at least one of the at least one organic layer contains a compound represented by the formulas (9-2) and (9-3).

A schematic configuration of an organic EL device according to an aspect of the invention will be described with reference to Figure 1.

The organic EL device 1 according to an aspect of the invention includes a substrate 2, an anode 3, an emitting layer 5 as an organic layer, a cathode 10, an organic layer 4 between the anode 3 and the emitting layer 5, and an organic layer 6 between the emitting layer 5 and the cathode 10.

Each of the organic layer 4 and the organic layer 6 may be a single layer or may consist of a plurality of layers.

Further, the organic layer 4 may contain a hole-transporting zone. The hole-transporting zone may contain a hole-injecting layer, a hole-transporting layer, an electron barrier layer, and the like. The organic layer 6 may contain an electron-transporting zone. The electron-transporting zone may contain an electron-injecting layer, an electron-transporting layer, a hole barrier layer, and the like.

The compound represented by the formulas (9-2) and (9-3)are contained in the organic layer 4, the emitting layer 5, or the organic layer 6. In one embodiment, the compound represented by the formulas (9-2) and (9-3)is contained in the emitting layer 5. The compound represented by the formulas (9-2) and (9-3)can function as a dopant material in the emitting layer 5.

In the organic EL device according to an aspect of the invention, at least one of the at least one organic layer contains a first compound and a second compound which is not the same as the first compound, and the first compound is a compound represented by the formulas (9-2) and (9-3).

In the organic EL device according to an aspect of the invention, the second compound is a heterocyclic compound or a fused aromatic compound.

In an organic EL device according to an aspect of the invention, the second compound is an anthracene derivative.

In an organic EL device according to an aspect of the invention, the second compound is a compound represented by the following formula (10):

### [Compound represented by the formula (10)]

A compound represented by the formula (10) will be described.

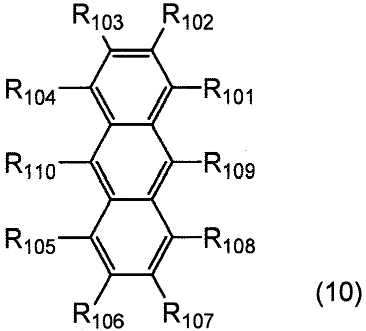

In the formula (10),
one or more sets of adjacent two or more of R₁₀₁ to R₁₁₀ form a substituted or unsubstituted, saturated or unsaturated ring by bonding with each other, or do not form the substituted or unsubstituted, saturated or unsaturated ring.

R₁₀₁ to R₁₁₀ which do not form the substituted or unsubstituted, saturated or unsaturated ring are independently
a hydrogen atom,
a substituent R, or
a group represented by the following formula (11):

   -L₁₀₁-Ar₁₀₁ (11)

In the formula (11),
L₁₀₁ is
   a single bond,
   a substituted or unsubstituted arylene group including 6 to 50 ring carbon atoms, or
   a substituted or unsubstituted divalent heterocyclic group including 5 to 50 ring atoms.
Ar₁₀₁ is
   a substituted or unsubstituted aryl group including 6 to 50 ring carbon atoms, or
   a substituted or unsubstituted monovalent heterocyclic group including 5 to 50 ring atoms.
the substituent R is
   a substituted or unsubstituted alkyl group including 1 to 50 carbon atoms,
   a substituted or unsubstituted alkenyl group including 2 to 50 carbon atoms,
   a substituted or unsubstituted alkynyl group including 2 to 50 carbon atoms,
   a substituted or unsubstituted cycloalkyl group including 3 to 50 ring carbon atoms,

      -Si(R₉₀₁)(R₉₀₂)(R₉₀₃),

      -O-(R₉₀₄),

      -S-(R₉₀₅),

      -N(R₉₀₆)(R₉₀₇),
   a halogen atom, a cyano group, a nitro group,
   a substituted or unsubstituted aryl group including 6 to 50 ring carbon atoms, or
   a substituted or unsubstituted monovalent heterocyclic group including 5 to 50 ring atoms.

When two or more of the substituent Rs are present, the two or more of the substituent Rs may be the same as or different from each other.

R₉₀₁ to R₉₀₇ are independently
a hydrogen atom,
a substituted or unsubstituted alkyl group including 1 to 50 carbon atoms,
a substituted or unsubstituted cycloalkyl group including 3 to 50 ring carbon atoms,
a substituted or unsubstituted aryl group including 6 to 50 ring carbon atoms, or
a substituted or unsubstituted monovalent heterocyclic group including 5 to 50 ring atoms.

When two or more of each of R₉₀₁ to R₉₀₇ are present, the two or more of each of R₉₀₁ to R₉₀₇ may be the same as or different from each other.

Provided that at least one of R₁₀₁ to R₁₁₀ which do not form the substituted or unsubstituted, saturated or unsaturated ring is a group represented by the formula (11). When two or more of the groups represented by the formula (11) are present, each of two or more of the groups represented by the formula (11) may be the same as or different from each other.

The compound represented by the formula (10) may have a deuterium atom as a hydrogen atom.

In one embodiment, at least one Ar₁₀₁ in the formula (10) is a substituted or unsubstituted aryl group including 6 to 50 ring carbon atoms.

In one embodiment, at least one Ar₁₀₁ in the formula (10) is a substituted or unsubstituted monovalent heterocyclic group including 5 to 50 ring atoms.

In one embodiment, all of Ar₁₀₁s in the formula (10) are a substituted or unsubstituted aryl group including 6 to 50 ring carbon atoms. The plurality of Ar₁₀₁s may be the same as or different from each other.

In one embodiment, one of Ar₁₀₁ in the formula (10) is a substituted or unsubstituted monovalent heterocyclic group including 5 to 50 ring atoms, and the remaining Ar₁₀₁ is a substituted or unsubstituted aryl group including 6 to 50 ring carbon atoms. The plurality of Ar₁₀₁s may be the same as or different from each other.

In one embodiment, at least one L₁₀₁ in the formula (10) is a single bond.

In one embodiment, all of L₁₀₁s in the formula (10) are single bonds.

In one embodiment, at least one L₁₀₁ in the formula (10) is a substituted or unsubstituted arylene group including 6 to 50 ring carbon atoms.

In one embodiment, at least one L₁₀₁ in the formula (10) is a substituted or unsubstituted phenylene group or a substituted or unsubstituted naphthyl group.

In one embodiment, the group represented by -L₁₀₁-Ar₁₀₁ in the formula (10) is selected from the group consisting of
a substituted or unsubstituted phenyl group,
a substituted or unsubstituted naphthyl group,
a substituted or unsubstituted biphenyl group,
a substituted or unsubstituted phenanthrenyl group,
a substituted or unsubstituted benzophenanthrenyl group,
a substituted or unsubstituted fluorenyl group,
a substituted or unsubstituted benzofluorenyl group,
a substituted or unsubstituted dibenzofuranyl group,
a substituted or unsubstituted naphthobenzofuranyl group,
a substituted or unsubstituted dibenzothiophenyl group, and
a substituted or unsubstituted carbazolyl group.

In one embodiment, the substituent R in the formula (10) are independently a substituted or unsubstituted alkyl group including 1 to 50 carbon atoms,
a substituted or unsubstituted cycloalkyl group including 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group, or
a substituted or unsubstituted aryl group including 6 to 50 ring carbon atoms.

R₉₀₁ to R₉₀₇ are as defined in the formula (10).

In one embodiment, the substituent in the case of "substituted or unsubstituted" in the formula (10) are independently
a substituted or unsubstituted alkyl group including 1 to 50 carbon atoms,
a substituted or unsubstituted alkenyl group including 2 to 50 carbon atoms,
a substituted or unsubstituted alkynyl group including 2 to 50 carbon atoms,
a substituted or unsubstituted cycloalkyl group including 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group,
a substituted or unsubstituted aryl group including 6 to 50 ring carbon atoms, or
a substituted or unsubstituted monovalent heterocyclic group including 5 to 50 ring atoms.

R₉₀₁ to R₉₀₇ are as defined in the formula (10).

In one embodiment, the substituent in the case of "substituted or unsubstituted" in the formula (10) are independently
a substituted or unsubstituted alkyl group including 1 to 50 carbon atoms,
a substituted or unsubstituted cycloalkyl group including 3 to 50 ring carbon atoms,

   -Si(R₉₀₁)(R₉₀₂)(R₉₀₃),

   -O-(R₉₀₄),

   -S-(R₉₀₅),

   -N(R₉₀₆)(R₉₀₇),
a halogen atom, a cyano group, a nitro group, or
a substituted or unsubstituted aryl group including 6 to 50 ring carbon atoms.

R₉₀₁ to R₉₀₇ are as defined in the formula (10).

In one embodiment, the substituent in the case of "substituted or unsubstituted" in the formula (10) is selected from the group consisting of
an alkyl group including 1 to 18 carbon atoms,
an aryl group including 6 to 18 ring carbon atoms, and
a monovalent heterocyclic group including 5 to 18 ring atoms.

In one embodiment, the substituent in the case of "substituted or unsubstituted" in the formula (10) is an alkyl group including 1 to 5 carbon atoms.

In one embodiment, the compound represented by the formula (10) is a compound represented by the following formula (20).

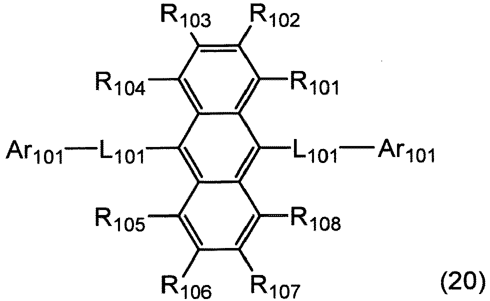

In the formula (20), R₁₀₁ to R₁₀₈, L₁₀₁, and Ar₁₀₁ are as defined in the formula (10).

The compound represented by the formula (20) may have a deuterium atom as a hydrogen atom.

In other words, in one embodiment, the compound represented by the formula (10) or the formula (20) has at least two groups represented by the formula (11).

In one embodiment, the compound represented by the formula (10) or the formula (20) has two or three groups represented by the formula (11).

In one embodiment, R₁₀₁ to R₁₁₀ in the formula (10) and (20) do not form the substituted or unsubstituted, saturated or unsaturated ring.

In one embodiment, R₁₀₁ to R₁₁₀ in the formula (10) and (20) are hydrogen atoms.

In one embodiment, in the compound represented by the formula (20), at least one Ar₁₀₁ is a monovalent group having a structure represented by the following formula (50).

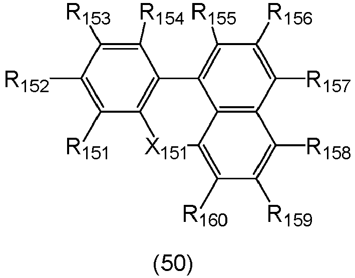

In the formula (50),
X₁₅₁ is O, S, or C(R₁₆₁)(R₁₆₂).

One of R₁₅₁ to R₁₆₀ is a single bond which bonds with L₁₀₁.

One or more sets of adjacent two or more of R₁₅₁ to R₁₅₄ and one or more sets of adjacent two or more of R₁₅₅ to R₁₆₀, which are not a single bond which bonds with L₁₀₁, form a substituted or unsubstituted, saturated or unsaturated ring by bonding with each other, or do not form a substituted or unsubstituted, saturated or unsaturated ring.

R₁₆₁ and R₁₆₂ form a substituted or unsubstituted, saturated or unsaturated ring by bonding with each other, or do not form a substituted or unsubstituted, saturated or unsaturated ring.

R₁₆₁ and R₁₆₂ which do not form the substituted or unsubstituted, saturated or unsaturated ring, and R₁₅₁ to R₁₆₀ which are not a single bond which bonds with L₁₀₁ and do not form the substituted or unsubstituted, saturated or unsaturated ring are independently a hydrogen atom or a substituent R.

The substituent R is as defined in the formula (10).

Ar₁₀₁, which is not a monovalent group having the structure represented by the formula (50) is
a substituted or unsubstituted aryl group including 6 to 50 ring carbon atoms, or
a substituted or unsubstituted divalent heterocyclic group including 5 to 50 ring atoms.

The position to be a single bond which bonds with L₁₀₁ in the formula (50) is not particularly limited.

In one embodiment, one of R₁₅₁ to R₁₅₄ or one of R₁₅₅ to R₁₆₀ in the formula (50) is a single bond which bonds with L₁₀₁.

In one embodiment, Ar₁₀₁ is a monovalent group represented by the following formula (50-R₁₅₂), (50-R₁₅₃), (50-R₁₅₄), (50-R₁₅₇), or (50-R₁₅₈).

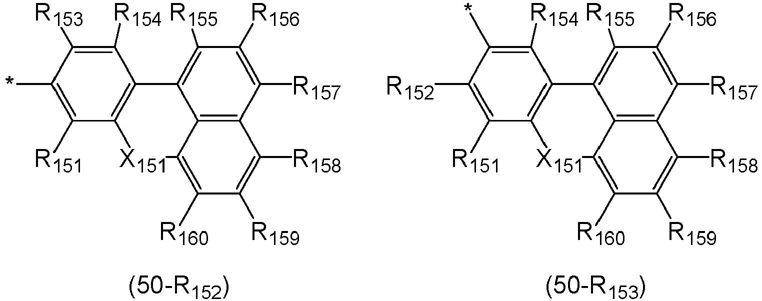

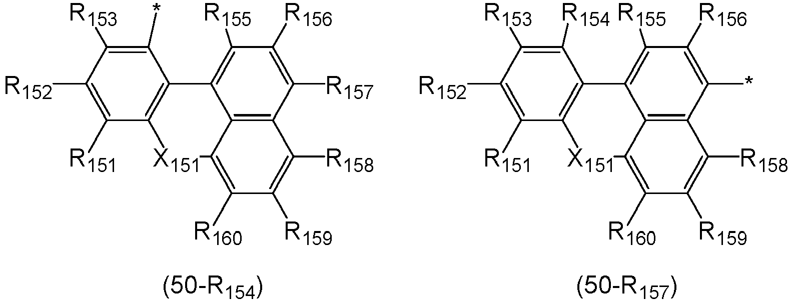

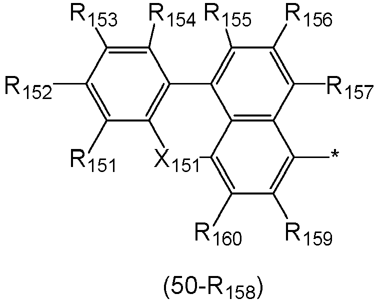

In the formulas (50-R₁₅₂), (50-R₁₅₃), (50-R₁₅₄), (50-R₁₅₇), and (50-R₁₅₈), X₁₅₁, R₁₅₁ to R₁₆₀ are as defined in the formula (50).
* is a single bond which bonds with L₁₀₁.

In one embodiment, the compound represented by the formula (20) is a compound represented by the following formula (30).

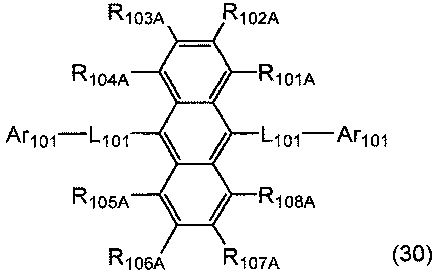

In the formula (30), L₁₀₁ and Ar₁₀₁ are as defined in the formula (10).

Adjacent two of R_{101A} to R_{108A} do not form a substituted or unsubstituted, saturated or unsaturated ring.

R_{101A} to R_{108A} are independently
a hydrogen atom, or
a substituent R.

The substituent R is as defined in the formula (10).

In other words, the compound represented by the formula (30) is a compound having two groups represented by the formula (11).

The compound represented by the formula (30) has substantially only protium atoms as hydrogen atoms.

Note that "has substantially only protium atoms" means the case where the ratio of protium compound based on the total moles of the compound having only protium atoms as hydrogen atoms (protium compound) and the compound having the same structure as the former and having a deuterium atom as a hydrogen atom (deuterium compound), is 90 mol% or more, 95 mol% or more, or 99 mol% or more.

In one embodiment, the compound represented by the formula (30) is a compound represented by the following formula (31).

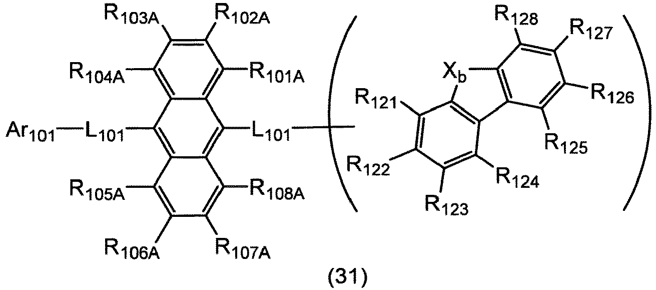

In the formula (31), L₁₀₁ and Ar₁₀₁ are as defined in the formula (10).

R_{101A} to R_{108A} are as defined in the formula (30) above.

X_{b} is O, S, N(R₁₃₁), or C(R₁₃₂)(R₁₃₃).

One of R₁₂₁ to R₁₂₈ and R₁₃₁ to R₁₃₃ is a single bond which bonds with L₁₀₁.

One or more sets of adjacent two or more of R₁₂₁ to R₁₂₈ which are not a single bond which bonds with L₁₀₁ form a substituted or unsubstituted, saturated or unsaturated ring by bonding with each other, or do not form the substituted or unsubstituted, saturated or unsaturated ring.

R₁₂₁ to R₁₂₈ which are not a single bond which bonds with L₁₀₁ and do not form the substituted or unsubstituted, saturated or unsaturated ring are independently a hydrogen atom, or
a substituent R.

The substituent R is as defined in the formula (10).

R₁₃₁ to R₁₃₃ which are not a single bond which bonds with L₁₀₁ are independently
a hydrogen atom,
a substituted or unsubstituted alkyl group including 1 to 50 carbon atoms,
a substituted or unsubstituted cycloalkyl group including 3 to 50 ring carbon atoms,
a substituted or unsubstituted aryl group including 6 to 50 ring carbon atoms, or
a substituted or unsubstituted monovalent heterocyclic group including 5 to 50 ring atoms.

When two or more of each of R₁₃₁ to R₁₃₃ are present, the two or more of each of R₁₃₁ to R₁₃₃ may be the same as or different from each other.

In one embodiment, the compound represented by the formula (31) is a compound represented by the following formula (32).

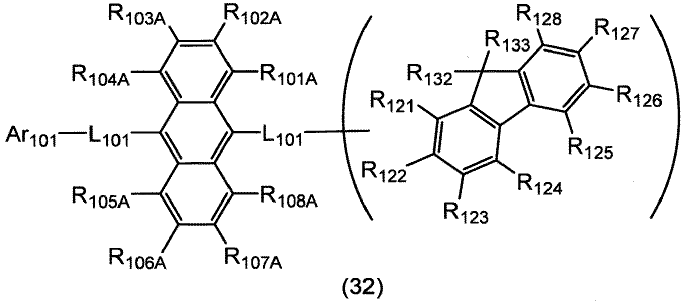

In the formula (32), R_{101A} to R_{108A}, L₁₀₁, Ar₁₀₁, R₁₂₁ to R₁₂₈, R₁₃₂, and R₁₃₃ are as defined in the formula (31).

In one embodiment, the compound represented by the formula (31) is a compound represented by the following formula (33).

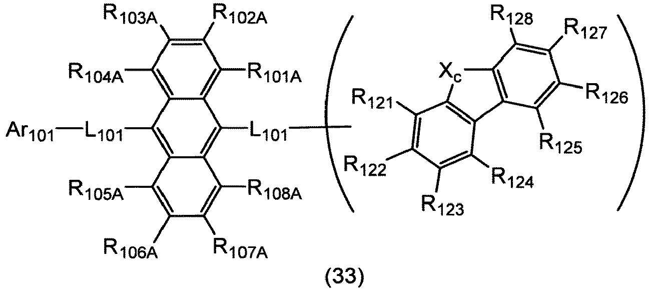

In the formula (33), R_{101A} to R_{108A}, L₁₀₁, Ar₁₀₁, and R₁₂₁ to R₁₂₈ are as defined in the formula (31).

X_{c} is O, S, or NR₁₃₁.

R₁₃₁ is as defined in the formula (31).

In one embodiment, the compound represented by the formula (31) is a compound represented by the following formula (34).

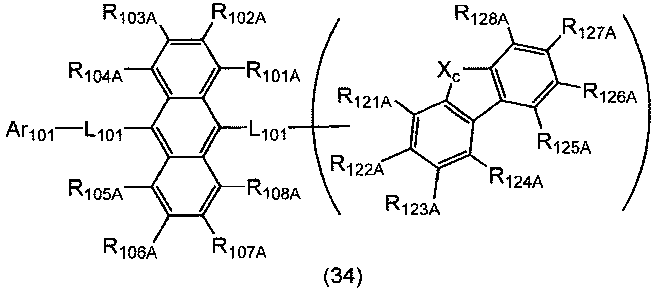

In the formula (34), R_{101A} to R_{108A}, L₁₀₁, and Ar₁₀₁ are as defined in the formula (31).

X_{c} is O, S, or NR₁₃₁.

R₁₃₁ is as defined in the formula (31).

One of R_{121A} to R_{128A} is a single bond which bonds with L₁₀₁.

One or more sets of adjacent two or more of R_{121A} to R_{128A} which are not a single bond which bonds with L₁₀₁ do not form a substituted or unsubstituted, saturated or unsaturated ring.

R_{121A} to R_{128A} which are not a single bond which bonds with L₁₀₁ are independently a hydrogen atom, or
a substituent R.

The substituent R is as defined in the formula (10).

In one embodiment, the compound represented by the formula (31) is a compound represented by the following formula (35).

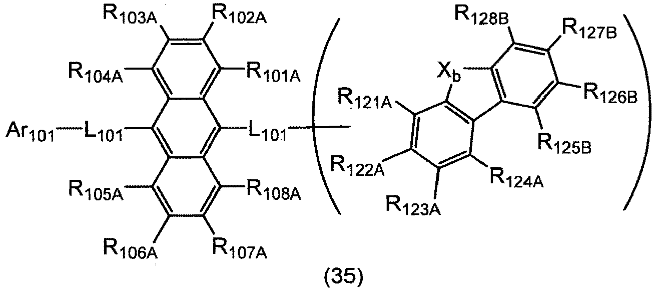

In the formula (35), R_{101A} to R_{108A}, L₁₀₁, Ar₁₀₁, and X_{b} are as defined in the formula (31).

One or more sets of adjacent two or more of R_{121A} to R_{124A} do not form a substituted or unsubstituted, saturated or unsaturated ring by bonding with each other.

Any one set of R_{125B} and R_{126B}, R_{126B} and R_{127B}, and R_{127B} and R_{128B} form a ring represented by the following formula (35a) or (35b) by bonding with each other.

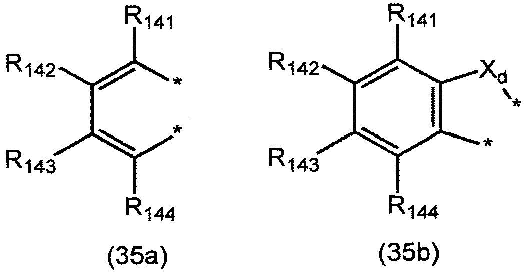

In the formulas (35a) and (35b),
two *s are respectively bonded with any one set of R_{125B} and R_{126B}, R_{126B} and R_{127B}, and R_{127B} and R_{128B}.

R₁₄₁ to R₁₄₄ are independently
a hydrogen atom, or
a substituent R.

The substituent R is as defined in the formula (10).

X_{d} is O or S.

One of R_{121A} to R_{124A} R_{125B} to R_{128B} which do not form the ring represented by the formula (35a) or (35b), and R₁₄₁ to R₁₄₄ is a single bond which bonds with L₁₀₁.

R_{121A} to R_{124A} which are not a single bond which bonds with L₁₀₁ and R_{125B} to R_{128B} which are not a single bond which bonds with L₁₀₁ and do not form the ring represented by the formula (35a) or (35b) are independently
a hydrogen atom, or
a substituent R.

The substituent R is as defined in the formula (10).

In one embodiment, the compound represented by the formula (35) is a compound represented by the following formula (36).

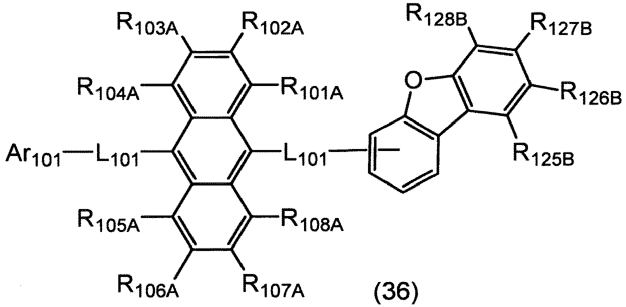

In the formula (36), R_{101A} to R_{108A}, L₁₀₁, Ar₁₀₁, and R_{125B} to R_{128B} are as defined in the formula (35).

In one embodiment, the compound represented by the formula (34) is a compound represented by the following formula (37).

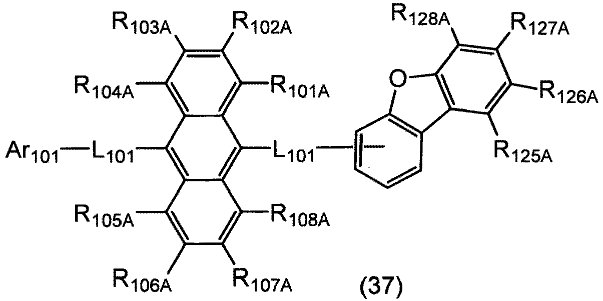

In the formula (37), R_{101A} to R_{108A}, R_{125A} to R_{128A}, L₁₀₁, and Ar₁₀₁ are as defined in the formula (34).

In one embodiment, R_{101A} to R_{108A} in the formulas (30) to (37) are hydrogen atoms.

In one embodiment, the compound represented by the formula (10) is a compound represented by the following formula (40).

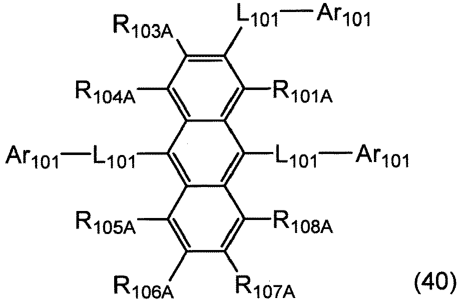

In the formula (40), L₁₀₁ and Ar₁₀₁ are as defined in the formula (10).

One or more sets of adjacent two or more of R_{101A} and R_{103A} to R_{108A} form a substituted or unsubstituted, saturated or unsaturated ring by bonding with each other, or do not form the substituted or unsubstituted, saturated or unsaturated ring.

R_{101A} and R_{103A} to R_{108A} which do not form the substituted or unsubstituted, saturated or unsaturated ring are independently
a hydrogen atom, or
a substituent R.

The substituent R is as defined in the formula (10).

In other words, the compound represented by the formula (40) is a compound having three groups represented by the formula (11). The compound represented by the formula (40) has substantially only protium atoms as hydrogen atoms.

In one embodiment, the compound represented by the formula (40) is a compound represented by the following formula (41).

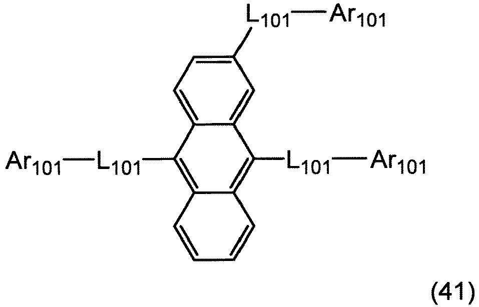

In the formula (41), L₁₀₁ and Ar₁₀₁ are as defined in the formula (40).

In one embodiment, the compound represented by the formula (40) is a compound represented by any one of the following formulas (42-1) to (42-3).

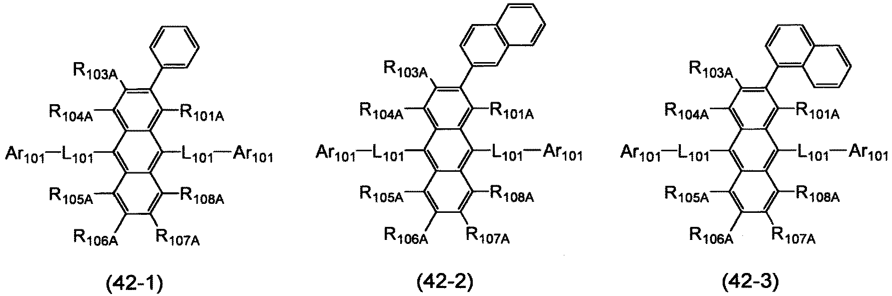

In the formula (42-1) to (42-3), R_{101A} to R_{108A}, L₁₀₁, and Ar₁₀₁ are as defined in the formula (40).

In one embodiment, the compound represented by the formulas (42-1) to (42-3) is a compound represented by any one of the following formulas (43-1) to (43-3).

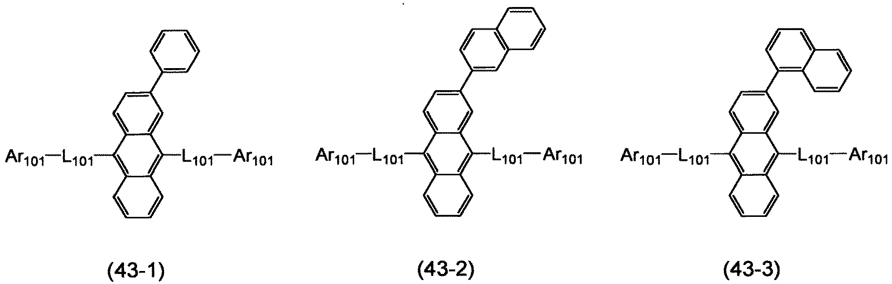

In the formulas (43-1) to (43-3), L₁₀₁ and Ar₁₀₁ are as defined in the formula (40).

In one embodiment, the group represented by -L₁₀₁-Ar₁₀₁ in the formulas (40), (41), (42-1) to (42-3), and (43-1) to (43-3) is selected from the group consisting of
a substituted or unsubstituted phenyl group,
a substituted or unsubstituted naphthyl group,
a substituted or unsubstituted biphenyl group,
a substituted or unsubstituted phenanthrenyl group,
a substituted or unsubstituted benzophenanthrenyl group,
a substituted or unsubstituted fluorenyl group,
a substituted or unsubstituted benzofluorenyl group,
a substituted or unsubstituted dibenzofuranyl group,
a substituted or unsubstituted naphthobenzofuranyl group,
a substituted or unsubstituted dibenzothiophenyl group, and
a substituted or unsubstituted carbazolyl group.

In one embodiment, the compound represented by the formula (10) or the formula (20) include a compound in which at least one of the hydrogen atoms possessed by these compounds is a deuterium atom.

In one embodiment, at least one of
R₁₀₁ to R₁₀₈ which are hydrogen atoms,
hydrogen atoms possessed by R₁₀₁ to R₁₀₈, which are the substituent R,
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
in the formula (20) is a deuterium atom.

The compounds represented by each of the formulas (30) to (37) include a compound in which at least one of the hydrogen atoms possessed by these compounds is a deuterium atom.

In one embodiment, at least one of the hydrogen atoms which bonds with the carbon atoms constituting the anthracene skeleton in the compounds represented by each of the formulas (30) to (37) is a deuterium atom.

In one embodiment, the compound represented by the formula (30) is a compound represented by the following formula (30D).

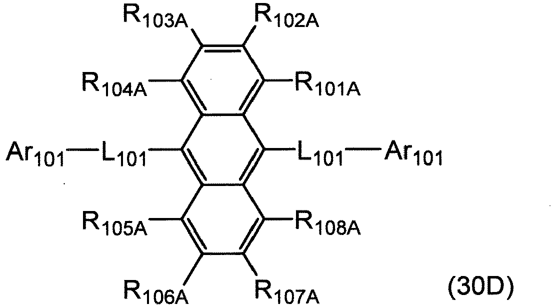

In the formula (30D), R_{101A} to R_{108A}, L₁₀₁, and Ar₁₀₁ are as defined in the formula (30).

Provided that at least one of:
R_{101A} to R_{110A}, which are hydrogen atoms,
hydrogen atoms possessed by R_{101A} to R_{110A}, which are the substituent R,
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
is a deuterium atom.

In other words, the compound represented by the formula (30D) is a compound in which at least one of the hydrogen atoms possessed by the compound represented by the formula (30) is a deuterium atom.

In one embodiment, at least one of R_{101A} to R_{108A}, which is a hydrogen atom, in the formula (30D) is a deuterium atom.

In one embodiment, the compound represented by the formula (30D) is a compound represented by the following formula (31D).

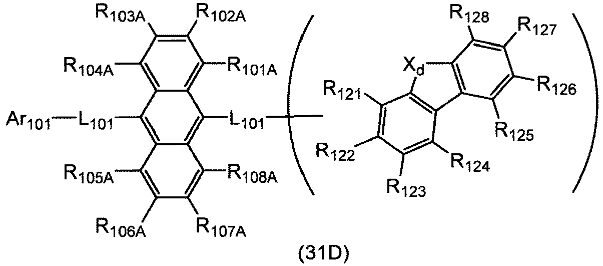

In the formula (31D), R_{101A} to R_{108A}, L₁₀₁, and Ar₁₀₁ are as defined in the formula (30D).

X_{d} is O or S.

One of R₁₂₁ to R₁₂₈ is a single bond which bonds with L₁₀₁.

One or more sets of adjacent two or more of R₁₂₁ to R₁₂₈ which are not a single bond which bonds with L₁₀₁ form a substituted or unsubstituted, saturated or unsaturated ring or do not form a substituted or unsubstituted by bonding with each other, saturated or unsaturated ring.

R₁₂₁ to R₁₂₈ which are not a single bond which bonds with L₁₀₁ and do not form the substituted or unsubstituted, saturated or unsaturated ring are independently
a hydrogen atom, or
a substituent R.

The substituent R is as defined in the formula (10).

Provided that at least one of:
R_{101A} to R_{110A} which are hydrogen atoms,
hydrogen atoms possessed by R_{101A} to R_{110A}, which are the substituent R,
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁,
R₁₂₁ to R₁₂₈ which are hydrogen atoms, and
hydrogen atoms possessed by R₁₂₁ to R₁₂₈, which are the substituent R
is a deuterium atom.

In one embodiment, the compound represented by the formula (31D) is a compound represented by the following formula (32D).

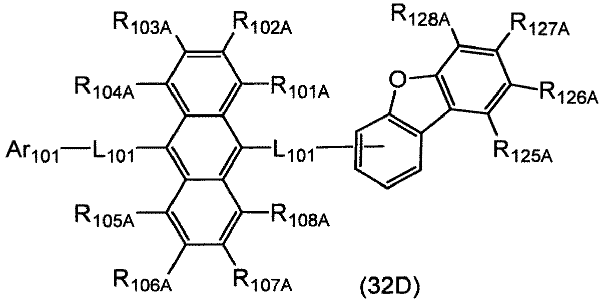

In the formula (32D), R_{101A} to R_{108A}, R_{125A} to R_{128A}, L₁₀₁, and Ar₁₀₁ are as defined in the formula (31D).

Provided that at least one of
R_{101A} to R_{108A}, which are hydrogen atoms,
hydrogen atoms possessed by R_{101A} to R_{108A} which are the substituent R,
R_{125A} to R_{128A} which are hydrogen atoms,
hydrogen atoms possessed by R_{125A} to R_{128A}, which are the substituent R,
hydrogen atoms which bonds with the carbon atoms constituting the dibenzofuran skeleton in the formula (32D),
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
is a deuterium atom.

In one embodiment, the compound represented by the formula (32D) is a compound represented by the following formula (32D-1) or (32D-2).

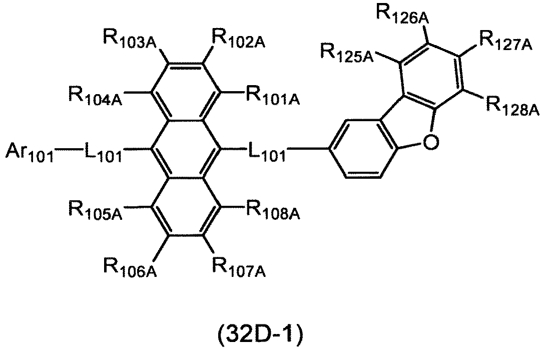

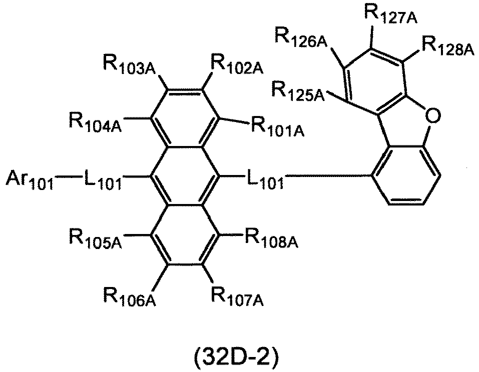

In the formulas (32D-1) and (32D-2), R_{101A} to R_{108A}, R_{125A} to R_{128A}, L₁₀₁, and Ar₁₀₁ are as defined in the formula (32D).

Provided that at least one of
R_{101A} to R_{108A} which are hydrogen atoms,
hydrogen atoms possessed by R_{101A} to R_{108A}, which are the substituent R,
R_{125A} to R_{128A} which are hydrogen atoms,
hydrogen atoms possessed by R_{125A} to R_{128A}, which are the substituent R,
hydrogen atoms which bonds with the carbon atom constituting the dibenzofuran skeleton in the formula (32D-1) or (32D-2),
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
is a deuterium atom.

In one embodiment, at least one of the hydrogen atoms possessed by the compounds represented by each of the formulas (40), (41), (42-1) to (42-3), and (43-1) to (43-3) is a deuterium atom.

In one embodiment, at least one of the hydrogen atoms which bond with the carbon atoms constituting the anthracene skeleton in the compound represented by the formula (41) (R_{101A} to R_{108A} which are hydrogen atoms) is a deuterium atom.

In one embodiment, the compound represented by the formula (40) is a compound represented by the following formula (40D).

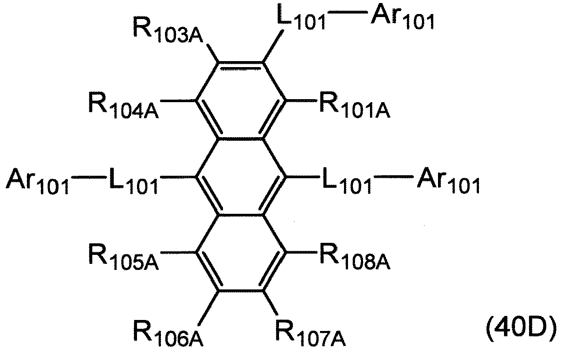

In the formula (40D), L₁₀₁ and Ar₁₀₁ are as defined in the formula (10).

One or more sets of adjacent two or more of R_{101A} and R_{103A} to R_{108A} do not form a substituted or unsubstituted, saturated or unsaturated ring.

R_{101A} and R_{103A} to R_{108A} are independently
a hydrogen atom, or
a substituent R.

The substituent R is as defined in the formula (10).

Provided that at least one of:
R_{101A} and R_{103A} to R_{108A}, which are hydrogen atoms,
hydrogen atoms possessed by R_{101A} and R_{103A} to R_{108A}, which are the substituent R,
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
is a deuterium atom.

In one embodiment, at least one of R_{101A} and R_{103A} to R_{108A} in the formula (40D) is a deuterium atom.

In one embodiment, the compound represented by the formula (40D) is a compound represented by the following formula (41D).

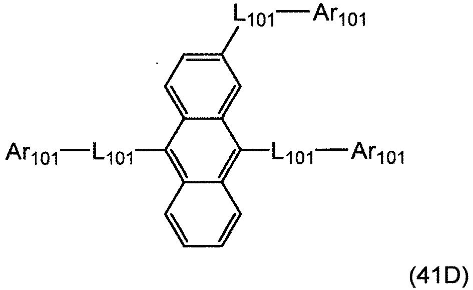

In the formula (41D), L₁₀₁ and Ar₁₀₁ are as defined in the formula (40D).

Provided that in the formula (41D), at least one of
hydrogen atoms which bond with the carbon atoms constituting the anthracene skeleton,
hydrogen atoms possessed by L₁₀₁,
hydrogen atoms possessed by the substituent of L₁₀₁,
hydrogen atoms possessed by Ar₁₀₁, and
hydrogen atoms possessed by the substituent of Ar₁₀₁
is a deuterium atom.

In one embodiment, the compound represented by the formula (40D) is a compound represented by any one of the following formulas (42D-1) to (42D-3).

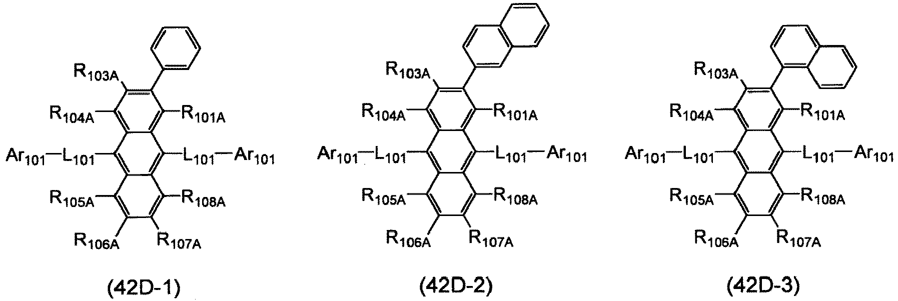

In the formulas (42D-1) to (42D-3), R_{101A} to R_{108A}, L₁₀₁, and Ar₁₀₁ are as defined in the formula (40D).

Provided that in the formula (42D-1), at least one of:
R_{101A} and R_{103A} to R_{108A}, which are hydrogen atoms,
hydrogen atoms possessed by R_{101A} and R_{103A} to R_{108A}, which are the substituent R,
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the phenyl group in the formula (42D-1) is a deuterium atom.

In the formula (42D-2), at least one of:
R_{101A} and R_{103A} to R_{108A}, which are hydrogen atoms,
hydrogen atoms possessed by R_{101A} and R_{103A} to R_{108A}, which are the substituent R,
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the naphthyl group in the formula (42D-2)
is a deuterium atom.

In the formula (42D-3), at least one of:
R_{101A} and R_{103A} to R_{108A}, which are hydrogen atoms,
hydrogen atoms possessed by R_{101A} and R_{103A} to R_{108A}, which are the substituent R,
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the naphthyl group in the formula (42D-3) is a deuterium atom.

In one embodiment, the compounds represented by the formulas (42D-1) to (42D-3) are compounds represented by the following formulas (43D-1) to (43D-3).

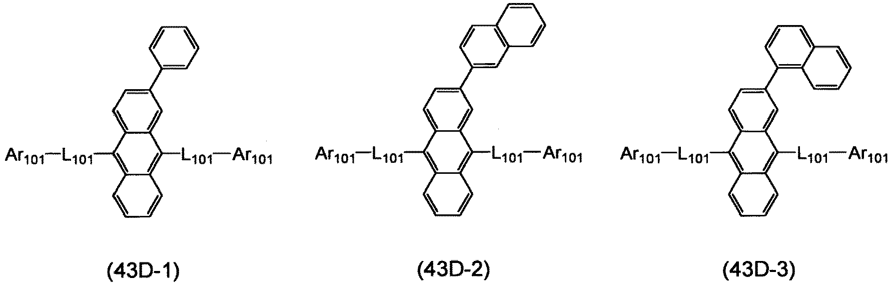

In the formulas (43D-1) to (43D-3), L₁₀₁ and Ar₁₀₁ are as defined in the formula (40D).

Provided that at least one of hydrogen atoms which bond with the carbon atoms constituting the anthracene skeleton in the formula (43D-1),
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the phenyl group in the formula (43D-1)
is a deuterium atom.

At least one of hydrogen atoms which bond with the carbon atoms constituting the anthracene skeleton in the formula (43D-2),
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the naphthyl group in the formula (43D-2)
is a deuterium atom.

At least one of hydrogen atoms which bond with the carbon atoms constituting the anthracene skeleton in the formula (43D-3),
hydrogen atoms possessed by L₁₀₁,
hydrogen atoms possessed by the substituent of L₁₀₁,
hydrogen atoms possessed by Ar₁₀₁,
hydrogen atoms possessed by the substituent of Ar₁₀₁, and
hydrogen atoms which bond with the carbon atoms constituting the naphthyl group in the formula (43D-3)
is a deuterium atom.

Specific examples of the compound represented by the formula (10) include the following compounds. The compound represented by the formula (10) is not limited to these specific examples. In the following specific examples, Me represents a methyl group, and D represents a deuterium atom.

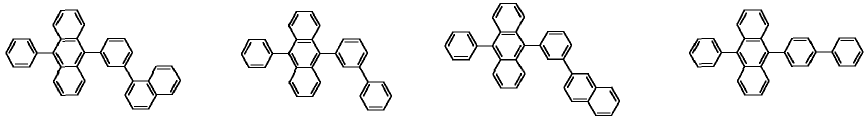

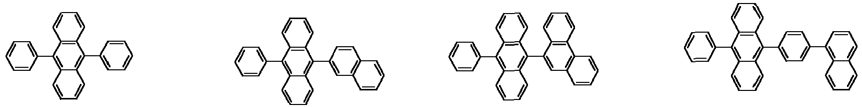

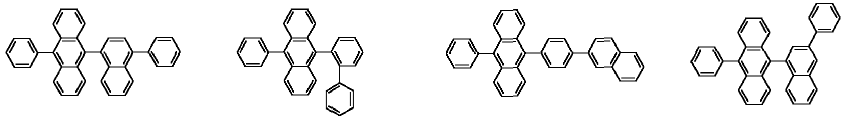

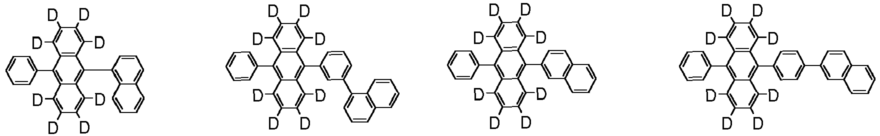

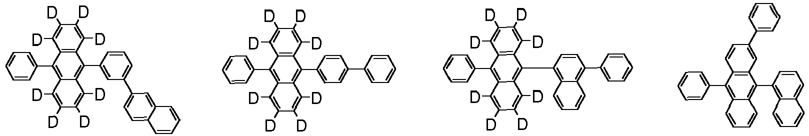

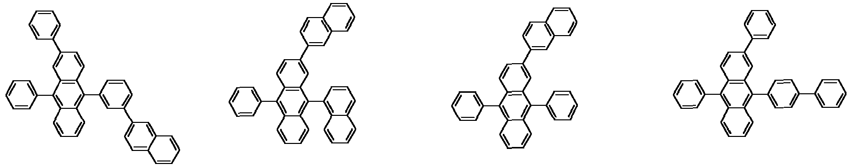

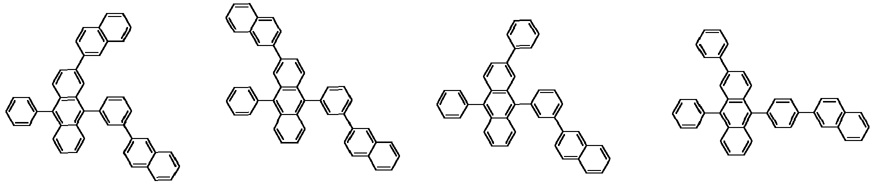

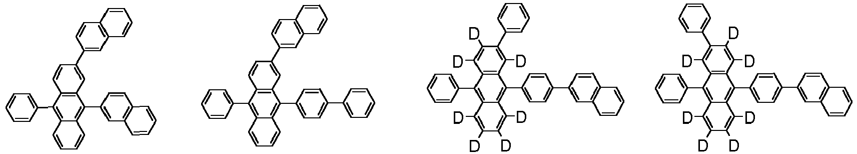

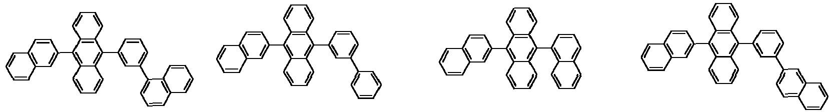

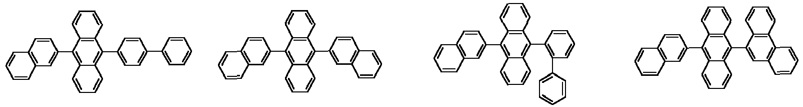

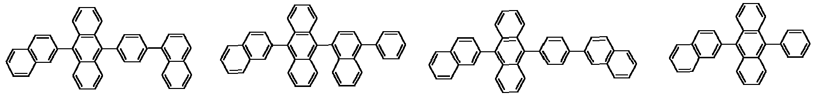

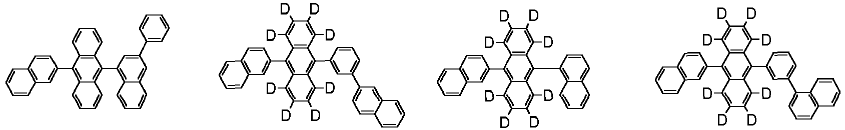

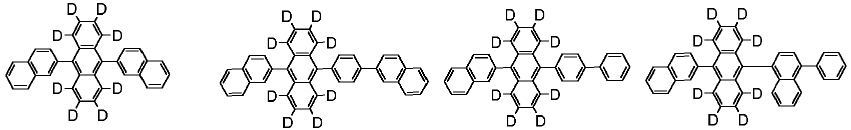

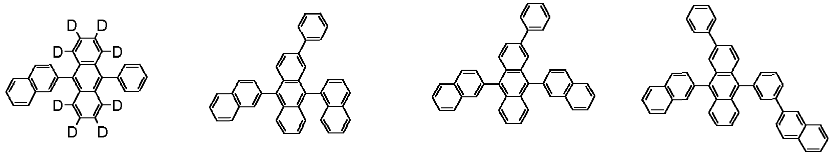

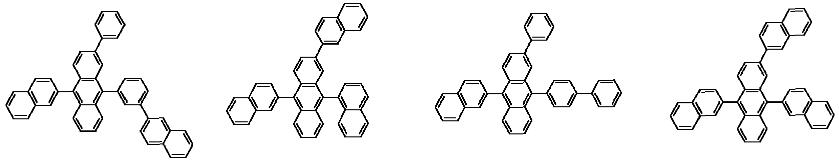

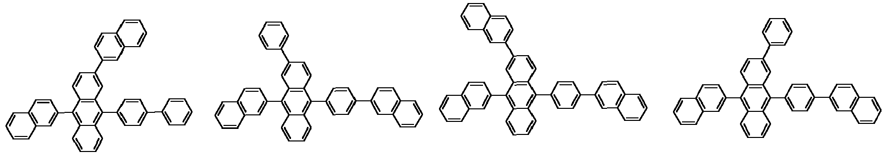

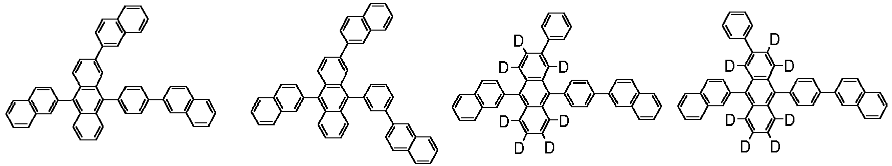

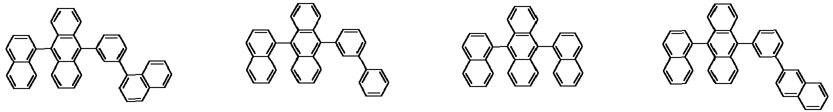

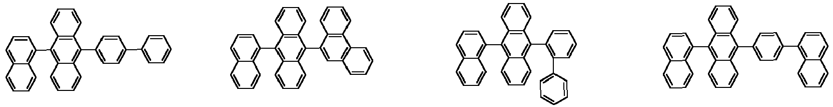

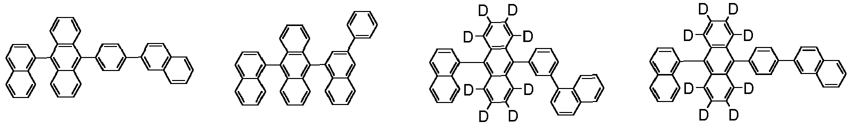

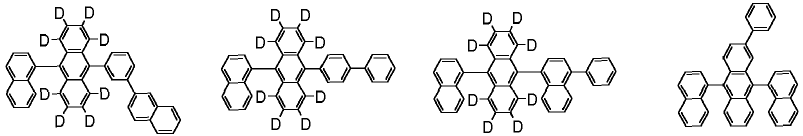

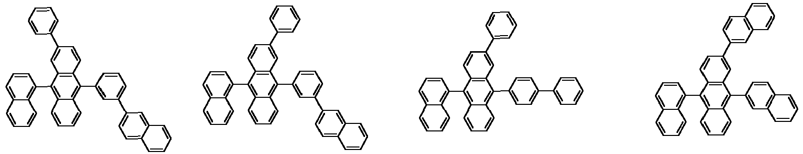

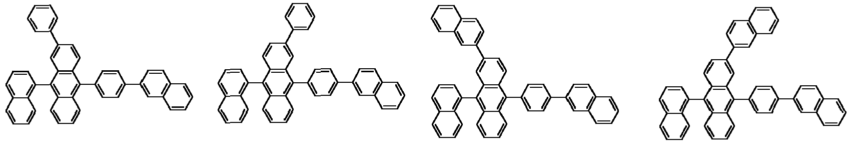

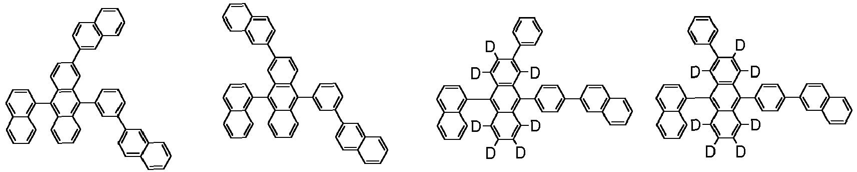

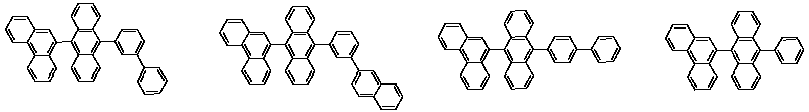

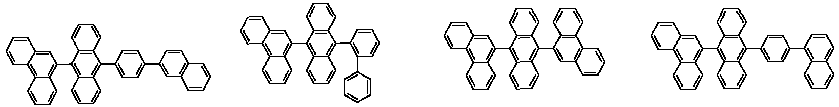

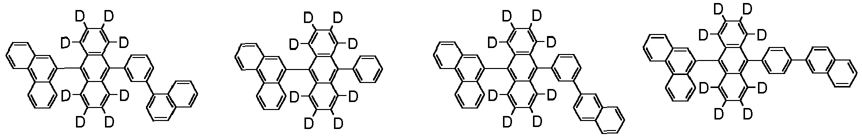

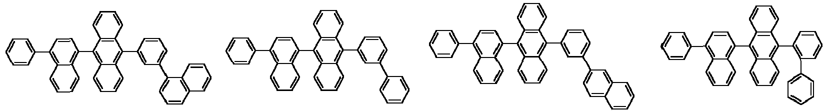

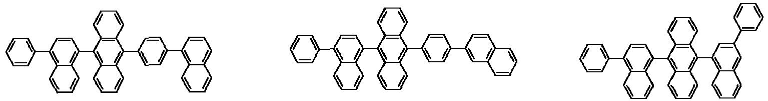

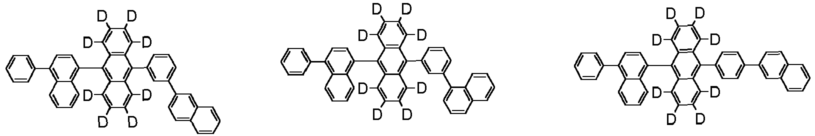

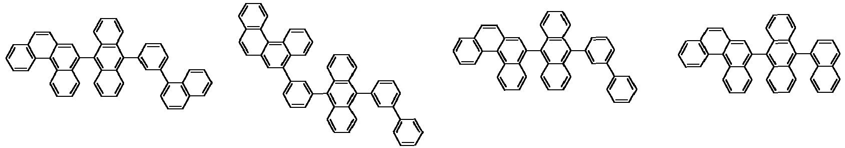

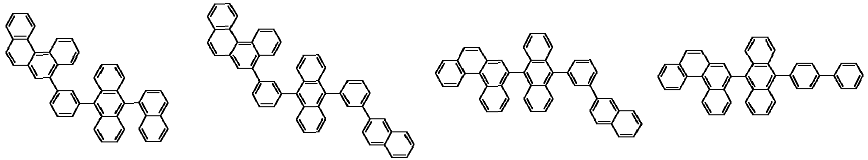

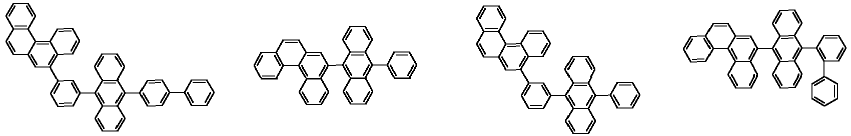

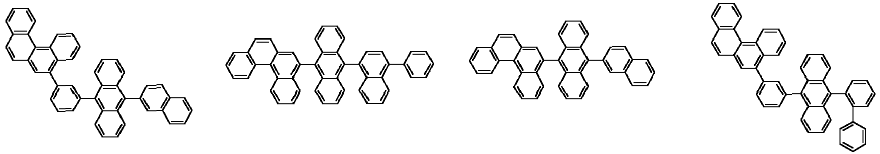

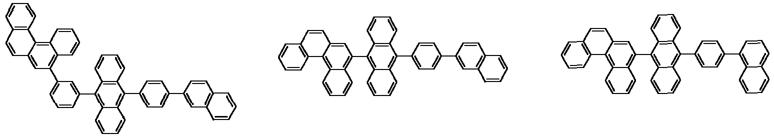

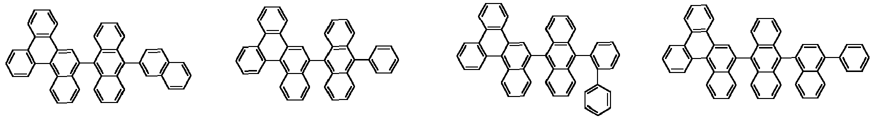

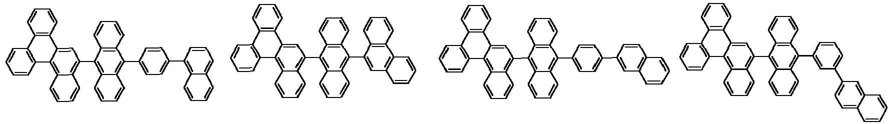

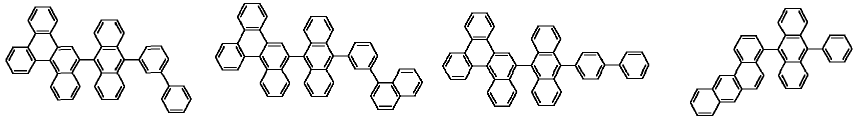

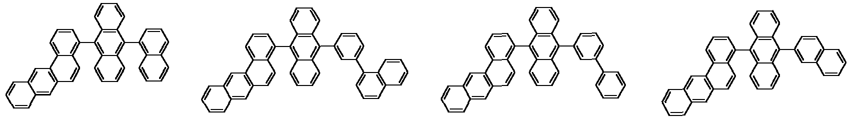

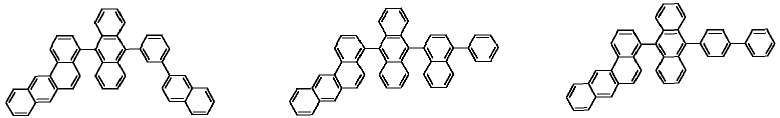

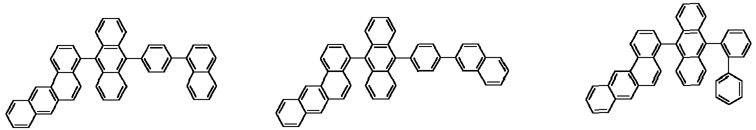

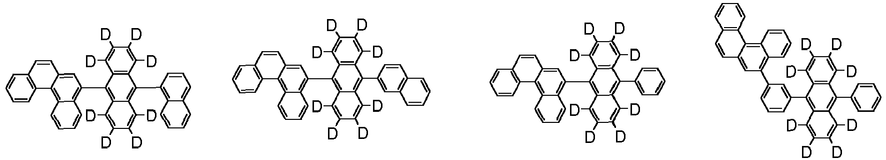

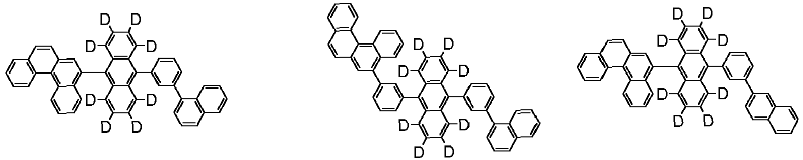

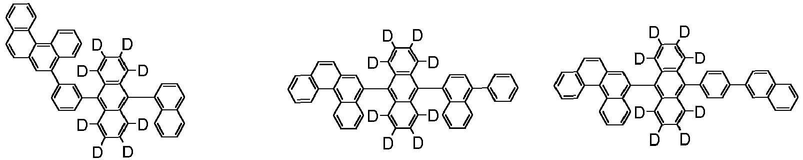

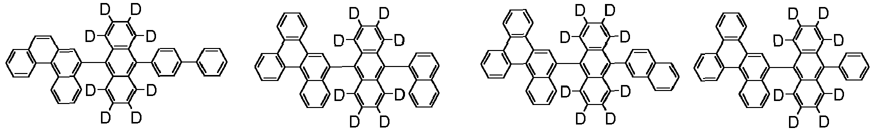

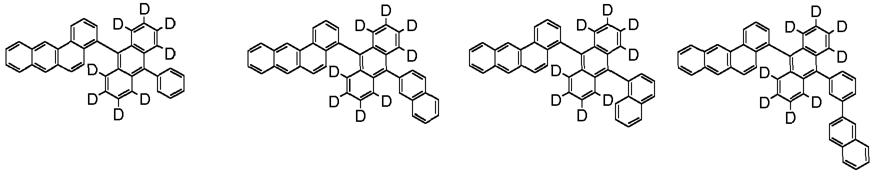

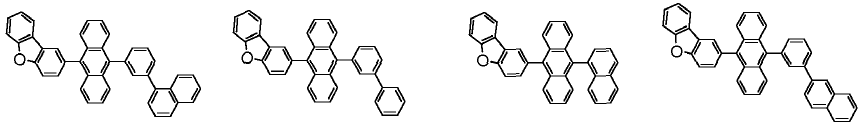

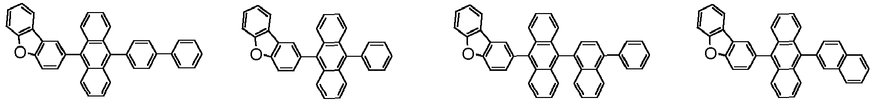

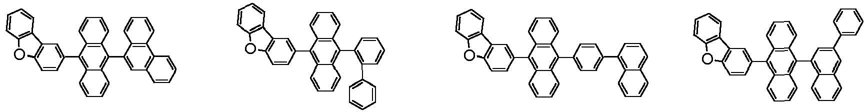

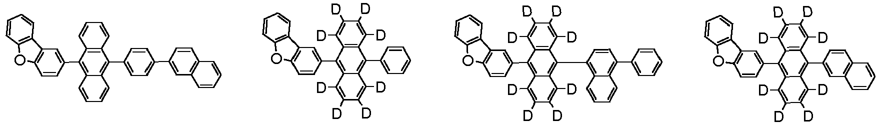

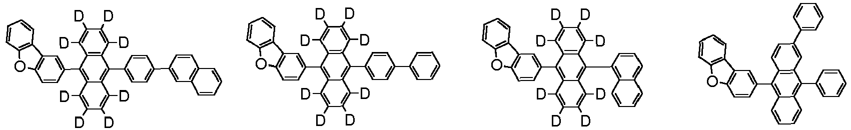

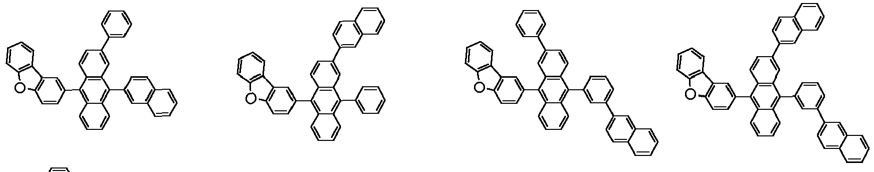

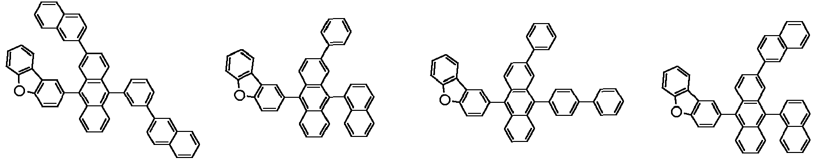

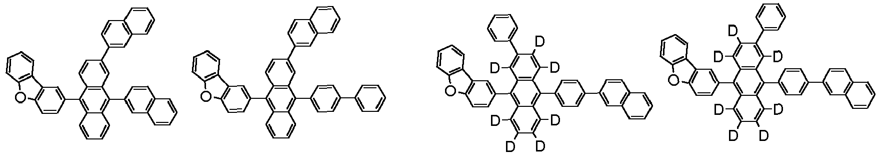

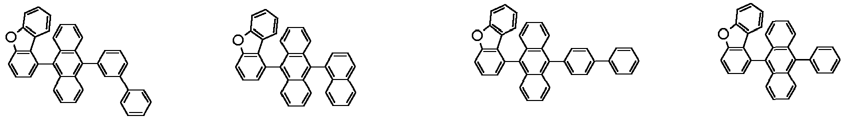

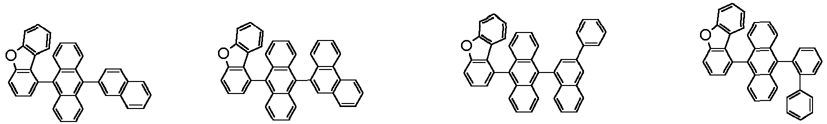

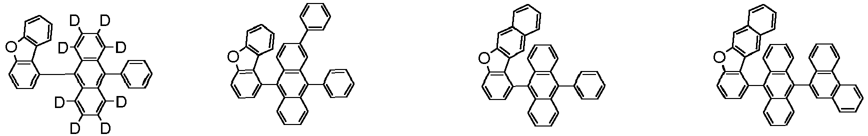

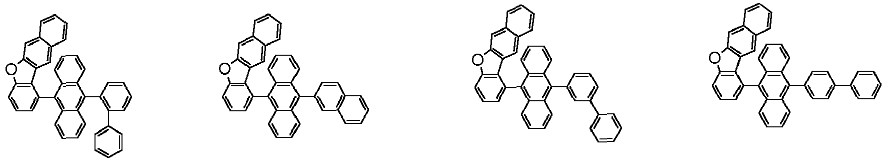

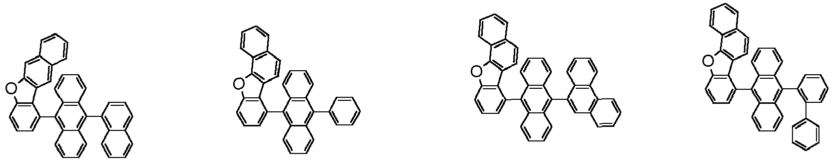

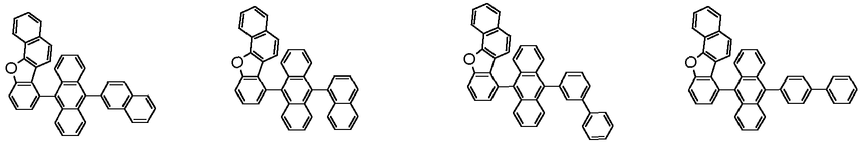

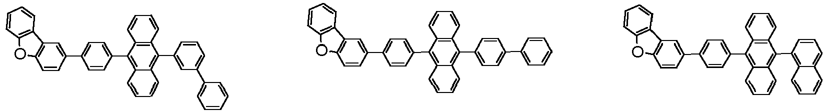

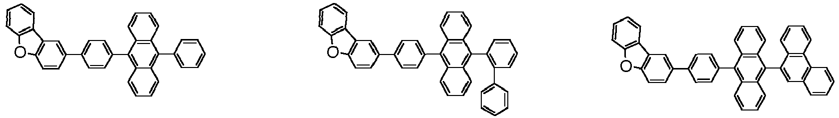

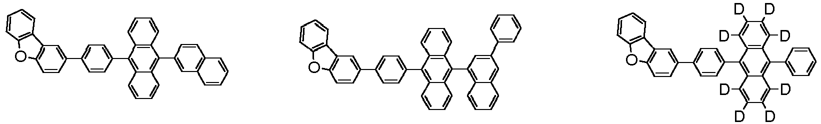

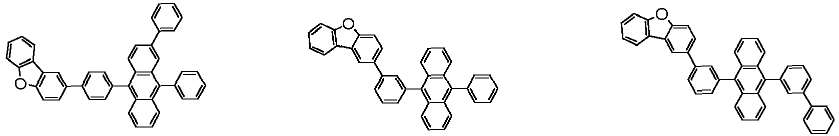

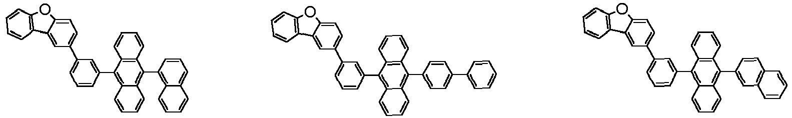

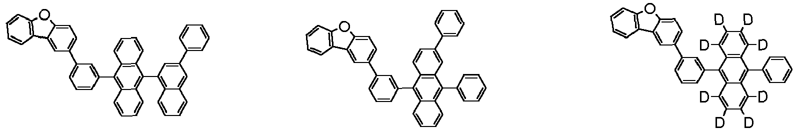

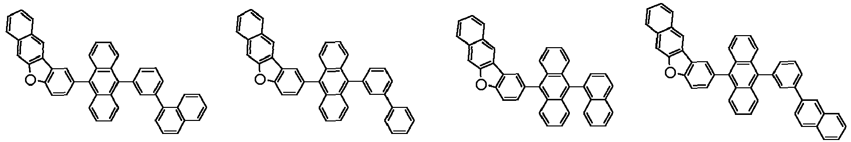

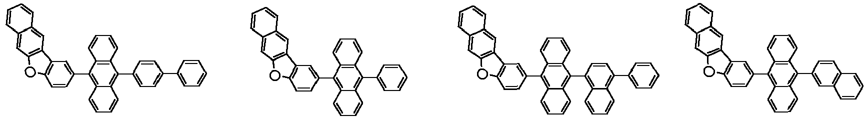

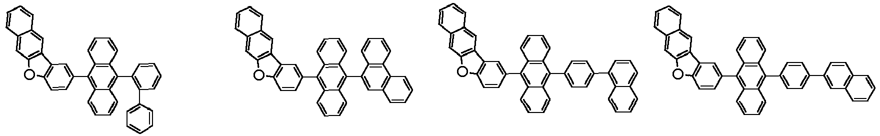

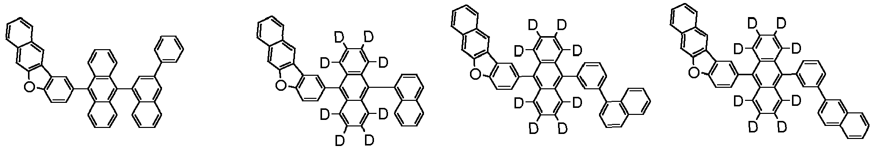

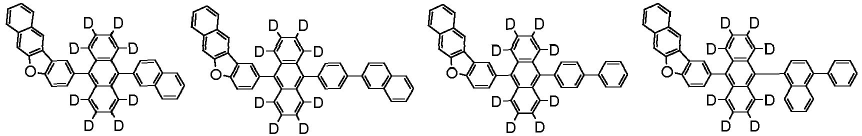

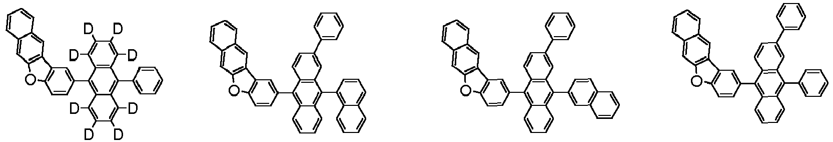

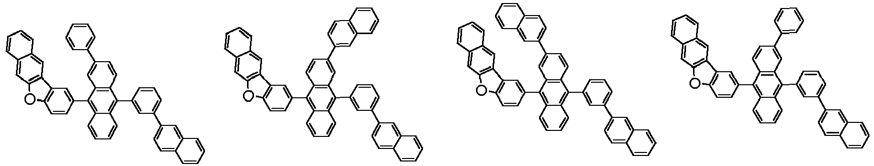

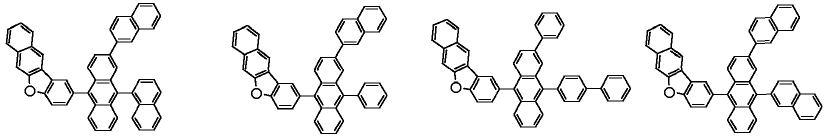

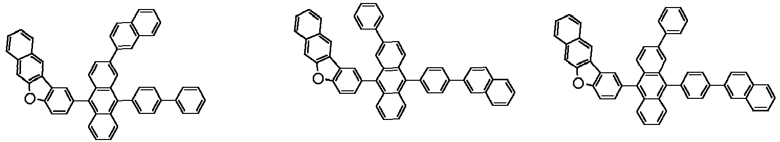

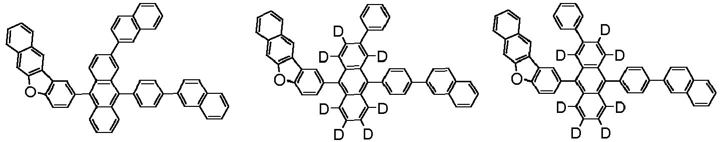

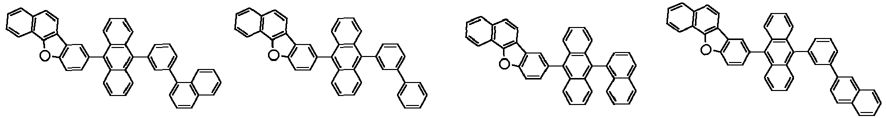

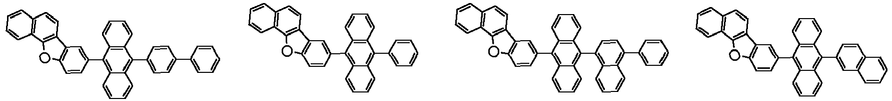

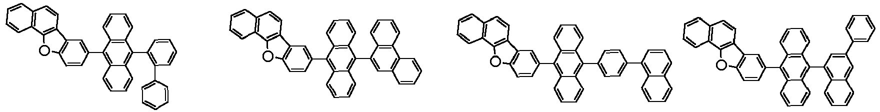

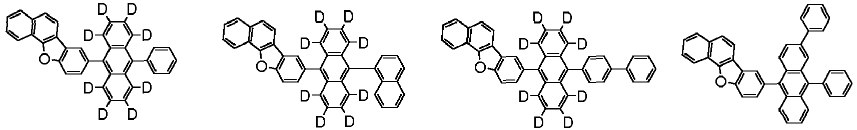

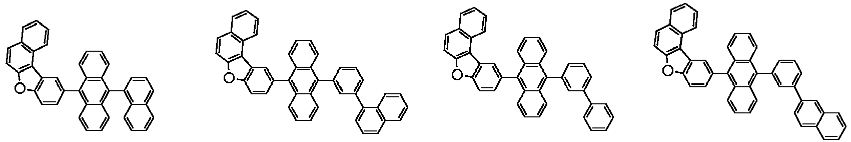

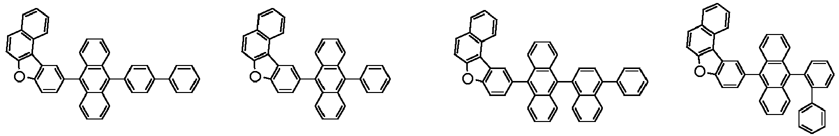

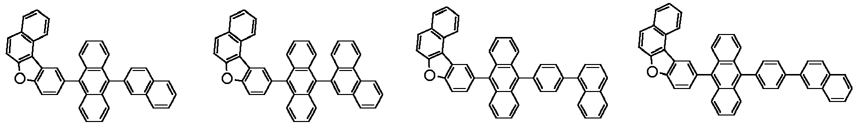

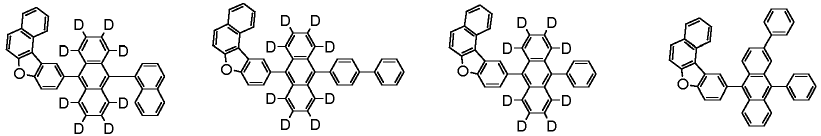

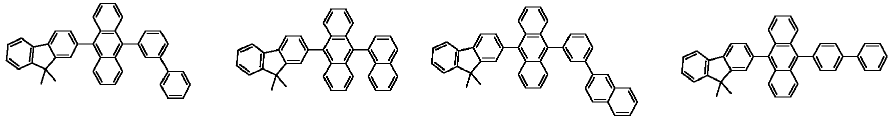

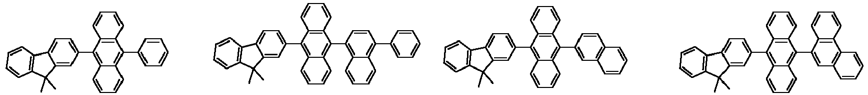

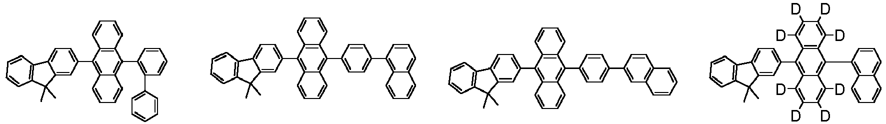

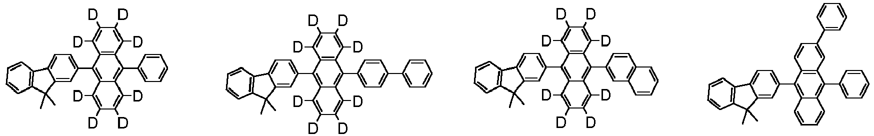

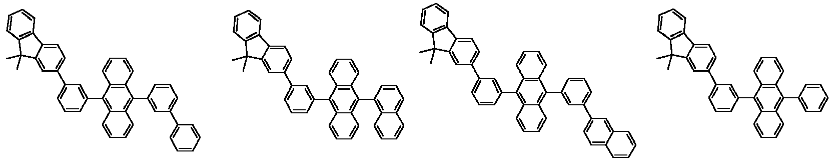

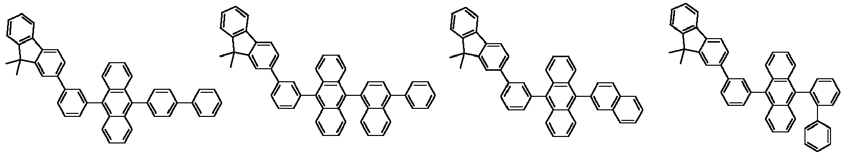

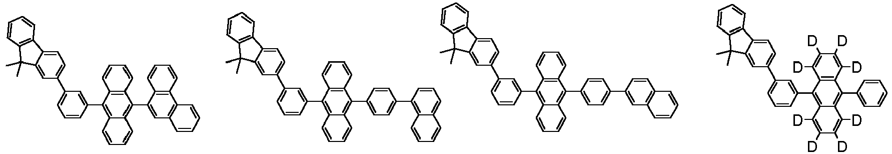

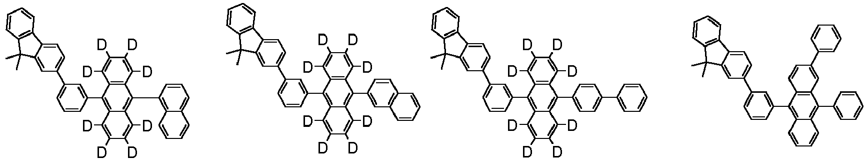

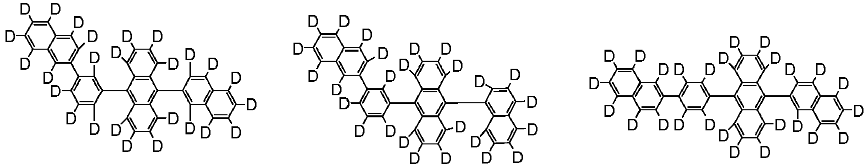

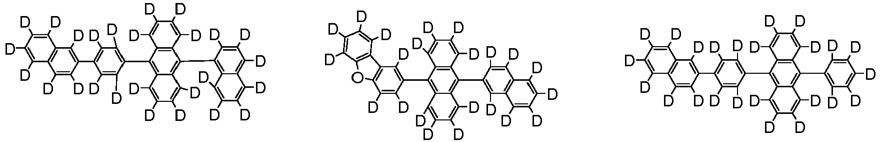

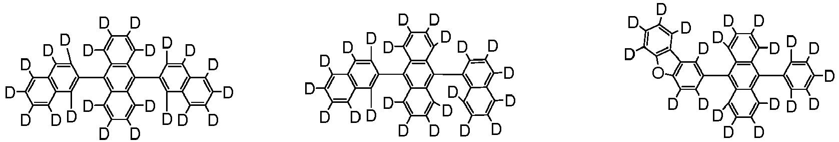

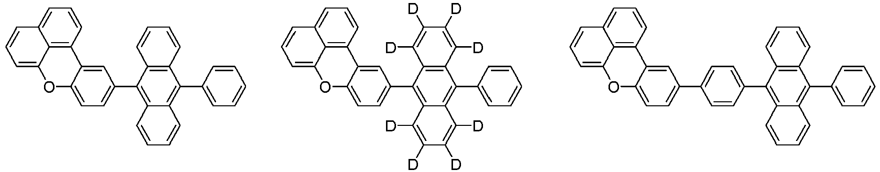

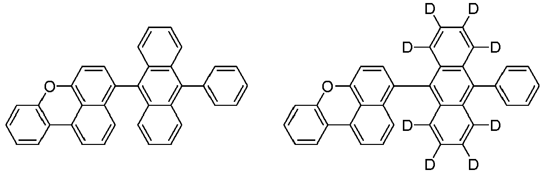

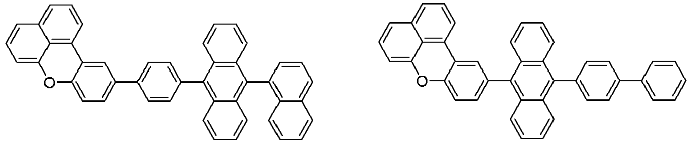

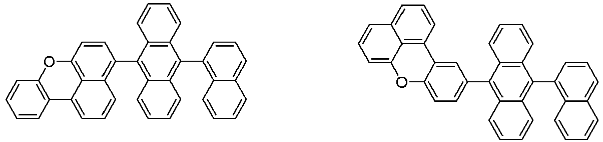

As described above, known materials and device configurations may be applied to the organic EL device according to an aspect of the invention, as long as the device contains a cathode, an anode, and an emitting layer between the cathode and the anode, wherein the emitting layer contains a compound represented by the formulas (9-2) and (9-3), and the effect of the invention is not impaired.

The content of the compound represented by the formulas (9-2) and (9-3)in the emitting layer is preferably 1% by mass or more and 20% by mass or less based on the total mass of the emitting layer.

Specific examples of a typified device configuration of the organic EL device of the invention include structures such as
(1) an anode/ an emitting layer/ a cathode,
(2) an anode/ a hole-injecting layer/ an emitting layer/ a cathode,
(3) an anode/ an emitting layer/ an electron-injecting-transporting layer/ a cathode,
(4) an anode/ a hole-injecting layer/ an emitting layer/ an electron-injecting (and/or) transporting layer/ a cathode,
(5) an anode/ an organic semiconductor layer/ an emitting layer/ a cathode,
(6) an anode/ an organic semiconductor layer/ an electron barrier layer/ an emitting layer/ a cathode,
(7) an anode/ an organic semiconductor layer/ an emitting layer/ an adhesion improving layer/ a cathode,
(8) an anode/ a hole-injecting (and/or) transporting layer/ an emitting layer/ an electron-injecting (and/or) transporting layer/ a cathode,
(9) an anode/ an insulating layer/ an emitting layer/ an insulating layer/ a cathode,
(10) an anode/ an inorganic semiconductor layer/ an insulating layer/ an emitting layer/ an insulating layer/ a cathode,
(11) an anode/ an organic semiconductor layer/ an insulating layer/ an emitting layer/ an insulating layer/ a cathode,
(12) an anode/ an insulating layer/ a hole-injecting (and/or) transporting layer/ an emitting layer/ an insulating layer/ a cathode, and
(13) an anode/ an insulating layer/ a hole-injecting (and/or) transporting layer/ an emitting layer/ an electron-injecting (and/or) transporting layer/ a cathode.

Among the above-described structures, the configuration of (8) is preferably used, but the device configuration of the organic EL device is not limited thereto.

The "hole-injecting (and/or) transporting layer" in this specification means "at least one of the hole-injecting layer and the hole-transporting layer", and the "electron-injecting (and/or) transporting layer" in this specification means "at least one of the electron-injecting layer and the electron-transporting layer".

Parts which can be used in the organic EL device according to an aspect of the invention, materials for forming respective layers, other than the above compounds, and the like, will be described below.

### (Substrate)

A substrate is used as a support of an emitting device. As the substrate, glass, quartz, plastics or the like can be used, for example. Further, a flexible substrate may be used. The "flexible substrate" means a bendable (flexible) substrate, and specific examples thereof include a plastic substrate formed of polycarbonate, polyvinyl chloride, or the like.

### (Anode)

For the anode formed on the substrate, metals, alloys, electrically conductive compounds, mixtures thereof, and the like, which have a large work function (specifically 4.0 eV or more) are preferably used. Specific examples thereof include indium oxide-tin oxide (ITO: Indium Tin Oxide), indium oxide-tin oxide containing silicon or silicon oxide, indium oxide-zinc oxide, tungsten oxide, indium oxide containing zinc oxide, graphene, and the like. In addition thereto, specific examples thereof include gold (Au), platinum (Pt), a nitride of a metallic material (for example, titanium nitride), and the like.

### (Hole-injecting layer)

The hole-injecting layer is a layer containing a substance having high hole-injecting property. As such a substance having high hole-injecting property molybdenum oxide, titanium oxide, vanadium oxide, rhenium oxide, ruthenium oxide, chromium oxide, zirconium oxide, hafnium oxide, tantalum oxide, silver oxide, tungsten oxide, manganese oxide, an aromatic amine compound, or a polymer compound (oligomers, dendrimers, polymers, etc.) can be given.

### (Hole-transporting layer)

The hole-transporting layer is a layer containing a substance having high hole-transporting property. For the hole-transporting layer, an aromatic amine compound, a carbazole derivative, an anthracene derivative, or the like can be used. A polymer compound such as poly(N-vinylcarbazole) (abbreviation: PVK) and poly(4-vinyltriphenylamine) (abbreviation: PVTPA) can also be used. However, a material other than the above-described materials may be used as long as the material has higher hole-transporting properties of holes in comparison with electrons. It should be noted that the layer containing the material having high hole-transporting properties may be formed into not only a single layer, but also a stacked layer in which two or more layers formed of the above-described materials are stacked.

### (Guest (dopant) material of an emitting layer)

The emitting layer is a layer containing a substance having a high emitting property, and various materials can be used in addition to the materials used in the invention described above (the compound represented by the formulas (9-2) and (9-3)). For example, as the substance having a high emitting property, a fluorescent compound which emits fluorescence or a phosphorescent compound which emits phosphorescence can be used. The fluorescent compound is a compound which can emit from a singlet excited state, and the phosphorescent compound is a compound which can emit from a triplet excited state.

As a blue fluorescent emitting material which can be used for an emitting layer, pyrene derivatives, styrylamine derivatives, chrysene derivatives, fluoranthene derivatives, fluorene derivatives, diamine derivatives, triarylamine derivatives, and the like can be used. As a green fluorescent emitting material which can be used for an emitting layer, aromatic amine derivatives and the like can be used. As a red fluorescent emitting material which can be used for an emitting layer, tetracene derivatives, diamine derivatives and the like can be used.

As a blue phosphorescent emitting material which can be used for an emitting layer, metal complexes such as iridium complexes, osmium complexes, platinum complexes and the like are used. As a green phosphorescent emitting material which can be used for an emitting layer, iridium complexes and the like are used. As a red phosphorescent emitting material which can be used for an emitting layer, metal complexes such as iridium complexes, platinum complexes, terbium complexes, europium complexes and the like are used.

### (Host material for an emitting layer)

The emitting layer may have a constitution in which the substance having a high emitting property (guest material) is dispersed in another substance (host material). As a substance for dispersing the substance having a high emitting property, in addition to the compound used in addition to the materials used in the invention described above (a compound represented by the formula (10)), a variety of substances can be used, and it is preferable to use a substance having a higher lowest unoccupied orbital level (LUMO level) and a lower highest occupied orbital level (HOMO level) rather than the substance having a high emitting property.

As a substance for dispersing the substance having a high emitting property (host material), 1) metal complexes such as aluminum complexes, beryllium complexes, or zinc complexes, 2) heterocyclic compounds such as oxadiazole derivatives, benzimidazole derivatives, or phenanthroline derivatives, 3) condensed aromatic compounds such as carbazole derivatives, anthracene derivatives, phenanthrene derivatives, pyrene derivatives, naphthacenes, fluoranthenes, triphenylene derivatives, fluorene derivatives, or chrysene derivatives, 4) aromatic amine compounds such as triarylamine derivatives or condensed polycyclic aromatic amine derivatives can be used.

A compound having delayed fluorescence (thermally activated delayed fluorescence) can be used as a host material. It is also preferable that the emitting layer contain materials used in the invention described above and a host compound having delayed fluorescence.

### (Electron-transporting layer)

An electron-transporting layer is a layer that comprises a substance having a high electron-transporting property. For the electron-transporting layer, 1) metal complexes such as aluminum complexes, beryllium complexes, zinc complexes, or the like; 2) heteroaromatic complexes such as imidazole derivatives, benzimidazole derivatives, azine derivatives, carbazole derivatives, phenanthroline derivatives, or the like; and 3) polymer compounds can be used.

### (Electron-injecting layer)

An electron-injecting layer is a layer which contains a substance having a high electron-injecting property. For the electron-injecting layer, metal complex compounds such as lithium (Li), ytterbium (Yb), lithium fluoride (LiF), cesium fluoride (CsF), calcium fluoride (CaF₂), 8-hydroxyquinolinolato-lithium (Liq); alkali metals such as lithium oxide (LiOₓ); alkaline earth metals; or a compound thereof can be used.

### (Cathode)

For the cathode, metals, alloys, electrically conductive compounds, mixtures thereof, and the like having a small work function (specifically, 3.8 eV or less) are preferably used. Specific examples of such a cathode material include elements belonging to Group 1 or Group 2 of the Periodic Table of the Elements, i.e., alkali metals such as lithium (Li) and cesium (Cs), alkaline earth metals such as magnesium (Mg), calcium (Ca) and strontium (Sr), and alloys containing these metals (e.g., MgAg and AlLi); rare earth metals such as europium (Eu) and ytterbium (Yb), and alloys containing these metals.

In the organic EL device according to an aspect of the invention, the methods for forming the respective layers are not particularly limited. A conventionally-known method for forming each layer according to a vacuum deposition process, a spin coating process or the like can be used. Each layer such as the emitting layer can be formed by a known method such as a vacuum deposition process, a molecular beam deposition process (MBE process), or an application process such as a dipping process, a spin coating process, a casting process, a bar coating process and a roll coating process, using a solution prepared by dissolving the material in a solvent.

In the organic EL device according to an aspect of the invention, the thickness of each layer is not particularly limited, but is generally preferable that the thickness be in the range of several nm to 1 µm in order to suppress defects such as pinholes, to suppress applied voltages to be low, and to improve luminous efficiency.

### [Electronic apparatus]

The electronic apparatus according to an aspect of the invention is characterized in that the organic EL device according to an aspect of the invention is equipped with.

Specific examples of the electronic apparatus include display components such as an organic EL panel module, and the like; display devices for a television, a cellular phone, a personal computer, and the like; and emitting devices such as a light, a vehicular lamp, and the like.

### Examples

Hereinafter, Examples according to the invention will be described. The invention is not limited in any way by these Examples.

### <Compound>

The compound represented by the formulas (9-2) and (9-3)used in Examples are shown below.

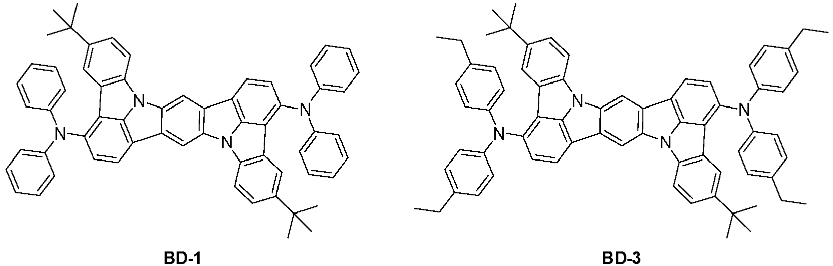

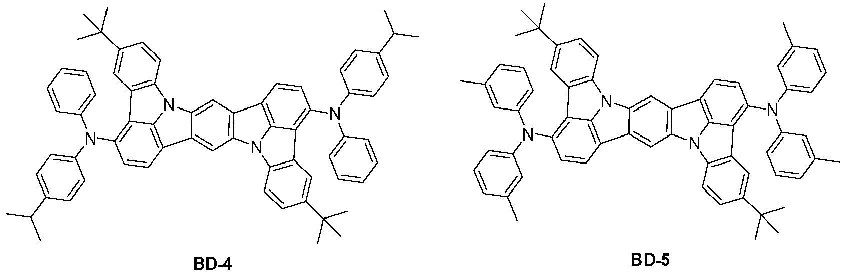

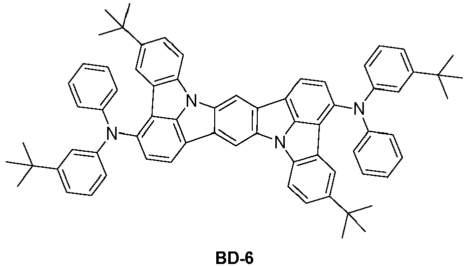

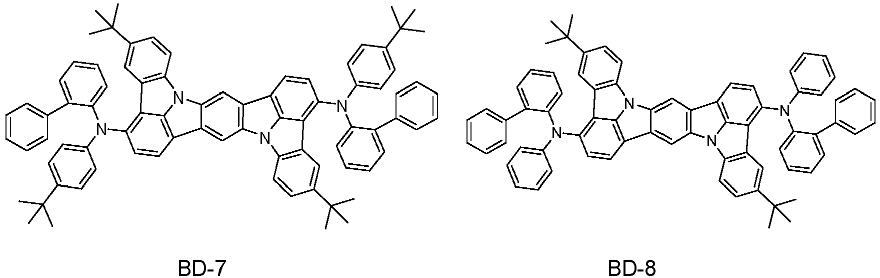

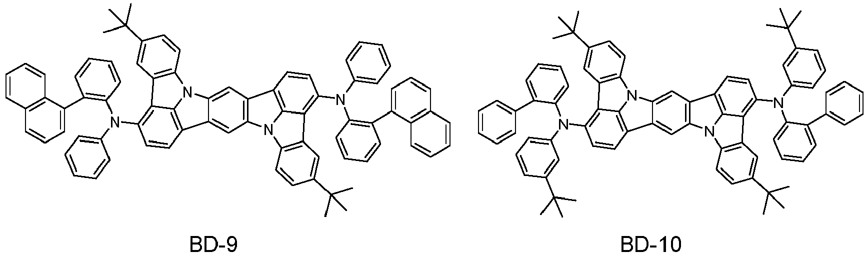

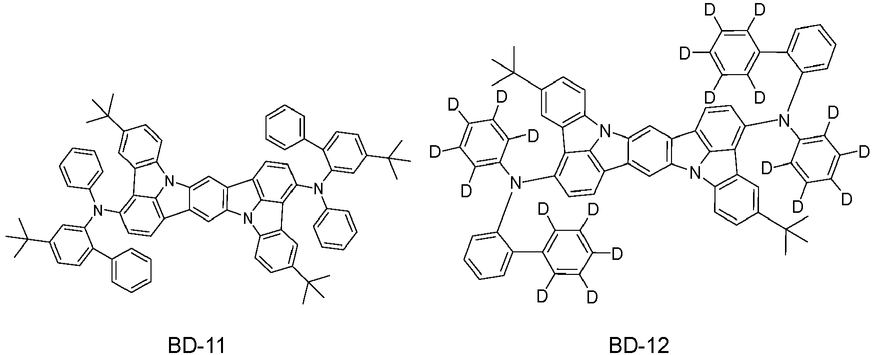

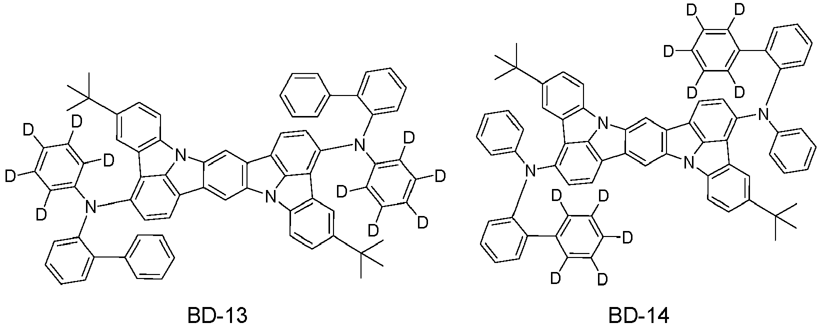

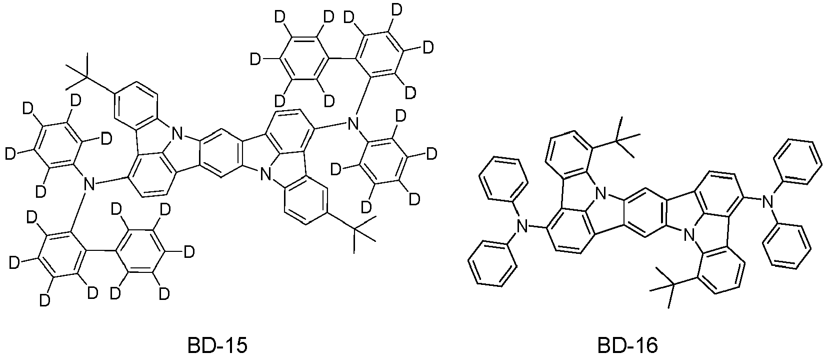

Compounds used in Comparative Examples are shown below.

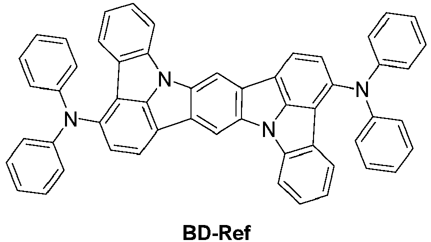

Other compounds used in Examples and Comparative Examples are shown below.

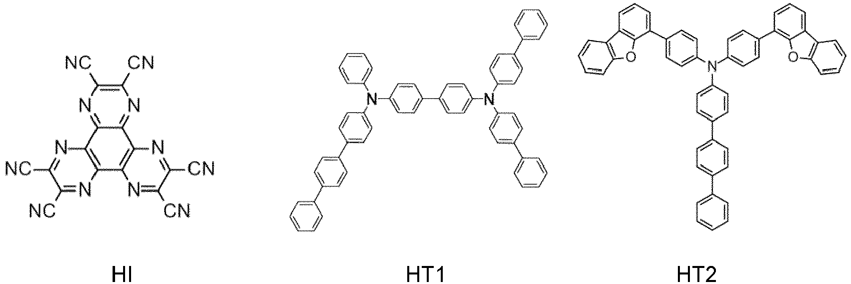

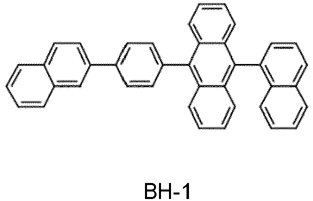

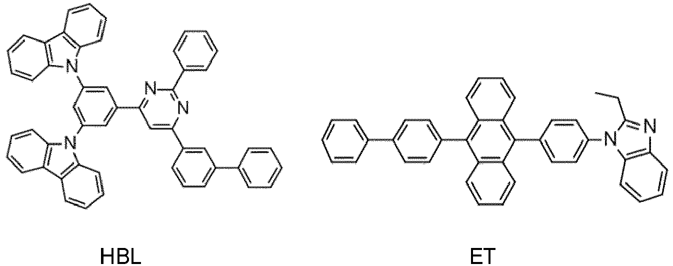

### Example 1

### <Fabrication of an organic EL device>

A 25 mm × 75 mm × 1.1 mm-thick glass substrate with an ITO transparent electrode (anode) (manufactured by GEOMATEC Co., Ltd.) was subjected to ultrasonic cleaning in isopropyl alcohol for 5 minutes, and then subjected to UV-ozone cleaning for 30 minutes. The thickness of the ITO film was 130 nm.

The glass substrate with the transparent electrode after being cleaned was mounted onto a substrate holder in a vacuum vapor deposition apparatus. First, Compound HI was deposited on a surface on the side on which the transparent electrode was formed so as to cover the transparent electrode to form a compound HI film having a thickness of 5 nm. This HI film functions as a hole-injecting layer.

Subsequent to the formation of the HI film, Compound HT1 was deposited thereon to form an HT1 film having a thickness of 80 nm on the HI film. The HT1 film functions as a first hole-transporting layer.

Subsequent to the formation of the HT1 film, Compound HT2 was deposited thereon to form an HT2 film having a thickness of 10 nm on the HT1 film. The HT2 film functions as a second hole-transporting layer.

BH-1 (host material) and BD-1 (dopant material) were co-deposited on the HT2 film to be 2% in a proportion (mass ratio) of the compound BD-1 to form an emitting layer having a thickness of 25 nm.

Compound HBL was deposited on the emitting layer to form an electron-transporting layer having a thickness of 10 nm. Compound ET as an electron-injecting material was deposited on the electron-transporting layer to form an electron-injecting layer having a thickness of 15 nm. LiF was deposited on the electron-injecting layer to form a LiF film having a thickness of 1 nm. Metal Al was deposited on the LiF film to form a metal cathode having a thickness of 80 nm.

The device configuration of the organic EL device of Example 1 is schematically shown as follows.

### ITO(130)/HI1(5)/HT1(80)/HT2(10)/BH-1:BD-1(25:2%)/HBL(10)/ET(15)/LiF(1)/Al(80)

The numerical values in parentheses indicate the film thickness (unit: nm).

### <Evaluation of organic EL device>

Voltage was applied to the obtained organic EL device so that the current density became 50 mA/cm², and the time until the luminance became 95% of the initial luminance (LT95) was measured. The results are shown in Table 1. The numerical values in the Table are relative values with LT95 of Comparative Example 1 described later being 100%.

### Examples 2 to 15 and Comparative Example 1

Organic EL devices were fabricated and evaluated by the same manner as in Example 1, except that compounds described in Table 1 were used as a dopant material of the emitting layer. The results are shown in Table 1.

**Table 1**

| | BD | LT95 Relative value (%) |
|---|---|---|
| Example 1 | BD-1 | 111 |
| Example 2 | BD-3 | 152 |
| Example 3 | BD-4 | 122 |
| Example 4 | BD-5 | 148 |
| Example 5 | BD-6 | 185 |
| Example 6 | BD-7 | 240 |
| Example 7 | BD-8 | 183 |
| Example 8 | BD-9 | 177 |
| Example 9 | BD-10 | 158 |
| Example 10 | BD-11 | 152 |
| Example 11 | BD-12 | 247 |
| Example 12 | BD-13 | 239 |
| Example 13 | BD-14 | 237 |
| Example 14 | BD-15 | 255 |
| Example 15 | BD-16 | 110 |
| Comp. Ex. 1 | BD-Ref | 100 |

### <Synthesis of compounds>

Compounds BD-1 to 16 and BD-Ref were synthesized by the following general synthetic route.

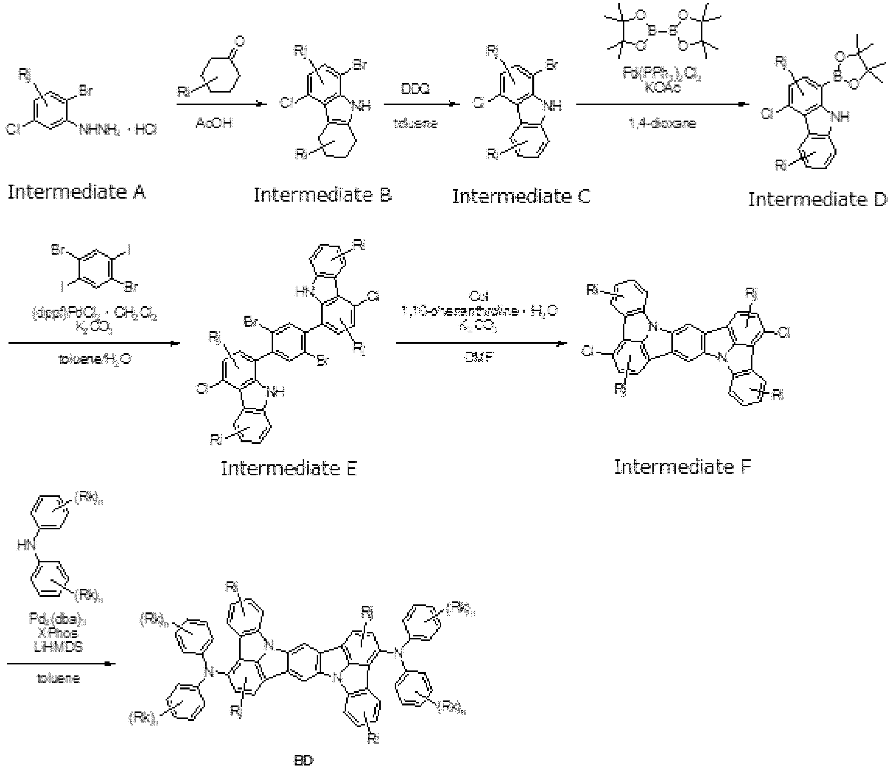

### (1) Synthesis of BD-1

BD-1 was synthesized according to the synthetic route described below.

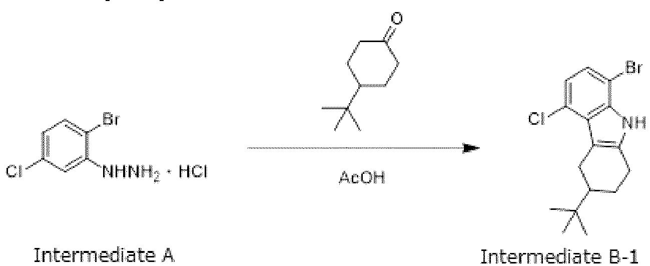

Intermediate A (9.00 g), 4-t-butylcyclohexane-1-one (5.38 g) was added to acetic acid (35 mL) and heated at 100°C for 6 hours with stirring under an argon atmosphere. Dichloromethane and water were added to the reaction solution, and the organic phase was separated and washed with an aqueous solution of sodium hydrogen carbonate. After concentration under reduced pressure, the washed solution was purified by column chromatography to obtain Intermediate B-1 (5.94 g, 50% yield)

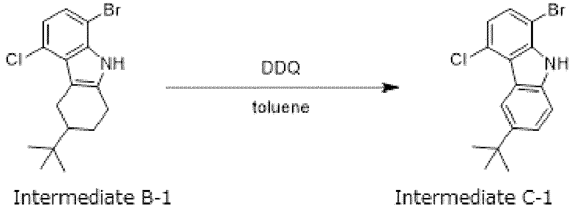

Intermediate B-1 (6.32 g), 2,3-dichloro-5,6-dicyano-p-benzoquinone (8.42 g) was added to toluene (90 mL) and heated at 100°C for 3 hours with stirring under an argon atmosphere. The reaction solution was filtered through Celite and then purified by column chromatography to obtain Intermediate C-1 (5.50 g, 88% yield).

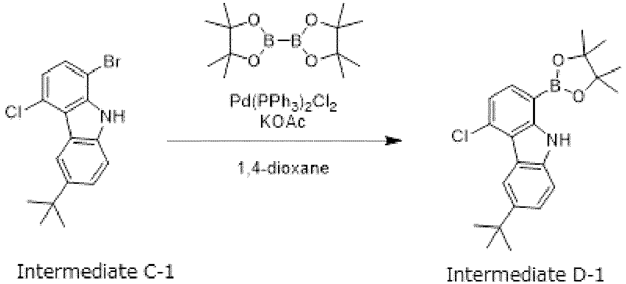

Intermediate C-1 (5.80 g), bis(pinacolato)diboron (13.12 g), [1,1'-bis(diphenylphosphino)ferrocene]dichloropalladium(II) (0.25 g), potassium acetate (3.38 g) were added to 1,4-dioxane (120 mL) and heated at 100°C for 4 hours with stirring under an argon atmosphere. The reaction solution was filtered through Celite and then purified by column chromatography and recrystallization to obtain Intermediate D-1 (3.65 g, 55% yield).

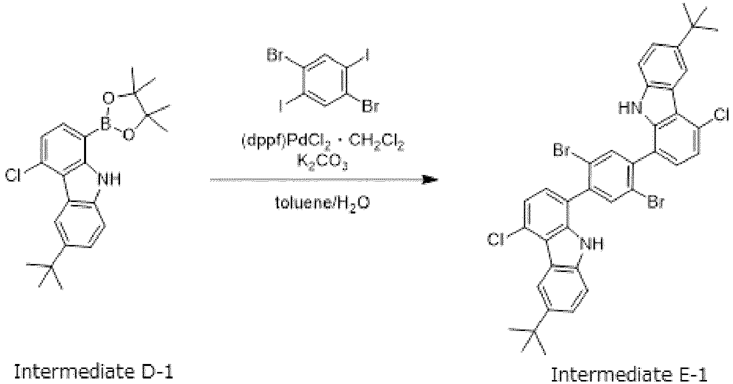

Intermediate D-1 (8.37 g), 1,4-dibromo-2,5-diiodobenzene (4.30 g), [1,1'-bis(diphenylphosphino)ferrocene]dichloropalladium(II) dichloromethane complex (0.29 g), potassium carbonate (3.66 g) were added to a mixed solvent of toluene (129 mL) and water (65 mL) and heated at 90°C for 7 hours with stirring under an argon atmosphere. To the reaction solution, toluene and water were added and stirred at room temperature. The organic phase was separated and subjected to column chromatography followed by washing with toluene to obtain Intermediate E-1 (4.42 g, 67% yield).

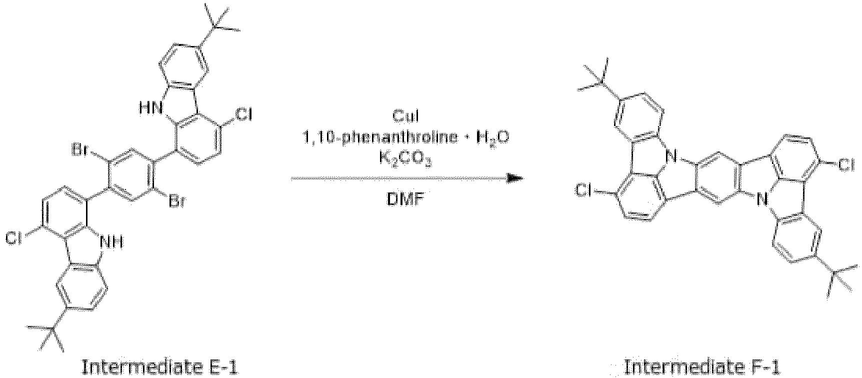

Intermediate E-1 (5.04 g), copper(I) iodide (0.13 g), 1,10-phenanthroline monohydrate (0.24 g), and potassium carbonate (2.33 g) were added to dimethylformamide (135 mL) and heated at 100°C for 3 hours with stirring under an argon atmosphere. Water (150 mL) was added to the reaction solution, and the solid was collected by filtration, followed by purification by column chromatography to obtain Intermediate F-1 (3.54 g, yield 90%).

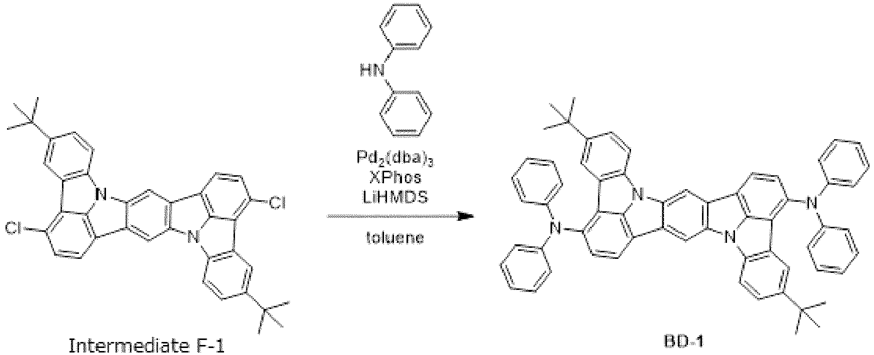

To a suspension of intermediate F-1 (0.71 g), diphenylamine (0.43 g), tris(dibenzylideneacetone)dipalladium(0) (0.06 g), and 2-dicyclohexylphosphino-2',4',6'-triisopropylbiphenyl (XPhos) (0.23 g) in toluene (60 mL), a toluene solution of lithium hexamethyldisilazide (LiHMDS) (1 M, 2.8 mL) was added dropwise, and the mixture was heated at 100°C for 3 hours with stirring under an argon atmosphere. After cooling to room temperature, the reaction solution was subjected to column chromatography. The resulting crude product was purified by column chromatography and recrystallization to obtain BD-1 (0.72 g, 70% yield). The molecular weight of BD-1 was 851.11, and the mass spectrum of the resulting compound was analyzed as m/z (ratio of mass to charge) = 851, thereby identified as Compound BD-1.

### (2) Synthesis of Compound BD-2

Synthesis was performed in the same manner as in the synthesis of Compound BD-1, except that 4,4'-dimethyl-diphenylamine was used instead of diphenylamine as a reaction raw material. The molecular weight of BD-2 was 907.22, and the mass spectrum of the resulting compound was analyzed as m/z (ratio of mass to charge) = 907, thereby identified as Compound BD-2.

### (3) Synthesis of Compound BD-3

Synthesis was performed in the same manner as in the synthesis of Compound BD-1, except that 4,4'-diethyl-diphenylamine was used instead of diphenylamine as a reaction raw material. The molecular weight of BD-3 was 963.33, and the mass spectrum of the resulting compound was analyzed as m/z (ratio of mass to charge) = 963, thereby identified as Compound BD-3.

### (4) Synthesis of Compound BD-4

Synthesis was performed in the same manner as in the synthesis of Compound BD-1, except that 4-isopropyl-diphenylamine was used instead of diphenylamine as a reaction raw material. The molecular weight of BD-4 was 935.27, and the mass spectrum of the resulting compound was analyzed as m/z (ratio of mass to charge) = 935, thereby identified as Compound BD-4.

### (5) Synthesis of Compound BD-5

Synthesis was performed in the same manner as in the synthesis of Compound BD-1, except that 3,3'-dimethyl-diphenylamine was used instead of diphenylamine as a reaction raw material. The molecular weight of BD-5 was 907.22, and the mass spectrum of the resulting compound was analyzed as m/z (ratio of mass to charge) = 907, thereby identified as Compound BD-5.

### (6) Synthesis of Compound BD-6

Synthesis was performed in the same manner as in the synthesis of Compound BD-1, except that 3-t-butyl-diphenylamine was used instead of diphenylamine as a reaction raw material. The molecular weight of BD-6 was 963.33, and the mass spectrum of the resulting compound was analyzed as m/z (ratio of mass to charge) = 963, thereby identified as Compound BD-6.

### (7) Synthesis of Compound BD-Ref

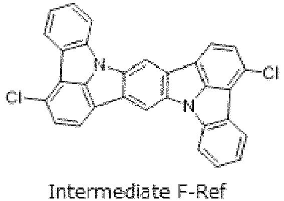

Intermediate F-Ref was synthesized in the same manner as in the synthesis of Intermediates B-1 to F-1, except that cyclohexanone was used instead of 4-t-butylcyclohexan-1-one as the reaction raw material.

Synthesis was performed in the same manner as in the synthesis of Compound BD-1, except that Intermediate F-Ref was used instead of Intermediate F-1 as a reaction raw material. The molecular weight of BD-Ref was 738.89, and the mass spectrum of the resulting compound was analyzed as m/z (ratio of mass to charge) = 738, thereby identified as Compound BD-Ref.

### (8) Synthesis of Compound BD-7

Synthesis was performed in the same manner as in the synthesis of Compound BD-1, except that N-(4-t-butylphenyl)-[1,1'-biphenyl]-2-amine was used instead of diphenylamine as a reaction raw material. The molecular weight of BD-7 was 1115.52, and the mass spectrum of the resulting compound was analyzed as m/z (ratio of mass to charge) = 1115, thereby identified as Compound BD-7.

### (9) Synthesis of Compound BD-8

Synthesis was performed in the same manner as in the synthesis of Compound BD-1, except that N-phenyl-[1,1'-biphenyl]-2-amine was used instead of diphenylamine as a reaction raw material. The molecular weight of BD-8 was 1003.31, and the mass spectrum of the resulting compound was analyzed as m/z (ratio of mass to charge) = 1003, thereby identified as Compound BD-8.

### (10) Synthesis of Compound BD-9

Synthesis was performed in the same manner as in the synthesis of Compound BD-1, except that 2-(1-naphthalenyl)-diphenylamine was used instead of diphenylamine as a reaction raw material. The molecular weight of BD-9 was 1103.43, and the mass spectrum of the resulting compound was analyzed as m/z (ratio of mass to charge) = 1103, thereby identified as Compound BD-9.

### (11) Synthesis of Compound BD-10

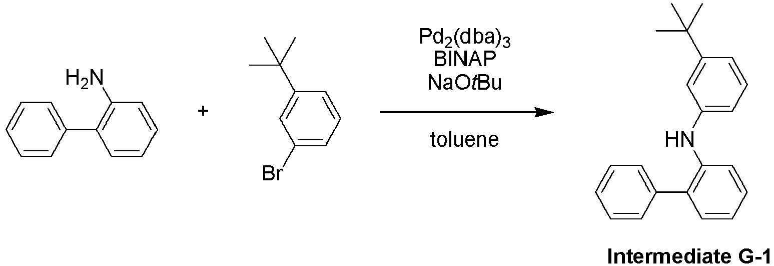

[1,1'-Biphenyl]-2-amine (21.4 g), 1-bromo-3-t-butylbenzene (27.0 g), tris(dibenzylideneacetone)dipaladium(0) (1.74 g), 2,2'-bis(diphenylphosphino)-1,1'-binaphthyl (BINAP) (2.37 g), sodium t-butoxide (17.1 g) were added to toluene (253 ml) and heated at 100°C for 3 hours with stirring under an argon atmosphere. The reaction solution was filtered through Celite and then purified by column chromatography to obtain Intermediate G-1 (28.6 g, 75% yield).

Synthesis was performed in the same manner as in the synthesis of Compound BD-1, except that Intermediate G-1 was used instead of diphenylamine as a reaction raw material. The molecular weight of BD-10 was 1115.52, and the mass spectrum of the resulting compound was analyzed as m/z (ratio of mass to charge) = 1115, thereby identified as Compound BD-10.

### (12) Synthesis of Compound BD-11

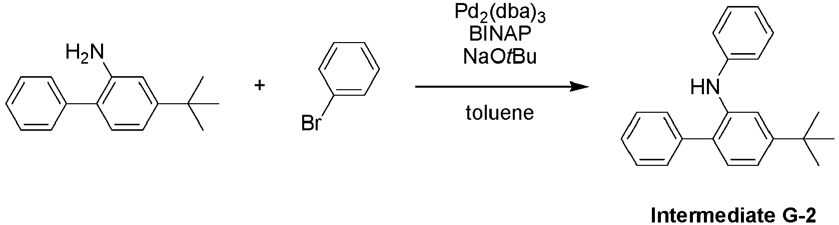

Intermediate G-2 was synthesized in the same manner as in the synthesis of Intermediate G-1, except that 4-t-butyl-[1,1'-biphenyl]-2-amine was used instead of [1,1'-biphenyl]-2-amine and bromobenzene was used instead of 1-bromo-3-t-butylbenzene, as a reaction raw material.

Synthesis was performed in the same manner as in the synthesis of Compound BD-1, except that Intermediate G-2 was used instead of diphenylamine as a reaction raw material. The molecular weight of BD-11 was 1115.52, and the mass spectrum of the resulting compound was analyzed as m/z (ratio of mass to charge) = 1115, thereby identified as Compound BD-11.

### (13) Synthesis of Compound BD-12

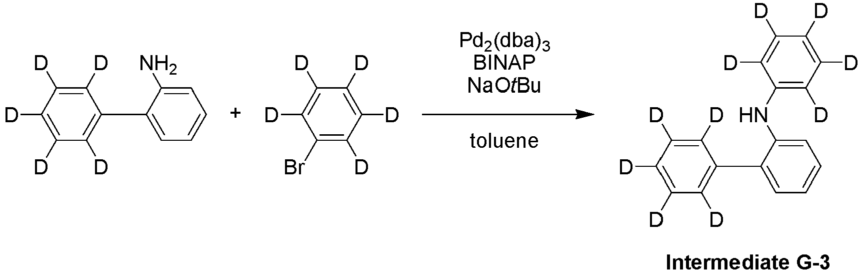

Intermediate G-3 was synthesized in the same manner as Intermediate G-1, except that [1,1'-biphenyl-2',3',4',5',6'-d5]-2-amine was used instead of [1,1'-biphenyl]-2-amine, and bromobenzene-d5 was used instead of 1-bromo-3-t-butylbenzene, as the raw reaction material.

Synthesis was performed in the same manner as in the synthesis of a compound BD-1, except that Intermediate G-3 was used instead of diphenylamine as a reaction raw material. The molecular weight of BD-12 was 1023.43, and the mass spectrum of the resulting compound was analyzed as m/z (ratio of mass to charge) = 1023, thereby identified as Compound BD-12.

### (14) Synthesis of Compound BD-13

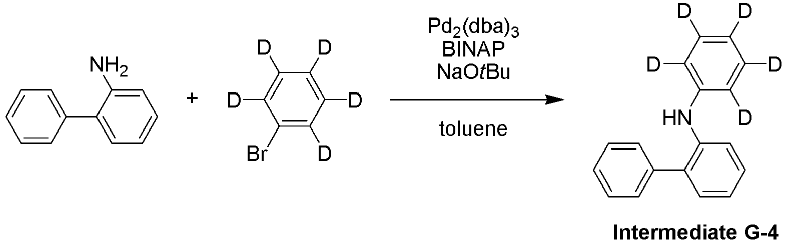

Intermediate G-4 was synthesized in the same manner as in the synthesis of Intermediate G-1, except that bromobenzene-d5 was used instead of 1-bromo-3-t-butylbenzene as the reaction raw material.

Synthesis was performed in the same manner as in the synthesis of Compound BD-1, except that Intermediate G-4 was used instead of diphenylamine as a reaction raw material. The molecular weight of BD-13 was 1013.37, and the mass spectrum of the resulting compound was analyzed as m/z (ratio of mass to charge) = 1013, thereby identified as Compound BD-13.

### (15) Synthesis of Compound BD-14

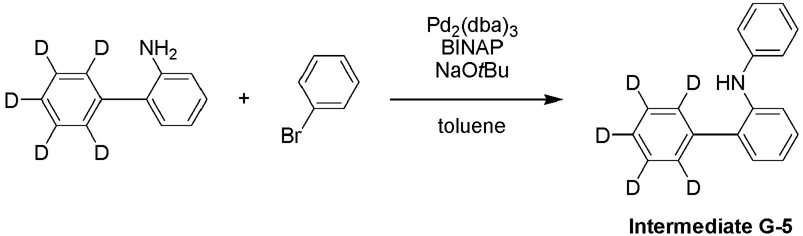

Intermediate G-5 was synthesized in the same manner as Intermediate G-1, except that [1,1'-biphenyl-2',3',4',5',6'-d5]-2-amine was used instead of [1,1'-biphenyl]-2-amine, and bromobenzene was used instead of 1-bromo-3-t-butylbenzene, as the raw reaction material.

Synthesis was performed in the same manner as in the synthesis of Compound BD-1, except that Intermediate G-5 was used instead of diphenylamine as a reaction raw material. The molecular weight of BD-14 was 1013.37, and the mass spectrum of the resulting compound was analyzed as m/z (ratio of mass to charge) = 1013, thereby identified as Compound BD-14.

### (16) Synthesis of Compound BD-15

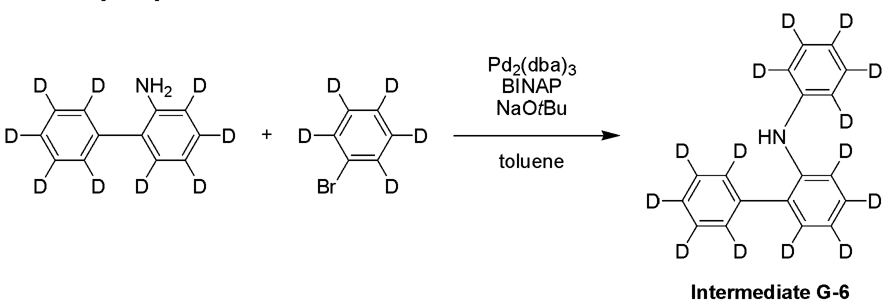

Intermediate G-6 was synthesized in the same manner as in the synthesis of Intermediate G-1, except that [1,1'-biphenyl-2',3,3',4,4',5,5',6,6'-d9]-2-amine was used instead of [1,1'-biphenyl]-2-amine and bromobenzene-d5 was used instead of 1-bromo-3-t-butylbenzene, as a reaction raw material.

Synthesis was performed in the same manner as in the synthesis of Compound BD-1, except that Intermediate G-6 was used instead of diphenylamine as a reaction raw material. The molecular weight of BD-15 was 1031.48, and the mass spectrum of the resulting compound was analyzed as m/z (ratio of mass to charge) = 1031, thereby identified as Compound BD-15.

### (17) Synthesis of Compound BD-16

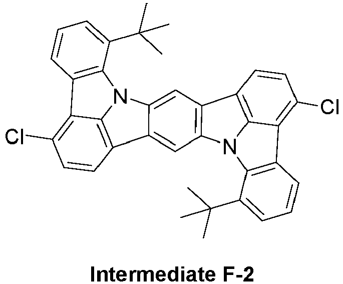

Intermediate F-2 was synthesized in the same manner as in the synthesis of Intermediates B-1 to F-1, except that 2-t-butylcyclohexan-1-one was used instead of 4-t-butylcyclohexan-1-one as the reaction raw material.

Synthesis was performed in the same manner as in the synthesis of Compound BD-1, except that Intermediate F-2 was used instead of Intermediate F-1 as a reaction raw material. The molecular weight of BD-16 was 851.11, and the mass spectrum of the resulting compound was analyzed as m/z (ratio of mass to charge) = 851, thereby identified as Compound BD-16.

## Claims

1. A compound represented by formula (9-2): 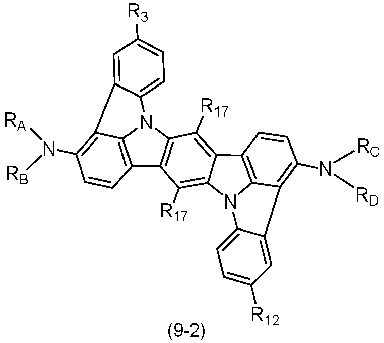 wherein in the formula (9-2),
R₃, and R₁₂ are independently a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), -C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms;
R₁₇ is a hydrogen atom, a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), -C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms; two R₁₇s may be the same as or different from each other; and
R_{A}, R_{B}, R_{C} and R_{D} are independently a substituted or unsubstituted alkyl group including 1 to 50 carbon atoms, a substituted or unsubstituted aryl group including 6 to 50 ring carbon atoms, or a substituted or unsubstituted monovalent heterocyclic group including 5 to 50 ring atoms.

2. A compound represented by formula (9-3): 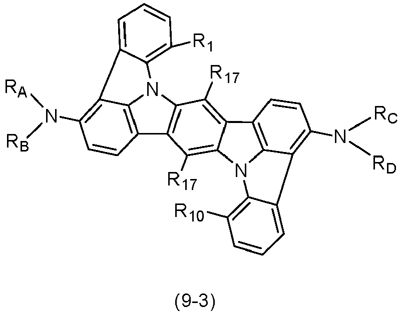 wherein in the formula (9-3),
R₁, and R₁₀ are independently a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), -C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms;
R₁₇ is a hydrogen atom, a substituted or unsubstituted alkyl group including 1 to 50 carbon atoms, a substituted or unsubstituted haloalkyl group including 1 to 50 carbon atoms, a substituted or unsubstituted alkenyl group including 2 to 50 carbon atoms, a substituted or unsubstituted alkynyl group including 2 to 50 carbon atoms, a substituted or unsubstituted cycloalkyl group including 3 to 50 ring carbon atoms, a substituted or unsubstituted alkoxy group including 1 to 50 carbon atoms, a substituted or unsubstituted alkylthio group including 1 to 50 carbon atoms, a substituted or unsubstituted aryloxy group including 6 to 50 ring carbon atoms, a substituted or unsubstituted arylthio group including 6 to 50 ring carbon atoms, a substituted or unsubstituted aralkyl group including 7 to 50 carbon atoms, -Si(R₃₁)(R₃₂)(R₃₃), -C(=O)R₃₄, -COOR₃₅, -N(R₃₆)(R₃₇), a halogen atom, a cyano group, a nitro group, a substituted or unsubstituted aryl group including 6 to 50 ring carbon atoms, or a substituted or unsubstituted monovalent heterocyclic group including 5 to 50 ring atoms; two R₁₇s may be the same as or different from each other; and
R_{A}, R_{B}, R_{C} and R_{D} are independently a substituted or unsubstituted alkyl group including 1 to 50 carbon atoms, a substituted or unsubstituted aryl group including 6 to 50 ring carbon atoms, or a substituted or unsubstituted monovalent heterocyclic group including 5 to 50 ring atoms.

3. The compound according to any one of claims 1 or 2, wherein R_{A}, R_{B}, R_{C} and R_{D} are independently a substituted or unsubstituted aryl group including 6 to 50 ring carbon atoms, or a substituted or unsubstituted monovalent heterocyclic group including 5 to 50 ring atoms.

4. The compound according to any one of claims 1 to 3, wherein R_{A}, R_{B}, Rc, and R_{D} are independently a substituted or unsubstituted phenyl group.

5. The compound according to any one of claims 1 to 4, wherein R_{A}, R_{B}, Rc, and R_{D} are independently a phenyl group substituted with an alkyl group including 1 to 5 carbon atoms, or an unsubstituted phenyl group.

6. The compound according to any one of claims 1 to 5, wherein R₁₇s are hydrogen atoms.

7. The compound according to any one of claims 1 to 6, wherein the substituent in the case of "substituted or unsubstituted" is selected from the group consisting of an alkyl group including 1 to 50 carbon atoms, a haloalkyl group including 1 to 50 carbon atoms, an alkenyl group including 2 to 50 carbon atoms, an alkynyl group including 2 to 50 carbon atoms, a cycloalkyl group including 3 to 50 ring carbon atoms, an alkoxy group including 1 to 50 carbon atoms, an alkylthio group including 1 to 50 carbon atoms, an aryloxy group including 6 to 50 ring carbon atoms, and an arylthio group including 6 to 50 ring carbon atoms, an aralkyl group including 7 to 50 carbon atoms, -Si(R₄₁)(R₄₂)(R₄₃), -C(=O)R₄₄, -COOR₄₅, -S(=O)₂R₄₆, -P(=O)(R₄₇)(R₄₈), -Ge(R₄₉)(R₅₀)(R₅₁), -N(R₅₂)(R₅₃), a hydroxy group, a halogen atom, a cyano group, a nitro group, an aryl group including 6 to 50 ring carbon atoms, and monovalent heterocyclic group including 5 to 50 ring atoms; and R₄₁ to R₅₃ are independently a hydrogen atom, an alkyl group including 1 to 50 carbon atoms, an aryl group including 6 to 50 ring carbon atoms, or a monovalent heterocyclic group including 5 to 50 ring atoms; when two or more of each of R₄₁ to R₅₃ are present, the two or more of each of R₄₁ to R₅₃ may be the same as or different from each other.

8. The compound according to any one of claims 1 to 7, wherein the substituent in the case of "substituted or unsubstituted" is a group selected from the group consisting of:
an alkyl group including 1 to 18 carbon atoms, and
an aryl group including 6 to 18 ring carbon atoms.

9. A material for an organic electroluminescence device, comprising a compound according to any one of claims 1 to 8.

10. An organic electroluminescence device comprising:
a cathode;
an anode; and
at least one organic layer disposed between the cathode and the anode, wherein
at least one layer of the at least one organic layer comprises the compound according to any one of claims 1 to 8.

11. The organic electroluminescence device according to claim 10, wherein the at least one layer of the at least one organic layer comprises a second compound which is not the same as the compound according to any one of claims 1 to 8.

12. The organic electroluminescence device according to claim 11, wherein the second compound is a compound represented by the following formula (10): 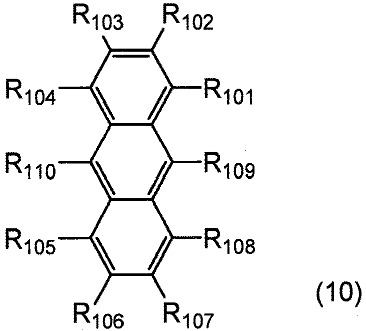 wherein in the formula (10),
one or more sets of adjacent two or more of R₁₀₁ to R₁₁₀ form a substituted or unsubstituted, saturated or unsaturated ring, or do not form the substituted or unsubstituted, saturated or unsaturated ring;
R₁₀₁ to R₁₁₀ which do not form the substituted or unsubstituted, saturated or unsaturated ring are independently
a hydrogen atom,
a substituent R, or
a group represented by the following formula (11):
-L₁₀₁-Ar₁₀₁ (11)
wherein in the formula (11),
L₁₀₁ is
a single bond,
a substituted or unsubstituted arylene group including 6 to 50 ring carbon atoms, or
a substituted or unsubstituted divalent heterocyclic group including 5 to 50 ring atoms;
Ar₁₀₁ is
a substituted or unsubstituted aryl group including 6 to 50 ring carbon atoms, or
a substituted or unsubstituted monovalent heterocyclic group including 5 to 50 ring atoms;
the substituent R is
a substituted or unsubstituted alkyl group including 1 to 50 carbon atoms,
a substituted or unsubstituted alkenyl group including 2 to 50 carbon atoms,
a substituted or unsubstituted alkynyl group including 2 to 50 carbon atoms,
a substituted or unsubstituted cycloalkyl group including 3 to 50 ring carbon atoms,
-Si(R₉₀₁)(R₉₀₂)(R₉₀₃),
-O-(R₉₀₄),
-S-(R₉₀₅),
-N(R₉₀₆)(R₉₀₇),
a halogen atom, a cyano group, a nitro group,
a substituted or unsubstituted aryl group including 6 to 50 ring carbon atoms, or
a substituted or unsubstituted monovalent heterocyclic group including 5 to 50 ring atoms;
when two or more substituents R are present, the two or more substituents R may be the same as or different to each other;
R₉₀₁ to R₉₀₇ are independently
a hydrogen atom,
a substituted or unsubstituted alkyl group including 1 to 50 carbon atoms,
a substituted or unsubstituted cycloalkyl group including 3 to 50 ring carbon atoms,
a substituted or unsubstituted aryl group including 6 to 50 ring carbon atoms, or
a substituted or unsubstituted monovalent heterocyclic group including 5 to 50 ring atoms;
when two or more of each of R₉₀₁ to R₉₀₇ are present, the two or more of each of R₉₀₁ to R₉₀₇ may be the same as or different from each other;
provided that at least one of R₁₀₁ to R₁₁₀ which does not form the substituted or unsubstituted, saturated or unsaturated ring is a group represented by the formula (11); and
when two or more of the formula (11) are present, the two or more groups represented by the formula (11) may be the same as or different from each other.

13. The organic electroluminescence device according to any one of claims 10 to 11, wherein at least one of the at least one organic layer is an emitting layer.

14. The organic electroluminescence device according to claim 13, wherein the emitting layer further comprises a host compound having delayed fluorescence.

## Patentansprüche

1. Verbindung, dargestellt durch Formel (9-2): 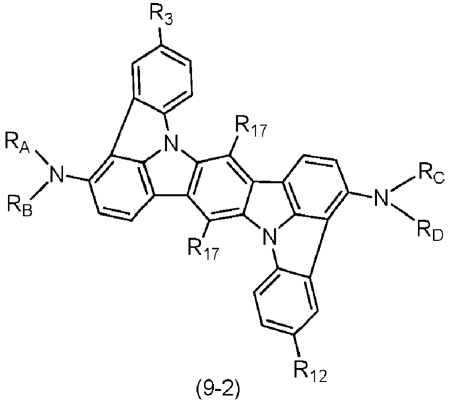 wobei in Formel (9-2)
R₃ und R₁₂ unabhängig eine substituierte oder unsubstituierte Alkylgruppe mit 1 bis 50 Kohlenstoffatomen, eine substituierte oder unsubstituierte Halogenalkylgruppe mit 1 bis 50 Kohlenstoffatomen, eine substituierte oder unsubstituierte Alkenylgruppe mit 2 bis 50 Kohlenstoffatomen, eine substituierte oder unsubstituierte Alkinylgruppe mit 2 bis 50 Kohlenstoffatomen, eine substituierte oder unsubstituierte Cycloalkylgruppe mit 3 bis 50 Ringkohlenstoffatomen, eine substituierte oder unsubstituierte Alkoxygruppe mit 1 bis 50 Kohlenstoffatomen, eine substituierte oder unsubstituierte Alkylthiogruppe mit 1 bis 50 Kohlenstoffatomen, eine substituierte oder unsubstituierte Aryloxygruppe mit 6 bis 50 Ringkohlenstoffatomen, eine substituierte oder unsubstituierte Arylthiogruppe mit 6 bis 50 Ringkohlenstoffatomen, eine substituierte oder unsubstituierte Aralkylgruppe mit 7 bis 50 Kohlenstoffatomen, -S(R₃₁)(R₃₂)(R₃₃), -C(=O)R₃₄, -COOR₃₅, -N(R₃₆)(R₃₇), ein Halogenatom, eine Cyanogruppe, eine Nitrogruppe, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen sind,
R₁₇ ein Wasserstoffatom, eine substituierte oder unsubstituierte Alkylgruppe mit 1 bis 50 Kohlenstoffatomen, eine substituierte oder unsubstituierte Halogenalkylgruppe mit 1 bis 50 Kohlenstoffatomen, eine substituierte oder unsubstituierte Alkenylgruppe mit 2 bis 50 Kohlenstoffatomen, eine substituierte oder unsubstituierte Alkinylgruppe mit 2 bis 50 Kohlenstoffatomen, eine substituierte oder unsubstituierte Cycloalkylgruppe mit 2 bis 50 Ringkohlenstoffatomen, eine substituierte oder unsubstituierte Alkoxygruppe mit 1 bis 50 Kohlenstoffatomen, eine substituierte oder unsubstituierte Alkylthiogruppe mit 1 bis 50 Kohlenstoffatomen, eine substituierte oder unsubstituierte Aryloxygruppe mit 6 bis 50 Ringkohlenstoffatomen, eine substituierte oder unsubstituierte Arylthiogruppe mit 6 bis 50 Kohlenstoffatomen, eine substituierte oder unsubstituierte Aralkylgruppe mit 7 bis 50 Kohlenstoffatomen, -S(R₃₁)(R₃₂)(R₃₃), -C(=O)R₃₄, -COOR₃₅, -N(R₃₆)(R₃₇), ein Halogenatom, eine Cyanogruppe, eine Nitrogruppe, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen ist, zwei R₁₇ gleich oder voneinander verschieden sein können und
R_{A}, R_{B}, R_{C} und R_{D} unabhängig eine substituierte oder unsubstituierte Alkylgruppe mit 1 bis 50 Kohlenstoffatomen, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen sind.

2. Verbindung, dargestellt durch Formel (9-3): 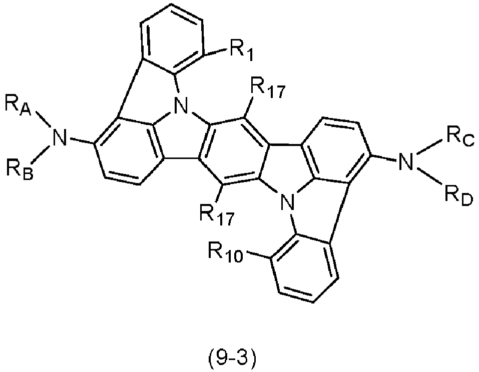 wobei in Formel (9-3),
R₁ und R₁₀ unabhängig eine substituierte oder unsubstituierte Alkylgruppe mit 1 bis 50 Kohlenstoffatomen, eine substituierte oder unsubstituierte Halogenalkylgruppe mit 1 bis 50 Kohlenstoffatomen, eine substituierte oder unsubstituierte Alkenylgruppe mit 2 bis 50 Kohlenstoffatomen, eine substituierte oder unsubstituierte Alkinylgruppe mit 2 bis 50 Kohlenstoffatomen, eine substituierte oder unsubstituierte Cycloalkylgruppe mit 3 bis 50 Ringkohlenstoffatomen, eine substituierte oder unsubstituierte Alkoxygruppe mit 1 bis 50 Kohlenstoffatomen, eine substituierte oder unsubstituierte Alkylthiogruppe mit 1 bis 50 Kohlenstoffatomen, eine substituierte oder unsubstituierte Aryloxygruppe mit 6 bis 50 Ringkohlenstoffatomen, eine substituierte oder unsubstituierte Arylthiogruppe mit 6 bis 50 Ringkohlenstoffatomen, eine substituierte oder unsubstituierte Aralkylgruppe mit 7 bis 50 Kohlenstoffatomen, -S(R₃₁)(R₃₂)(R₃₃), -C(=O)R₃₄, -COOR₃₅, -N(R₃₆)(R₃₇), ein Halogenatom, eine Cyanogruppe, eine Nitrogruppe, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen sind,
R₁₇ ein Wasserstoffatom, eine substituierte oder unsubstituierte Alkylgruppe mit 1 bis 50 Kohlenstoffatomen, eine substituierte oder unsubstituierte Halogenalkylgruppe mit 1 bis 50 Kohlenstoffatomen, eine substituierte oder unsubstituierte Alkenylgruppe mit 2 bis 50 Kohlenstoffatomen, eine substituierte oder unsubstituierte Alkinylgruppe mit 2 bis 50 Kohlenstoffatomen, eine substituierte oder unsubstituierte Cycloalkylgruppe mit 2 bis 50 Ringkohlenstoffatomen, eine substituierte oder unsubstituierte Alkoxygruppe mit 1 bis 50 Kohlenstoffatomen, eine substituierte oder unsubstituierte Alkylthiogruppe mit 1 bis 50 Kohlenstoffatomen, eine substituierte oder unsubstituierte Aryloxygruppe mit 6 bis 50 Ringkohlenstoffatomen, eine substituierte oder unsubstituierte Arylthiogruppe mit 6 bis 50 Kohlenstoffatomen, eine substituierte oder unsubstituierte Aralkylgruppe mit 7 bis 50 Kohlenstoffatomen, -S(R₃₁)(R₃₂)(R₃₃), -C(=O)R₃₄, -COOR₃₅, -N(R₃₆)(R₃₇), ein Halogenatom, eine Cyanogruppe, eine Nitrogruppe, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen ist, zwei R₁₇ gleich oder voneinander verschieden sein können und
R_{A}, R_{B}, R_{C} und R_{D} unabhängig eine substituierte oder unsubstituierte Alkylgruppe mit 1 bis 50 Kohlenstoffatomen, eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen sind.

3. Verbindung gemäß einem der Ansprüche 1 oder 2, wobei R_{A}, R_{B}, R_{C} und R_{D} unabhängig eine substituierte oder unsubstituierte Arylgruppe mit 6 bis 50 Ringkohlenstoffatomen oder eine substituierte oder unsubstituierte einwertige heterocyclische Gruppe mit 5 bis 50 Ringatomen sind.

4. Verbindung gemäß einem der Ansprüche 1 bis 3, wobei R_{A}, R_{B}, R_{C} und R_{D} unabhängig eine substituierte oder unsubstituierte Phenylgruppe sind.

5. Verbindung gemäß einem der Ansprüche 1 bis 4, wobei R_{A}, R_{B}, R_{C} und R_{D} unabhängig eine Phenylgruppe, die mit einer Alkylgruppe mit 1 bis 5 Kohlenstoffatomen substituiert ist, oder eine unsubstituierte Phenylgruppe sind.

6. Verbindung gemäß einem der Ansprüche 1 bis 5, wobei die R₁₇ Wasserstoffatome sind.

7. Verbindung gemäß einem der Ansprüche 1 bis 6, wobei der Substituent für den Fall "substituiert oder unsubstituiert" aus der Gruppe, bestehend aus einer Alkylgruppe mit 1 bis 50 Kohlenstoffatomen, einer Halogenalkylgruppe mit 1 bis 50 Kohlenstoffatomen, einer Alkenylgruppe mit 2 bis 50 Kohlenstoffatomen, einer Alkinylgruppe mit 2 bis 50 Kohlenstoffatomen, einer Cycloalkylgruppe mit 3 bis 50 Ringkohlenstoffatomen, einer Alkoxygruppe mit 1 bis 50 Kohlenstoffatomen, einer Alkylthiogruppe mit 1 bis 50 Kohlenstoffatomen, einer Aryloxygruppe mit 6 bis 50 Ringkohlenstoffatomen und einer Arylthiogruppe mit 6 bis 50 Ringkohlenstoffatomen, einer Aralkylgruppe mit 7 bis 50 Kohlenstoffatomen, -S(R₄₁)(R₄₂)(R₄₃), -C(=O)R₄₄, -COOR₄₅, -S(=O)₂R₄₆, -P(=O)(R₄₇)(R₄₈), -Ge(R₄₉)(R₅₀)(R₅₁), -N(R₅₂)(R₅₃), einer Hydroxygruppe, einem Halogenatom, einer Cyanogruppe, einer Nitrogruppe, einer Arylgruppe mit 6 bis 50 Ringkohlenstoffatomen und einer einwertigen heterocyclischen Gruppe mit 5 bis 50 Ringatomen, ausgewählt ist und R₄₁ bis R₅₃ unabhängig ein Wasserstoffatom, eine Alkylgruppe mit 1 bis 50 Kohlenstoffatomen, eine Arylgruppe mit 6 bis 50 Ringkohlenstoffatomen oder eine einwertige heterocyclische Gruppe mit 5 bis 50 Ringatomen sind, wenn zwei oder mehr von jedem von R₄₁ bis R₅₃ vorhanden sind, die zwei oder mehr von jedem von R₄₁ bis R₅₃ gleich oder voneinander verschieden sein können.

8. Verbindung gemäß einem der Ansprüche 1 bis 7, wobei der Substituent für den Fall "substituiert oder unsubstituiert" eine Gruppe, ausgewählt aus der Gruppe, bestehend aus
einer Alkylgruppe mit 1 bis 18 Kohlenstoffatomen und einer Arylgruppe mit 6 bis 18 Ringkohlenstoffatomen, ist.

9. Material für eine organische Elektrolumineszenzvorrichtung, umfassend eine Verbindung gemäß einem der Ansprüche 1 bis 8.

10. Organische Elektrolumineszenzvorrichtung, umfassend:
eine Kathode,
eine Anode und
mindestens eine organische Schicht, die zwischen der Kathode und der Anode angeordnet ist, wobei
mindestens eine Schicht von der mindestens einen organischen Schicht die Verbindung gemäß einem der Ansprüche 1 bis 8 umfasst.

11. Organische Elektrolumineszenzvorrichtung gemäß Anspruch 10, wobei die mindestens eine Schicht von der mindestens einen organischen Schicht eine zweite Verbindung umfasst, die nicht dieselbe wie die Verbindung gemäß einem der Ansprüche 1 bis 8 ist.

12. Organische Elektrolumineszenzvorrichtung gemäß Anspruch 11, wobei die zweite Verbindung eine durch die folgende Formel (10) dargestellte Verbindung ist: 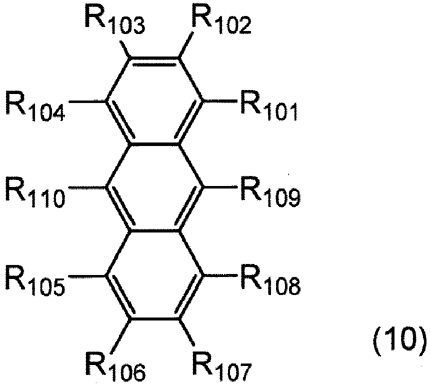 wobei in der Formel (10)
ein oder mehrere Sätze von benachbarten zwei oder mehr von R₁₀₁ bis R₁₁₀ einen substituierten oder unsubstituierten, gesättigten oder ungesättigten Ring bilden oder keinen substituierten oder unsubstituierten, gesättigten oder ungesättigten Ring bilden,
R₁₀₁ bis R₁₁₀, die keinen substituierten oder unsubstituierten, gesättigten oder ungesättigten Ring bilden, unabhängig
ein Wasserstoffatom,
ein Substituent R oder
eine durch die folgende Formel (11) dargestellte Gruppe sind:
-L₁₀₁-Ar₁₀₁ (11)
wobei in der Formel (11)
L₁₀₁
eine Einfachbindung,
eine substituierte oder unsubstituierte Arylengruppe mit 6 bis 50 Ringkohlenstoffatomen oder
eine substituierte oder unsubstituierte zweiwertige heterocyclische Gruppe mit 5 bis 50 Ringatomen ist,
Ar₁₀₁
eine substituierte oder unsubstituierte Arylgruppe mit 6 bis 50 Ringkohlenstoffatomen oder
eine substituierte oder unsubstituierte einwertige heterocyclische Gruppe mit 5 bis 50 Ringatomen ist,
der Substituent R
eine substituierte oder unsubstituierte Alkylgruppe mit 1 bis 50 Kohlenstoffatomen,
eine substituierte oder unsubstituierte Alkenylgruppe mit 2 bis 50 Kohlenstoffatomen,
eine substituierte oder unsubstituierte Alkinylgruppe mit 2 bis 50 Kohlenstoffatomen,
eine substituierte oder unsubstituierte Cycloalkylgruppe mit 3 bis 50 Ringkohlenstoffatomen,
-Si(R₉₀₁)(R₉₀₂)(R₉₀₃),
-O-(R₉₀₄),
-S-(R₉₀₅),
-N(R₉₀₆)(R₉₀₇),
ein Halogenatom, eine Cyanogruppe, eine Nitrogruppe,
eine substituierte oder unsubstituierte Arylgruppe mit 6 bis 50 Kohlenstoffatomen oder
eine substituierte oder unsubstituierte einwertige heterocyclische Gruppe mit 5 bis 50 Ringatomen ist,
wenn zwei oder mehr Substituenten R vorhanden sind, die zwei oder mehr Substituenten R gleich oder voneinander verschieden sein können,
R₉₀₁ bis R₉₀₇ unabhängig
ein Wasserstoffatom,
eine substituierte oder unsubstituierte Alkylgruppe mit 1 bis 50 Kohlenstoffatomen,
eine substituierte oder unsubstituierte Cycloalkylgruppe mit 3 bis 50 Ringkohlenstoffatomen,
eine substituierte oder unsubstituierte Arylgruppe mit 6 bis 50 Ringkohlenstoffatomen oder
eine substituierte oder unsubstituierte einwertige heterocyclische Gruppe mit 5 bis 50 Ringatomen sind,
wenn zwei oder mehr von jedem von R₉₀₁ bis R₉₀₇ vorhanden sind, die zwei oder mehr von jedem R₉₀₁ bis R₉₀₇ gleich oder voneinander verschieden sein können,
vorausgesetzt, dass mindestens eines von R₁₀₁ bis R₁₁₀, das keinen substituierten oder unsubstituierten, gesättigten oder ungesättigten Ring bildet, eine durch die Formel (11) dargestellte Gruppe ist, und
wenn zwei oder mehr von der Formel (11) vorhanden sind, die zwei oder mehr durch die Formel (11) dargestellten Gruppen gleich oder voneinander verschieden sein können.

13. Organische Elektrolumineszenzvorrichtung gemäß einem der Ansprüche 10 bis 11, wobei mindestens eine von der mindestens einen organischen Schicht eine emittierende Schicht ist.

14. Organische Elektrolumineszenzvorrichtung gemäß Anspruch 13, wobei die emittierende Schicht ferner eine Wirtsverbindung mit verzögerter Fluoreszenz umfasst.

## Revendications

1. Composé représenté par la formule (9-2) : 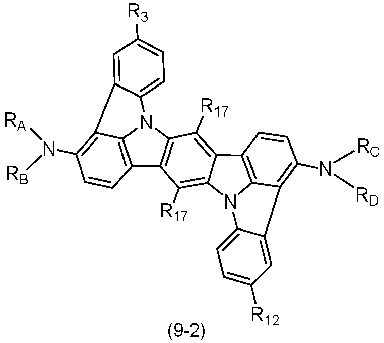 dans lequel, dans la formule (9-2),
R₃, et R₁₂ sont indépendamment un groupe alkyle substitué ou non substitué incluant 1 à 50 atomes de carbone, un groupe haloalkyle substitué ou non substitué incluant 1 à 50 atomes de carbone, un groupe alcényle substitué ou non substitué incluant 2 à 50 atomes de carbone, un groupe alcynyle substitué ou non substitué incluant 2 à 50 atomes de carbone, un groupe cycloalkyle substitué ou non substitué incluant 3 à 50 atomes de carbone de cycle, un groupe alcoxy substitué ou non substitué incluant 1 à 50 atomes de carbone, un groupe alkylthio substitué ou non substitué incluant 1 à 50 atomes de carbone, un groupe aryloxy substitué ou non substitué incluant 6 à 50 atomes de carbone de cycle, un groupe arylthio substitué ou non substitué incluant 6 à 50 atomes de carbone de cycle, un groupe aralkyle substitué ou non substitué incluant 7 à 50 atomes de carbone, -Si(R₃₁)(R₃₂)(R₃₃), - C(=O)R₃₄, -COOR₃₅, -N(R₃₆)(R₃₇), un atome d'halogène, un groupe cyano, un groupe nitro, un groupe aryle substitué ou non substitué incluant 6 à 50 atomes de carbone de cycle, ou un groupe hétérocyclique monovalent substitué ou non substitué incluant 5 à 50 atomes de carbone de cycle ;
R₁₇ est un atome d'hydrogène, un groupe alkyle substitué ou non substitué incluant 1 à 50 atomes de carbone, un groupe haloalkyle substitué ou non substitué incluant 1 à 50 atomes de carbone, un groupe alcényle substitué ou non substitué incluant 2 à 50 atomes de carbone, un groupe alcynyle substitué ou non substitué incluant 2 à 50 atomes de carbone, un groupe cycloalkyle substitué ou non substitué incluant 3 à 50 atomes de carbone de cycle, un groupe alcoxy substitué ou non substitué incluant 1 à 50 atomes de carbone, un groupe alkylthio substitué ou non substitué incluant 1 à 50 atomes de carbone, un groupe aryloxy substitué ou non substitué incluant 6 à 50 atomes de carbone de cycle, un groupe arylthio substitué ou non substitué incluant 6 à 50 atomes de carbone de cycle, un groupe aralkyle substitué ou non substitué incluant 7 à 50 atomes de carbone, -Si(R₃₁)(R₃₂)(R₃₃), - C(=O)R₃₄, -COOR₃₅, -N(R₃₆)(R₃₇), un atome d'halogène, un groupe cyano, un groupe nitro, un groupe aryle substitué ou non substitué incluant 6 à 50 atomes de carbone de cycle, ou un groupe hétérocyclique monovalent substitué ou non substitué incluant 5 à 50 atomes de carbone de cycle ; deux R₁₇ peuvent être identiques ou différents l'un de l'autre ; et
R_{A}, R_{B}, Rc et R_{D} sont indépendamment un groupe alkyle substitué ou non substitué incluant 1 à 50 atomes de carbone, un groupe aryle substitué ou non substitué incluant 6 à 50 atomes de carbone de cycle, ou un groupe hétérocyclique monovalent substitué ou non substitué incluant 5 à 50 atomes de carbone de cycle.

2. Composé représenté par la formule (9-3) : 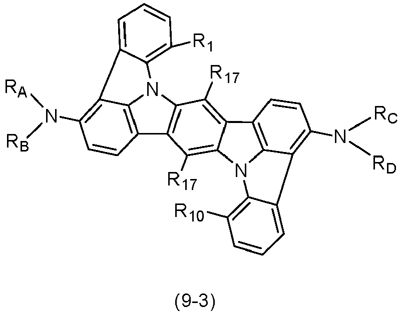 dans lequel, dans la formule (9-3),
R₁, et R₁₀ sont indépendamment un groupe alkyle substitué ou non substitué incluant 1 à 50 atomes de carbone, un groupe haloalkyle substitué ou non substitué incluant 1 à 50 atomes de carbone, un groupe alcényle substitué ou non substitué incluant 2 à 50 atomes de carbone, un groupe alcynyle substitué ou non substitué incluant 2 à 50 atomes de carbone, un groupe cycloalkyle substitué ou non substitué incluant 3 à 50 atomes de carbone de cycle, un groupe alcoxy substitué ou non substitué incluant 1 à 50 atomes de carbone, un groupe alkylthio substitué ou non substitué incluant 1 à 50 atomes de carbone, un groupe aryloxy substitué ou non substitué incluant 6 à 50 atomes de carbone de cycle, un groupe arylthio substitué ou non substitué incluant 6 à 50 atomes de carbone de cycle, un groupe aralkyle substitué ou non substitué incluant 7 à 50 atomes de carbone, -Si(R₃₁)(R₃₂)(R₃₃), - C(=O)R₃₄, -COOR₃₅, -N(R₃₆)(R₃₇), un atome d'halogène, un groupe cyano, un groupe nitro, un groupe aryle substitué ou non substitué incluant 6 à 50 atomes de carbone de cycle, ou un groupe hétérocyclique monovalent substitué ou non substitué incluant 5 à 50 atomes de carbone de cycle ;
R₁₇ est un atome d'hydrogène, un groupe alkyle substitué ou non substitué incluant 1 à 50 atomes de carbone, un groupe haloalkyle substitué ou non substitué incluant 1 à 50 atomes de carbone, un groupe alcényle substitué ou non substitué incluant 2 à 50 atomes de carbone, un groupe alcynyle substitué ou non substitué incluant 2 à 50 atomes de carbone, un groupe cycloalkyle substitué ou non substitué incluant 3 à 50 atomes de carbone de cycle, un groupe alcoxy substitué ou non substitué incluant 1 à 50 atomes de carbone, un groupe alkylthio substitué ou non substitué incluant 1 à 50 atomes de carbone, un groupe aryloxy substitué ou non substitué incluant 6 à 50 atomes de carbone de cycle, un groupe arylthio substitué ou non substitué incluant 6 à 50 atomes de carbone de cycle, un groupe aralkyle substitué ou non substitué incluant 7 à 50 atomes de carbone, -Si(R₃₁)(R₃₂)(R₃₃), - C(=O)R₃₄, -COOR₃₅, -N(R₃₆)(R₃₇), un atome d'halogène, un groupe cyano, un groupe nitro, un groupe aryle substitué ou non substitué incluant 6 à 50 atomes de carbone de cycle, ou un groupe hétérocyclique monovalent substitué ou non substitué incluant 5 à 50 atomes de carbone de cycle ; deux R₁₇ peuvent être identiques ou différents l'un de l'autre ; et
R_{A}, R_{B}, Rc et R_{D} sont indépendamment un groupe alkyle substitué ou non substitué incluant 1 à 50 atomes de carbone, un groupe aryle substitué ou non substitué incluant 6 à 50 atomes de carbone de cycle, ou un groupe hétérocyclique monovalent substitué ou non substitué incluant 5 à 50 atomes de carbone de cycle.

3. Composé selon l'une quelconque des revendications 1 ou 2, dans lequel R_{A}, R_{B}, Rc et R_{D} sont indépendamment un groupe aryle substitué ou non substitué incluant 6 à 50 atomes de carbone de cycle, ou un groupe hétérocyclique monovalent substitué ou non substitué incluant 5 à 50 atomes de carbone de cycle.

4. Composé selon l'une quelconque des revendications 1 à 3, dans lequel R_{A}, R_{B}, R_{C}, et R_{D} sont indépendamment un groupe phényle substitué ou non substitué.

5. Composé selon l'une quelconque des revendications 1 à 4, dans lequel R_{A}, R_{B}, R_{C}, et R_{D} sont indépendamment un groupe phényle substitué par un groupe alkyle incluant 1 à 5 atomes de carbone, ou un groupe phényle non substitué.

6. Composé selon l'une quelconque des revendications 1 à 5, dans lequel les R₁₇ sont des atomes d'hydrogène.

7. Composé selon l'une quelconque des revendications 1 à 6, dans lequel le substituant dans le cas de « substitué ou non substitué » est choisi dans le groupe consistant en groupe alkyle incluant 1 à 50 atomes de carbone, groupe haloalkyle incluant 1 à 50 atomes de carbone, groupe alcényle incluant 2 à 50 atomes de carbone, groupe alcynyle incluant 2 à 50 atomes de carbone, groupe cycloalkyle incluant 3 à 50 atomes de carbone de cycle, groupe alcoxy incluant 1 à 50 atomes de carbone, groupe alkylthio incluant 1 à 50 atomes de carbone, groupe aryloxy incluant 6 à 50 atomes de carbone de cycle, et groupe arylthio incluant 6 à 50 atomes de carbone de cycle, groupe aralkyle incluant 7 à 50 atomes de carbone, -Si(R₄₁)(R₄₂)(R₄₃), -C(=O)R₄₄, -COOR₄₅, -S(=O)₂R₄₆, -P(=O)(R₄₇)(R₄₈), - Ge(R₄₉)(R₅₀)(R₅₁), -N(R₅₂)(R₅₃), groupe hydroxy, atome d'halogène, groupe cyano, groupe nitro, groupe aryle incluant 6 à 50 atomes de carbone de cycle, et groupe hétérocyclique monovalent incluant 5 à 50 atomes de carbone de cycle ; et R₄₁ à R₅₃ sont indépendamment un atome d'hydrogène, un groupe alkyle incluant 1 à 50 atomes de carbone, un groupe aryle incluant 6 à 50 atomes de carbone de cycle, ou un groupe hétérocyclique monovalent incluant 5 à 50 atomes de carbone de cycle ; lorsque deux ou plus de chacun de R₄₁ à R₅₃ sont présents, les deux ou plus de chacun de R₄₁ à R₅₃ peuvent être identiques ou différents les uns des autres.

8. Composé selon l'une quelconque des revendications 1 à 7, dans lequel le substituant dans le cas de « substitué ou non substitué » est un groupe choisi dans le groupe consistant en :
groupe alkyle incluant 1 à 18 atomes de carbone, et
groupe aryle incluant 6 à 18 atomes de carbone de cycle.

9. Matériau pour un dispositif électroluminescent organique, comprenant un composé selon l'une quelconque des revendications 1 à 8.

10. Dispositif électroluminescent organique comprenant :
une cathode ;
une anode ; et
au moins une couche organique disposée entre la cathode et l'anode, dans lequel
au moins une couche de ladite au moins une couche organique comprend le composé selon l'une quelconque des revendications 1 à 8.

11. Dispositif électroluminescent organique selon la revendication 10, dans lequel la au moins une couche de la au moins une couche organique comprend un second composé qui n'est pas identique au composé selon l'une quelconque des revendications 1 à 8.

12. Dispositif électroluminescent organique selon la revendication 11, dans lequel le second composé est un composé représenté par la formule (10) suivante : 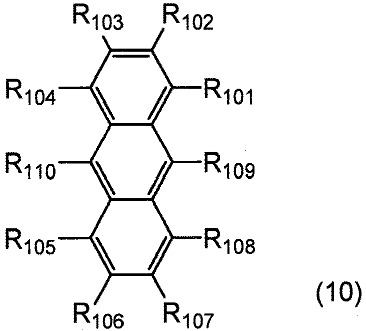 dans lequel, dans la formule (10),
un ou plusieurs ensembles de deux éléments adjacents ou plus parmi R₁₀₁ à R₁₁₀ forment un cycle saturé ou insaturé substitué ou non substitué, ou ne forment pas le cycle saturé ou insaturé substitué ou non substitué ;
R₁₀₁ à R₁₁₀ qui ne forment pas le cycle saturé ou insaturé substitué ou non substitué sont indépendamment
un atome d'hydrogène,
un substituant R, ou
un groupe représenté par la formule (11) suivante :
-L₁₀₁-Ar₁₀₁ (11)
dans lequel, dans la formule (11),
L₁₀₁ est
une liaison simple,
un groupe arylène substitué ou non substitué incluant 6 à 50 atomes de carbone de cycle, ou
un groupe hétérocyclique divalent substitué ou non substitué incluant 5 à 50 atomes de carbone de cycle ;
Ar₁₀₁ est
un groupe aryle substitué ou non substitué incluant 6 à 50 atomes de carbone de cycle, ou
un groupe hétérocyclique monovalent substitué ou non substitué incluant 5 à 50 atomes de carbone de cycle ;
le substituant R est
un groupe alkyle substitué ou non substitué incluant 1 à 50 atomes de carbone,
un groupe alcényle substitué ou non substitué incluant 2 à 50 atomes de carbone,
un groupe alcynyle substitué ou non substitué incluant 2 à 50 atomes de carbone,
un groupe cycloalkyle substitué ou non substitué incluant 3 à 50 atomes de carbone de cycle,
-Si(R₉₀₁)(R₉₀₂)(R₉₀₃),
-O-(R₉₀₄),
-S-(R₉₀₅),
-N(R₉₀₆)(R₉₀₇),
un atome d'halogène, un groupe cyano, un groupe nitro,
un groupe aryle substitué ou non substitué incluant 6 à 50 atomes de carbone de cycle, ou
un groupe hétérocyclique monovalent substitué ou non substitué incluant 5 à 50 atomes de carbone de cycle ;
lorsque deux substituants R ou plus sont présents, les deux substituants R ou plus peuvent être identiques ou différents les uns des autres ;
R₉₀₁ à R₉₀₇ sont indépendamment
un atome d'hydrogène,
un groupe alkyle substitué ou non substitué incluant 1 à 50 atomes de carbone,
un groupe cycloalkyle substitué ou non substitué incluant 3 à 50 atomes de carbone de cycle,
un groupe aryle substitué ou non substitué incluant 6 à 50 atomes de carbone de cycle, ou
un groupe hétérocyclique monovalent substitué ou non substitué incluant 5 à 50 atomes de carbone de cycle ;
lorsque deux ou plus de chacun parmi R₉₀₁ à R₉₀₇ sont présents, les deux ou plus de chacun parmi R₉₀₁ à R₉₀₇ peuvent être identiques ou différents les uns des autres ;
à condition qu'au moins un parmi R₁₀₁ à R₁₁₀ qui ne forme pas le cycle saturé ou insaturé substitué ou non substitué soit un groupe représenté par la formule (11) ; et
lorsque deux ou plus de la formule (11) sont présents, les deux groupes ou plus représentés par la formule (11) peuvent être identiques ou différents les uns des autres.

13. Dispositif électroluminescent organique selon l'une quelconque des revendications 10 à 11, dans lequel au moins une de la au moins une couche organique est une couche émettrice.

14. Dispositif électroluminescent organique selon la revendication 13, dans lequel la couche émettrice comprend en outre un composé hôte présentant une fluorescence retardée.
